# EUROPEAN PATENT SPECIFICATION

(11) **EP 3 802 548 B1**
(45) Date of publication and mention of the grant of the patent: **14.08.2024**
(21) Application number: 19731187.1
(22) Date of filing: 07.06.2019
(51) Int. Cl.: C07D 513/04, A61P 43/00, A61K 31/519, A61K 31/4365

(54) **NOVEL COMPOUNDS FOR DIAGNOSIS**
NEUARTIGE VERBINDUNGEN ZUR DIAGNOSE
NOUVEAUX COMPOSÉS POUR DIAGNOSTIC

(30) Priority: 08.06.2018 EP 18176768; 05.12.2018 EP 18210495
(43) Date of publication of application: 14.04.2021
(73) Proprietor: AC Immune SA, 1015 Lausanne (CH)
(72) Inventor: MOLETTE, Jérôme, 01280 Prevessin Moens (FR)
(74) Representative: Vossius & Partner Patentanwälte Rechtsanwälte mbB
(86) International application number: PCT/EP2019/065031
(87) International publication number: WO 2019/234243

(56) References cited:
- WO-A1-2017/153601

## Description

### Field of the invention

The present invention relates to novel compounds that can be employed in the diagnosis, monitoring of disease progression or monitoring of drug activity of a group of disorders and abnormalities associated with alpha-synuclein (a-synuclein, A-synuclein, aSynuclein, asynuclein, A-syn, α-syn, aSyn, SNCA, Non-amyloid beta component of Alzheimer's disease (AD) amyloid plaques, Non-A4 component of amyloid precursor, NACP) aggregates including, but not limited to, Lewy bodies and/or Lewy neurites, such as Parkinson's disease (PD). The instant compounds are particularly useful in the diagnosis of the preclinical state of such a disorder, monitoring residual disorder, or predicting the responsiveness of a patient who is suffering from such a disorder to the treatment with a certain medicament.

### Background of the invention

Many diseases of aging are based on or associated with extracellular or intracellular deposits of amyloid or amyloid-like protein aggregates that contribute to the pathogenesis as well as to the progression of the disease. The best characterized amyloidogenic protein that forms extracellular aggregates is amyloid beta (Aβ, Abeta).

Amyloidogenic proteins, that form mainly intracellular aggregates, include, but are not limited to Tau, alpha-synuclein, and huntingtin (htt). Disorders and abnormalities associated with alpha-synuclein include pre-disease states as well as diseases involving alpha-synuclein aggregates. These diseases are generally listed as synucleinopathies (or α-synucleinopathies) and include, but are not limited to, Parkinson's disease (sporadic, familial with alpha-synuclein mutations and/or multiplications, familial with mutations other than alpha-synuclein, pure autonomic failure and Lewy body dysphagia), dementia with Lewy bodies ("pure" Lewy body dementia), sporadic Alzheimer's disease, familial Alzheimer's disease with APP mutations, familial Alzheimer's disease with PS-1, PS-2 or other mutations, familial British dementia, Lewy body variant of Alzheimer's disease and Down syndrome. Synucleinopathies with neuronal and glial aggregates of alpha-synuclein include multiple system atrophy (MSA) (Shy-Drager syndrome, striatonigral degeneration and olivopontocerebellar atrophy). Other diseases that may have alpha-synuclein-immunoreactive lesions include traumatic brain injury, chronic traumatic encephalopathy, motor neuron disease, neuroaxonal dystrophy, neurodegeneration with brain iron accumulation type 1 (Hallervorden-Spatz syndrome), prion diseases, ataxia telangiectatica, Meige's syndrome, subacute sclerosing panencephalitis, Gaucher disease as well as other lysosomal storage disorders (including Kufor-Rakeb syndrome and Sanfilippo syndrome) and rapid eye movement (REM) sleep behavior disorder (Jellinger, Mov. Disord. 2003, 18 Suppl. 6, S2-12; Galvin et al., JAMA Neurology 2001, 58 (2), 186-190; Kovari et al., Acta Neuropathol. 2007, 114(3), 295-8; Saito et al., J. Neuropathol. Exp. Neurol. 2004, 63(4), 323-328; McKee et al., Brain, 2013, 136(Pt 1), 43-64; Puschmann et al., Parkinsonism Relat Disord 2012, 18S1, S24-S27; Usenovic et al., J Neurosci. 2012, 32(12), 4240-4246; Winder-Rhodes et al., Mov. Disord. 2012, 27(2), 312-315; Ferman et al., J. Int. Neuropsychol Soc. 2002, 8(7), 907-914).

Alpha-synuclein is a 140 amino acid natively unfolded protein (Iwai et al., Biochemistry 1995, 34(32), 10139-10145). The sequence of alpha-synuclein can be divided into three main domains: 1) the N-terminal region comprising of residues 1-60, which contains the 11-mer amphipatic imperfect repeat residues with highly conserved hexamer (KTKEGV). This region has been implicated in regulating alpha-synuclein binding to membranes and its internalization; 2) the hydrophobic Non Amyloid beta Component (NAC) domain spanning residues 61-95; which is essential for alpha-synuclein aggregation; and 3) the C-terminal region spanning residues 96-140 which is highly acidic and proline-rich, having not known distinct structural and functional property. Alpha-synuclein has been shown to undergo several post translational modifications, including but not limited to truncations, phosphorylation, ubiquitination, oxidation and/or transglutaminase covalent crosslinking (Fujiwara et al., Nat. Cell Biol. 2002, 4(2); 160-164; Hasegawa et al., J. Biol. Chem. 2002, 277(50), 49071-49076; Li et al., Proc. Natl. Acad. Sci. USA 2005, 102(6), 2162-2167; Oueslati et al., Prog Brain Res 2010, 183, 115-145; Schmid et al., J Biol Chem 2009, 284(19), 13128-13142). Interestingly, the majority of these modifications involve residues within the C-terminal region.

Several phosphorylation sites have been detected in the carboxyl-terminal region on Tyr-125, -133, and -136, and on Ser-129 (Negro et al., FASEB J 2002, 16(2), 210-212). Extensive and selective phosphorylation of alpha-synuclein at Ser-129 is evident in synucleinopathy lesions, including Lewy bodies (Fujiwara et al., Nat Cell Biol 2002, 4(2); 160-164). Other post-translational modifications in the carboxyl-terminal, including glycosylation on Ser-129 (McLean et al., Neurosci Lett 2002, 323(3), 219-223) and nitration on Tyr-39, Tyr-125, -133, and -136 (Takahashi et al., Brain Res 2002, 938(1-2), 73-80; Hodara et al., J Biol Chem 2004, 279(46), 47746-47753), may affect aggregation of alpha-synuclein. Truncation of the carboxyl-terminal region by proteolysis has been reported to play a role in alpha-synuclein fibrillogenesis in various neurodegenerative diseases (Rochet et al., Biochemistry 2000, 39(35), 10619-10626). Full-length as well as partially truncated and insoluble aggregates of alpha-synuclein have been detected in highly purified Lewy bodies (Crowther et al., FEBS Lett 1998, 436(3), 309-312).

Abnormal protein aggregation appears to be a common feature in brain aging and in several neurodegenerative diseases, although a clear role in the disease process remains to be defined. In *in vitro* models, alpha-synuclein (or some of its truncated forms) readily assembles into filaments resembling those isolated from brains of patients with LB dementia and familiar PD (Crowther et al., FEBS Lett 1998, 436(3), 309-312). Alpha-synuclein and its mutated forms (A53T, A30P, E46K, H50Q, G51D, A53E and A53V) have a random coil conformation and do not form significant secondary structures in aqueous solution at low concentrations; however, at higher concentrations they are prone to self-aggregate, producing amyloid fibrils (Wood et al., J Biol Chem 1999, 274(28), 19509-19512). Several differences in the aggregation behavior of the PD-linked mutants and the wild-type protein have been documented. Monomeric alpha-synuclein aggregates *in vitro* form stable fibrils via a metastable oligomeric (i.e., protofibril) state (Voiles et al., Biochemistry 2002, 41(14), 4595-4602).

PD is the most common neurodegenerative motor disorder. PD is mainly an idiopathic disease, although in at least 5% of the PD patients the pathology is linked to mutations in one or several specific genes (Lesage et al., Hum. Mol. Genet., 2009, 18, R48-59). The pathogenesis of PD remains elusive, however, growing evidence suggests a role for the pathogenic folding of the alpha-synuclein protein that leads to the formation of a variety of aggregates. Indeed, the hallmarks of PD are the neuronal loss mainly of dopaminergic neurons in the substantia nigra but not limited to this brain region and the presence of intracellular alpha-synuclein aggregate structures in the neuronal soma and neurites called Lewy Bodies and Lewy Neurites, respectively. Alpha-synuclein is a natively unfolded presynaptic protein that can misfold and aggregate into larger oligomeric and fibrillar forms which are linked to the pathogenesis of PD. Recent studies have implicated small soluble oligomeric and protofibrillar forms of alpha-synuclein as the most neurotoxic species (Lashuel et al., J. Mol. Biol., 2002, 322, 1089-102), however the precise role of alpha-synuclein aggregates in the neuronal cell toxicity remains to be clarified (review: Cookson, Annu. Rev. Biochem., 2005, 74, 29-52).

The diagnosis of PD is largely clinical and depends on the presence of a specific set of symptoms and signs (the initial core feature being bradykinesia, rigidity, rest tremor and postural instability), the absence of atypical features, a slowly progressive course, and a response to drug therapy. The diagnosis requires clinical skills but is open to a degree of subjectivity and error, as several other degenerative and non-degenerative diseases can mimic PD (MSA, progressive supranuclear palsy (PSP), AD, essential tremor, dystonic tremor), (Guideline No. 113: Diagnosis and pharmacological management of PD, January 2010. SIGN). The final confirmation of the diagnosis is made by post-mortem neuropathological analysis.

Computed tomography (CT) and conventional magnetic resonance imaging (MRI) brain scans of people with PD usually appear normal. These techniques are nevertheless useful to rule out other diseases that can be secondary causes of parkinsonism, such as basal ganglia tumors, vascular pathology and hydrocephalus. A specific technique of MRI, diffusion MRI, has been reported to be useful at discriminating between typical and atypical parkinsonism, although its exact diagnostic value is still under investigation. Dopaminergic function in the basal ganglia can be measured with different PET and SPECT radiotracers. Examples are ioflupane (¹²³I) (trade name DaTSCAN) and iometopane (Dopascan) for SPECT or fluorodeoxyglucose (¹⁸F) and DTBZ for PET. A pattern of reduced dopaminergic activity in the basal ganglia can aid in diagnosing PD (Brooks, J. Nucl. Med., 2010, 51, 596-609; Redmond, Neuroscientist, 2002, 8, 457-88; Wood, Nat. Rev. Neurol., 2014, 10, 305).

Strategies are being developed to apply recent advances of the cause of PD to the development of biochemical biomarkers (Schapira, Curr Opin Neurol 2013; 26(4):395-400). Such biomarkers that have been investigated in different body fluids (e.g. cerebrospinal fluid (CSF), plasma, saliva) include alpha-synuclein levels but also DJ-1, Tau and Abeta, as well as neurofilament proteins, interleukins, osteopontin and hypocrontin (Schapira, Curr Opin Neurol 2013; 26(4):395-400), but so far none of these biomarkers alone or in combination can be used as a determinant diagnostic test. To our knowledge no approved alpha-synuclein diagnostic agent is currently on the market or available for clinical use despite a crucial need for it, for PD research and drug development (Eberling et al., J Parkinsons Dis. 2013; 3(4):565-7).

The ability to image alpha-synuclein deposition in the brain would be extremely useful and would result in a huge advancement for PD diagnosis, research and drug development. The accumulation of aggregated alpha-synuclein in the brain is a pathological hallmark of PD and a priority target for drug development given its hypothesized contribution to neurodegeneration. *In vivo* imaging of alpha-synuclein pathology could be useful as a biomarker of the presence of the disease and disease progression and as a pharmacodynamics tool for drug development. The development of an alpha-synuclein PET imaging agent is considered as very important for the diagnosis and monitoring of the effects of therapeutics targeting alpha-synuclein (Eberling, Dave and Frasier, J. Parkinson's Disease, 3, 565-567 (2013)). Despite a huge effort to identify an alpha-synuclein PET ligand, so far few compounds were identified but they are not optimal for a number of reasons: low or no affinity to pathological aggregates of alpha-synuclein present in the diseased brains, low or non-selectivity for alpha-synuclein over other aggregated proteins and inappropriate physicochemical properties (Eberling et al., J Parkinsons Dis. 2013; 3(4):565-7; Neal et al., Mol Imaging Biol. 2013; 15:585-595; Bagchi et al., PLoS One 2013;8(2):e55031; Yu et al., Bioorganic and Medicinal chemistry 2012;20:4625-4634; Zhang et al., Appl Sci (Basel) 2014;4(1):66-78; Chu et al., J Med Chem, 2015, 58 (15):6002-17).

In order to achieve high alpha-synuclein selectivity, molecular probes have been used which recognize and bind to the pathological alpha-synuclein. In order to reduce background signal interference resulting from non-specific off-target binding, and to reduce dosing requirements, alpha-synuclein imaging compounds should bind with high affinity and selectivity to their target. For imaging of alpha-synuclein aggregates associated with neurological disorders such as PD, imaging compounds need to penetrate the blood brain barrier and pass into the relevant regions of the brain. For targeting intracellular amyloid-like inclusions such as alpha-synuclein, cell permeability is a further requirement of imaging compounds. A further prerequisite in order to avoid unnecessary accumulation of a compound which may result in increased risk of unwanted side-effects is a fast compound wash-out from the brain (or other targeting organ).

WO 2011/128455 refers to specific compounds which are suitable for treating disorders associated with amyloid proteins or amyloid-like proteins. US 2012/0302755 relates to certain imaging agents for detecting neurological dysfunction. Further compounds for the diagnosis of neurodegenerative disorders on the olfactory epithelium are discussed in WO 2012/037928.

WO 2010/063701 refers to a certain *in vivo* imaging agent for use in a method to determine the presence of, or susceptibility to, PD, wherein the *in vivo* imaging agent comprises an alpha-synuclein binder labelled with an *in vivo* imaging moiety, and wherein the *in vivo* imaging agent binds to alpha-synuclein with a binding affinity.

US 2014/0142089 relates to a method for preventing or treating a degenerative brain disease, the method comprising administering to a subject in need thereof an effective amount of a pharmaceutical composition comprising a specific compound, a pharmaceutically acceptable salt, an isomer, a solvate, a hydrate, and a combination thereof.

WO 2009/155017 describes aryl or heteroaryl substituted azabenzoxazole derivatives, which are stated to be useful as tracers in positron emission tomography (PET) imaging to study amyloid deposits in brain *in vivo* to allow diagnosis of Alzheimer's disease.

WO 2016/033445 refers to a specific compound for imaging huntingtin protein.

WO 2017/153601 refers to the compounds that can be employed in the diagnosis, monitoring of disease progression or monitoring of drug activity, of a group of disorders and abnormalities associated with alpha-synuclein.

### Summary of the invention

It was an object of the present invention to provide compounds that can be employed in the diagnosis, monitoring of disease progression, monitoring of drug activity, of a disorder or abnormality associated with alpha-synuclein aggregates including, but not limited to, Lewy bodies and/or Lewy neurites, such as PD. In particular, the compounds should be suitable for diagnosis of the preclinical state of such a disorder, monitoring residual disorder, or predicting the responsiveness of a patient who is suffering from such a disorder to the treatment with a certain medicament.

Furthermore, there exists a need in the art for compounds which can be used as imaging agents for alpha-synuclein aggregates including, but not limited to, Lewy bodies and/or Lewy neurites. In particular, it was an object of the present invention to provide compounds that are suitable as a diagnostic composition for positron emission tomography imaging of synucleinopathies, e.g., wherein the compounds are detectably labeled with ¹⁸F. The present inventors have surprisingly found that these objects can be achieved by the compounds of formula (I) as described hereinafter.

The compounds of formula (I) display high binding affinity to alpha-synuclein aggregates. Moreover, the compounds of formula (I) display high selectivity for alpha-synuclein over Aβ enabling the differentiation of PD from other proteinopathies that share common clinical and pathological features. Due to their unique design features, these compounds display properties such as appropriate lipophilicity and molecular weight, brain uptake and pharmacokinetics, cell permeability, solubility, and autofluorescence in order to be successful imaging probes for detection and quantification of alpha-synuclein aggregates including, but not limited to, Lewy bodies and/or Lewy neurites load *in vivo, ex vivo* and *in vitro.*

The present invention discloses novel compounds of formula (I) having enhanced binding properties to alpha-synuclein aggregates including, but not limited to, Lewy bodies and/or Lewy neurites. Some compounds of formula (I) have inproved pharmacokinetic (PK) parameters, such as brain washout and reduced unspecific retention. The compounds of this invention may be labeled (e.g., radiolabeled), so that they may be used for *ex vivo* and *in vivo* imaging to detect alpha-synuclein aggregates including, but not limited to, Lewy bodies and/or Lewy neurites. The present invention provides methods for the detection of alpha-synuclein aggregates including, but not limited to, Lewy bodies and/or Lewy neurites, *ex vivo* using a compound of formula (I) or a pharmaceutical composition thereof. The present invention provides compounds of formula (I) for use as imaging agents, particularly for diagnosis of presymptomatic and/or premotor PD and/or advanced PD and/or other alpha-synucleinopathies, e.g., using positron emission tomography (PET). The invention would serve as a biomarker for monitoring of topographic progression of pathology, leading to improvement of clinical diagnosis and clinical study design. The present invention further provides a diagnostic composition comprising a compound of formula (I) and optionally a pharmaceutically acceptable carrier, diluent, adjuvant and/or excipient.

The present invention is set out in the appended set of claims.

The compounds, which are disclosed in the examples or in claims as well as
stereoisomers, racemic mixtures, pharmaceutically acceptable salts, hydrates, solvates, and polymorphs thereof, are considered to be particularly suitable compounds of the present invention.

The compounds of the present invention can optionally contain a detectable label selected from radionuclides, positron emitters, gamma emitters, fluorescent labels, luminescent labels and chromogenic labels. These compounds will be denoted as "detectably labeled derivatives" for brevity in the following.

### Definitions

Within the meaning of the present application the following definitions apply:
"Alkyl" refers to a saturated straight or branched organic moiety consisting of carbon and hydrogen atoms. Examples of suitable alkyl groups have 1 to 6 carbon atoms, preferably 1 to 4 carbon atoms, and include methyl, ethyl, propyl, isopropyl, n-butyl, t-butyl and isobutyl.

"Fluoro-alkyl" refers to an "Alkyl" bound to a fluorine, for example -CH₂CH₂F.

"Alkyl-O-alkyl" refers to an "Alkyl" bound to an oxygen bound to an "Alkyl", for example CH₃OCH₂CH₂-.

"Hal" or "halogen" refers to F, Cl, Br, and I. With respect to diagnostic applications, F (e.g., ¹⁹F and ¹⁸F) is particularly preferred.

The term "leaving group" (**LG**) as employed herein is any leaving group and means an atom or group of atoms that can be replaced by another atom or group of atoms. Examples are given e.g. in Synthesis (1982), p. 85-125, table 2, Carey and Sundberg, Organische Synthese, (1995), page 279-281, table 5.8; or Netscher, Recent Res. Dev. Org. Chem., 2003, 7, 71-83, scheme 1, 2, 10 and 15 and others). (Coenen, Fluorine-18 Labeling Methods: Features and Possibilities of Basic Reactions, (2006), in: Schubiger P.A., Friebe M., Lehmann L., (eds), PET-Chemistry - The Driving Force in Molecular Imaging. Springer, Berlin Heidelberg, pp.15-50, explicitly: scheme 4 pp. 25, scheme 5 pp 28, table 4 pp 30, Figure 7 pp 33). Preferably, the "leaving group" (**LG**) is selected from halogen, trimethylammonium, C₁₋₄ alkyl sulfonate (e.g. mesylate or triflate) and C₆₋₁₀ aryl sulfonate (e.g. tosylate or nosylate).

The terms "protecting group" (**PG**) and "amino protecting group" as employed herein is any protecting group which is suitable for protecting an amine group during an envisaged chemical reaction. Examples of suitable protecting groups are well-known to a person skilled in the art. Suitable protecting groups are discussed, e.g., in the textbook Greene and Wuts, Protecting groups in Organic Synthesis, third edition, page 494-653, which is included herein by reference. Protecting groups can be chosen from carbamates, amides, imides, N-alkyl amines, N-aryl amines, imines, enamines, boranes, N-P protecting groups, N-sulfenyl, N-sulfonyl and N-silyl. Specific preferred examples of protecting groups (**PG**) are carbobenzyloxy (Cbz), p-methoxybenzyl carbonyl (Moz or MeOZ), tert-butyloxycarbonyl (BOC), 9-fluorenylmethyloxycarbonyl (FMOC), benzyl (Bn), p-methoxybenzyl (PMB), 3,4-dimethoxybenzyl (DMPM), p-methoxyphenyl (PMP), triphenylmethyl (Trityl), methoxyphenyl diphenylmethyl (MMT), or dimethoxytrityl (DMT).

Compounds of the present invention having one or more optically active carbons can exist as racemates and racemic mixtures, stereoisomers (including diastereomeric mixtures and individual diastereomers, enantiomeric mixtures and single enantiomers, mixtures of conformers and single conformers), tautomers, atropisomers, and rotamers. All isomeric forms are included in the present invention. Compounds described in this invention containing olefinic double bonds include E and Z geometric isomers. Also included in this invention are all salt forms, polymorphs, hydrates and solvates.

The term "polymorphs" refers to the various crystalline structures of the compounds of the present invention. This may include, but is not limited to, crystal morphologies (and amorphous materials) and all crystal lattice forms. Salts of the present invention can be crystalline and may exist as more than one polymorph. Solvates, hydrates as well as anhydrous forms of the salt are also encompassed by the invention. The solvent included in the solvates is not particularly limited and can be any pharmaceutically acceptable solvent. Examples include water and C₁₋₄ alcohols (such as methanol or ethanol).

"Pharmaceutically acceptable salts" are defined as derivatives of the disclosed compounds wherein the parent compound is modified by making acid or base salts thereof. Examples of pharmaceutically acceptable salts include, but are not limited to, mineral or organic acid salts of basic residues such as amines; alkali or organic salts of acidic residues such as carboxylic acids; and the like. The pharmaceutically acceptable salts include the conventional non-toxic salts or the quaternary ammonium salts of the parent compound formed, for example, from non-toxic inorganic or organic acids. For example, such conventional non-toxic salts include those derived from inorganic acids such as, but not limited to, hydrochloric, hydrobromic, sulfuric, sulfamic, phosphoric, nitric and the like; and the salts prepared from organic acids such as, but not limited to, acetic, propionic, succinic, glycolic, stearic, lactic, malic, tartaric, citric, ascorbic, pamoic, maleic, hydroxymaleic, phenylacetic, glutamic, benzoic, salicylic, sulfanilic, 2-acetoxybenzoic, fumaric, toluenesulfonic, methanesulfonic, ethane disulfonic, oxalic, isethionic, and the like. The pharmaceutically acceptable salts of the present invention can be synthesized from the parent compound which contains a basic or acidic moiety by conventional chemical methods. Generally, such salts can be prepared by reacting the free acid or base forms of these compounds with a stoichiometric amount of the appropriate base or acid in water or in an organic solvent, or in a mixture of the two. Organic solvents include, but are not limited to, nonaqueous media like ethers, ethyl acetate, ethanol, isopropanol, or acetonitrile. Lists of suitable salts can be found in Remington's Pharmaceutical Sciences, 18th ed., Mack Publishing Company, Easton, PA, 1990, p. 1445, the disclosure of which is hereby incorporated by reference.

"Pharmaceutically acceptable" is defined as those compounds, materials, compositions, and/or dosage forms which are, within the scope of sound medical judgment, suitable for use in contact with the tissues of human beings and animals without excessive toxicity, irritation, allergic response, or other problem or complication commensurate with a reasonable benefit/risk ratio.

The compounds in the form of a prodrug, namely a compound which is metabolized *in vivo* to the active metabolite are also disclosed but not claimed. The reference by Goodman and Gilman (The Pharmacological Basis of Therapeutics, 8 ed, McGraw-Hill, Int. Ed. 1992, "Biotransformation of Drugs", p 13-15) describing prodrugs generally is hereby incorporated herein by reference.

The patients or subjects in the present invention are typically animals, particularly mammals, more particularly humans.

"Alpha-synuclein aggregates" are: multimeric beta-sheet rich assemblies of alpha-synuclein monomers that can form soluble oligomers, soluble/insoluble protofibrils, mature fibrils and/or amorphous aggregates which coalesce into intracellular deposits detected as a range of Lewy pathologies in PD and other synucleinopathies. Aggregated alpha-synuclein is the basis for pathologies in patients and can be detected in the following morphologies: Lewy bodies, Lewy neurites, premature Lewy bodies or pale bodies, perikaryal deposits with diffuse, granular, punctate or pleomorphic patterns. Moreover, alpha-synuclein aggregates are the major component of intracellular fibrillary inclusions detected in oligodendrocytes (also referred to as glial cytoplasmic inclusions) and in neuronal somata, axons and nuclei (referred to as neuronal cytoplasmic inclusions) that are the histological hallmarks of multiple system atrophy. Alpha-synuclein aggregates in patients often display substantial increase in post-translational modifications such as phosphorylation, ubiquitination, nitration, and truncation.

"Lewy bodies" are abnormal aggregates of protein that develop inside nerve cells in PD, Lewy body dementia and other synucleinopathies. Lewy bodies appear as spherical masses that displace other cell components. Morphologically, Lewy bodies can be classified as being brainstem or cortical type. Classic brainstem Lewy bodies are eosinophilic cytoplasmic inclusions consisting of a dense core surrounded by a halo of 5-10nm-wide radiating fibrils, the primary structural component of which is alpha-synuclein; cortical Lewy bodies differ by lacking a halo. The presence of Lewy bodies is a hallmark of PD.

"Lewy neurites" are abnormal neuronal processes in diseased neurons, containing granular material, abnormal alpha-synuclein filaments similar to those found in Lewy bodies, dot-like, varicose structures and axonal spheroids. Like Lewy bodies, Lewy neurites are a feature of α-synucleinopathies such as dementia with Lewy bodies, Parkinson's disease, and multiple system atrophy.

The "preclinical state" of disease is defined as the phase of disease where disease-associated changes on the molecular level are not leading to overt clinical representation in the patient.

The preferred definitions given in the "Definition"-section apply to all of the embodiments described below unless stated otherwise.

### Description of the Figures

Figure 1: Whole brain pharmacokinetic measured after iv injection in NHP (non-human primate) of ¹⁸**F-6** and **¹⁸F-Example 70** of WO2017/153601.
**Figure 2****:** Normalization of SUV whole brain NHP pharmacokinetic to compare washout properties of ¹⁸**F-6** and **¹⁸F-Example 70** of WO2017/153601.
**Figure 3****:** Incubation of amygdala (AMG) brain sections from several PD/PDD donors or sections of the middle frontal gyrus (MFG) and amygdala brain region from non-demented controls (NDC) with **³H-6.** To determine non-specific binding, adjacent sections for each case were incubated with **³H-6** in the presence of unlabeled ligand in excess (5µM). Representative images of the total ('-') and non-specific ('+') autoradiography signal for each case are shown.

### Detailed description of the invention

The compounds of the present invention will be described in the following. It is to be understood that all possible combinations of the following definitions are also envisaged.

In one embodiment, the present invention relates to a compound of formula (I): 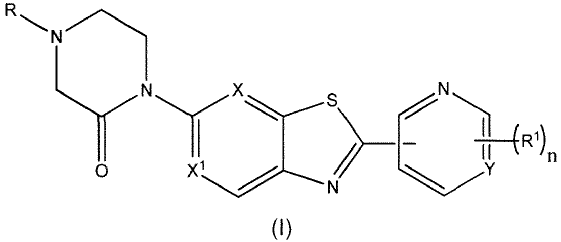 and detectably labeled derivatives, stereoisomers, racemic mixtures, pharmaceutically acceptable salts, hydrates, solvates and polymorphs thereof.

The compound of the formula (I) can be, for instance, a compound of formula (I*) 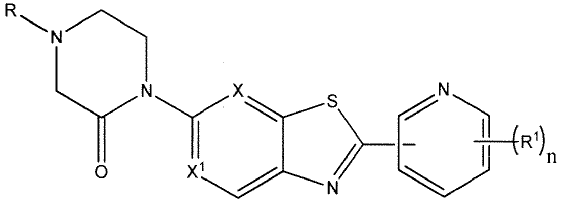 wherein R, X, X¹, R¹ and n are as defined herein. In one embodiment, R¹ is independently selected from the group consisting of fluorine, 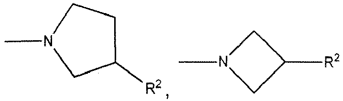 and -NR³R⁴.

Preferred compounds are also compounds of the formula (Ia), (lb) or (Ic)

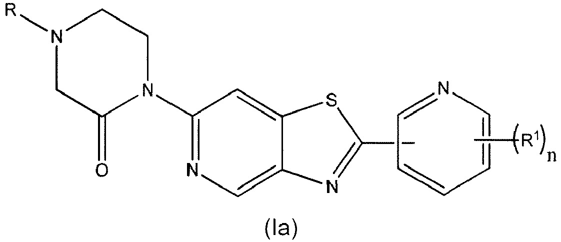

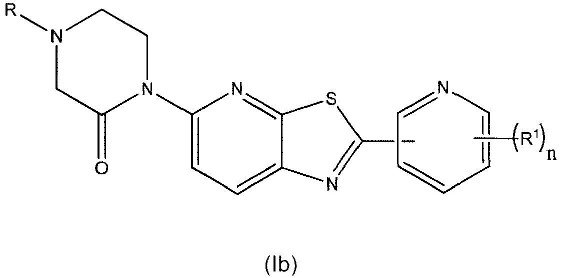

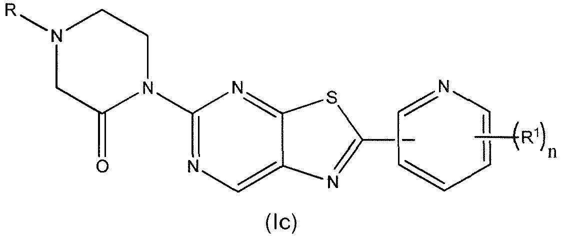

**R** is selected from the group consisting of hydrogen and alkyl. Preferably **R** is alkyl.

**R¹** is independently selected from the group consisting of fluorine, 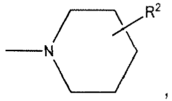 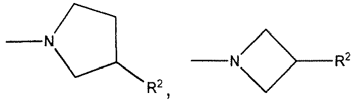 and -NR³R⁴. Preferably **R¹** is selected from fluorine, 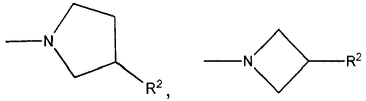 and -NR³R⁴, more preferably 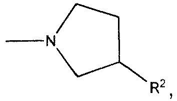 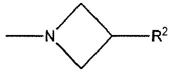 and -NR³R⁴, even more preferably **R¹** is

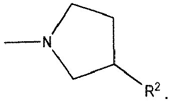

**R²** is selected from the group consisting of fluorine and hydrogen, more preferably **R²** is fluorine.

**R³** and **R⁴** are independently selected from the group consisting of alkyl, fluoro-alkyl, alkyl-O-alkyl and hydrogen. More preferably **R³** and **R⁴** are independently selected from the group consisting of alkyl or hydrogen.

**X** and **X¹** are independently selected from the group consisting of N and CH, provided that at least one of X and X¹ is N.

**Y** is independently selected from the group consisting of N and CH or **Y** is C if **Y** is attached to R1.

**n** is 1 or 2, preferably n is 1.

In the compounds of formula (I) preferably at least one of **R¹**, **R²**, **R³** and **R⁴** contains fluorine.

In one embodiment, the present invention relates to a compound of formula (II):

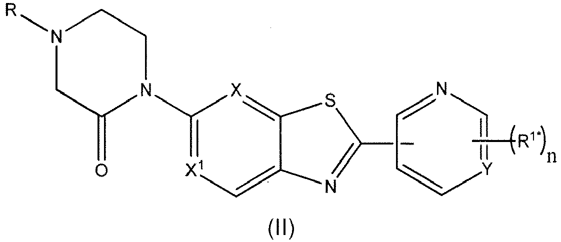

In one embodiment the compound of the formula (II) is a compound of the formula (II*) 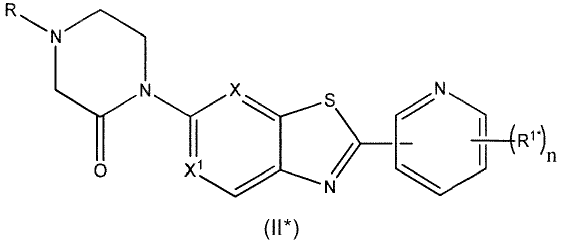 wherein R, X, X', R^{1*} and n are as defined herein. In one embodiment, **R^{1*}** is independently selected from the group consisting of **LG,** 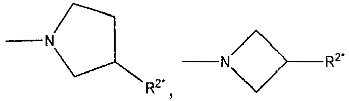 and -NR^{3*}R^{4*}.

**R^{1*}** is independently selected from the group consisting of **LG,** 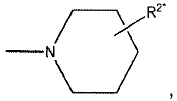 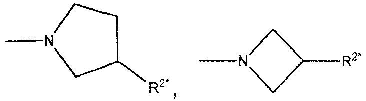 and -NR^{3*}R^{4*}. Preferably **R^{1*}** is selected from **LG,** 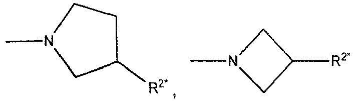 and -NR^{3*}R^{4*}, more preferably from 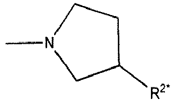 and 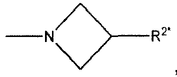 , even more preferably **R^{1*}** is

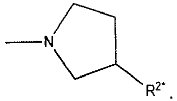

**R^{2*}** is selected from the group consisting of **LG** and hydrogen, more preferably **R^{2*}** is **LG.**

**R^{3*}** and **R^{4*}** are independently selected from the group consisting of alkyl, LG-alkyl, alkyl-O-alkyl, PG and hydrogen.

In the compounds of formula (II) preferably at least one of **R^{1*}, R^{2*}, R^{3*}** and **R^{4*}** contains **LG.**

**LG** is a leaving group selected from the group consisting of halogen, trimethylammonium, C₁₋₄ alkyl sulfonate or C₆₋₁₀ aryl sulfonate. Preferably **LG** is C₁₋₄ alkyl sulfonate or C₆₋₁₀ aryl sulfonate. More preferably the **LG** is mesylate, triflate, tosylate or nosylate.

**PG** is an amino protecting group selected from carbobenzyloxy, p-methoxybenzyl carbonyl, tert-butyloxycarbonyl, 9-fluorenylmethyloxycarbonyl, benzyl, p-methoxybenzyl, 3,4-dimethoxybenzyl, p-methoxyphenyl, triphenylmethyl, methoxyphenyl diphenylmethyl, and dimethoxytrityl.

Preferred compounds of formula (I) are the compounds given in the example section of the present specification.

The compounds of the present invention can be detectably labeled
with a detectable label selected from
radionuclides, positron emitters, gamma emitters, as well as fluorescent, luminescent and chromogenic labels. With respect to the detectably labeled compounds of the present invention which include a radioisotope, a positron emitter, or a gamma emitter, it is to be understood that the radioisotope, positron emitter, or gamma emitter is to be present in an amount which is not identical to the natural amount of the respective radioisotope, positron emitter, or gamma emitter. Furthermore, the employed amount should allow detection thereof by the chosen detection method.

Examples of suitable isotopes such as radionuclides, positron emitters and gamma emitters include ²H, ³H, ¹⁸F, ¹²³I, ¹²⁴I, ¹²⁵I, ¹³¹I, ¹¹C, ¹³N, ¹⁵O, and ⁷⁷Br, preferably ²H, ³H, ¹¹C, ¹³N, ¹⁵O, and ¹⁸F, more preferably ²H, ³H and ¹⁸F, even more preferably ¹⁸F.
more preferably ²H, ³H, ¹³N, and ¹⁸F, even
¹⁸F-labeled compounds are particularly suitable for imaging applications such as PET. The corresponding compounds which include fluorine having a natural ¹⁹F isotope are also of particular interest as they can be used as analytical standards and references during manufacturing, quality control, release and clinical use of their ¹⁸F-analogs.

Isotopic variations of the compounds of the invention can generally be prepared by conventional procedures such as by the illustrative methods or by the preparations described in the Examples and Preparative Examples hereafter using appropriate isotopic variations of suitable reagents, commercially available or prepared by known synthetic techniques.

Radionuclides, positron emitters and gamma emitters can be included into the compounds of the present invention by methods which are usual in the field of organic synthesis. Typically, they will be introduced by using a correspondingly labeled starting material when the desired compound of the present invention is prepared. Illustrative methods of introducing detectable labels are described, for instance, in US 2012/0302755.

The position at which the detectable label is to be attached to the compounds of the present invention is not particularly limited.

The radionuclides, positron emitters and gamma emitters, for example, can be attached at any position where the corresponding non-emitting atom can also be attached. For instance, ¹⁸F can be attached at any position which is suitable for attaching F. The same applies to the other radionuclides, positron emitters and gamma emitters. If ³H is employed as a detectable label it is preferably attached in the form of -C(³H)₃ at any position at which a methyl group can be attached. Alternatively, ³H *per se* can be attached at any available position. If ²H is employed as a detectable label it is preferably attached in the form of -C(²H)₃ at any position at which a methyl group can be attached. ¹¹C, ¹³N, and ¹⁵O can be incorporated into the compounds of the present invention at any position where C, N and O appear.

Further, substitution with isotopes such as deuterium, i.e., ²H, may afford certain diagnostic advantages resulting from greater metabolic stability by reducing for example defluorination, increased *in vivo* half-life or reduced dosage requirements, while keeping or improving the original compound efficacy.

### Diagnostic compositions

The compounds of formula (I) are particularly suitable for imaging of alpha-synuclein aggregates including, but not limited to, Lewy bodies and/or Lewy neurites. With respect to alpha-synuclein protein, compounds of formula (I) are particularly suitable for binding to various types of alpha-synuclein aggregates including, but not limited to, Lewy bodies and/or Lewy neurite.

Due to their design and to the binding characteristics, the compounds of formula (I) are suitable for use in the diagnosis of disorders and abnormalities associated with alpha-synuclein aggregates including, but not limited to, Lewy bodies and/or Lewy neurites. The compounds of formula (I) are particularly suitable for positron emission tomography imaging of alpha-synuclein aggregates including, but not limited to, Lewy bodies and/or Lewy neurites. Diseases involving alpha-synuclein aggregates are generally listed as synucleinopathies (or α-synucleinopathies). The compounds of formula (I) are suitable for use in the diagnosis of disorders including, but not limited to, PD (sporadic, familial with alpha-synuclein mutations, familial with mutations other than alpha-synuclein, pure autonomic failure and Lewy body dysphagia), dementia with Lewy bodies ("pure" Lewy body dementia), sporadic Alzheimer's disease, familial Alzheimer's disease with APP mutations, familial Alzheimer's disease with PS-1, PS-2 or other mutations, familial British dementia, Lewy body variant of Alzheimer's disease and Down syndrome. Synucleinopathies with neuronal and glial aggregates of alpha synuclein include multiple system atrophy (Shy-Drager syndrome, striatonigral degeneration and olivopontocerebellar atrophy). Other diseases that may have alpha-synuclein-immunoreactive lesions include traumatic brain injury, chronic traumatic encephalopathy, motor neuron disease, neuroaxonal dystrophy, neurodegeneration with brain iron accumulation type 1 (Hallervorden-Spatz syndrome), prion diseases, ataxia telangiectatica, Meige's syndrome, subacute sclerosing panencephalitis, Gaucher disease as well as other lysosomal storage disorders (including Kufor-Rakeb syndrome and Sanfilippo syndrome) and rapid eye movement (REM) sleep behavior disorder. (Jellinger, Mov Disord 2003, 18 Suppl. 6, S2-12; Galvin et al., JAMA Neurology 2001, 58 (2), 186-190; Kovari et al., Acta Neuropathol. 2007, 114(3), 295-8; Saito et al., J Neuropathol Exp Neurol. 2004, 63(4), 323-328; McKee et al., Brain, 2013, 136(Pt 1), 43-64; Puschmann et al., Parkinsonism Relat Disord 2012, 18S1, S24-S27; Usenovic et al., J Neurosci. 2012, 32(12), 4240-4246; Winder-Rhodes et al., Mov Disord. 2012, 27(2), 312-315; Ferman et al., J Int Neuropsychol Soc. 2002, 8(7), 907-914). Preferably, the compounds of formula (I) are suitable for use in the diagnosis of PD.

In the methods of diagnosing a disorder or abnormality associated with alpha-synuclein aggregates, such as PD, or a preclinical state thereof in a subject, the method comprises:
a) administering to the subject a diagnostically effective amount of a compound of formula (I);
b) allowing the compound of formula (I) to distribute into the sample or a specific body part or body area (such as brain tissue, or body fluids such as cerebrospinal fluid (CSF)); and
c) imaging the sample or a specific body part or body area, wherein an increase in binding of the compound of formula (I) to the sample or a specific body part or body area compared to a normal control level of binding indicates that the subject is suffering from or is at risk of developing a disorder or abnormality associated with alpha-synuclein aggregates.

The compounds of formula (I) can be used for imaging of alpha-synuclein aggregates in any sample or a specific body part or body area (for example in the enteric nervous system, gut, eyes and heart) of a patient which is suspected to contain alpha-synuclein aggregates. The compounds of formula (I) are able to pass the blood-brain barrier. Consequently, they are particularly suitable for imaging of alpha-synuclein aggregates in the brain, as well as in body fluids such as cerebrospinal fluid (CSF).

In diagnostic applications, the compound of formula (I) is preferably administered in a diagnostic composition comprising the compound of formula (I). A "diagnostic composition" is defined in the present invention as a composition comprising one or more compounds of formula (I) a form suitable for administration to a patient, e.g., a mammal such as a human, and which is suitable for use in the diagnosis of the specific disorder or anormality at issue. Preferably a diagnostic composition further comprises a physiologically acceptable carrier, diluent, adjuvant or excipient. Administration is preferably carried out as defined below, more preferably by injection of the composition as an aqueous solution. Such a composition may optionally contain further ingredients such as buffers; pharmaceutically acceptable solubilisers (e.g., cyclodextrins or surfactants such as Pluronic, Tween or phospholipids); and pharmaceutically acceptable stabilisers or antioxidants (such as ascorbic acid, gentisic acid or para-aminobenzoic acid). The dose of the compound of formula (I) will vary depending on the exact compound to be administered, the weight of the patient, and other variables as would be apparent to a physician skilled in the art.

While it is possible for the compounds of formula (I) to be administered alone, it is preferable to formulate them into a diagnostic composition in accordance with standard pharmaceutical practice. Thus, the invention also provides a diagnostic composition which comprises a diagnostically effective amount of a compound of formula (I) optionally in combination with a pharmaceutically acceptable carrier, diluent, adjuvant and/or excipient.

Pharmaceutically acceptable excipients are well known in the pharmaceutical art, and are described, for example, in Remington's Pharmaceutical Sciences, 15th Ed., Mack Publishing Co., New Jersey (1975). The pharmaceutical excipient can be selected with regard to the intended route of administration and standard pharmaceutical practice. The excipient must be acceptable in the sense of being not deleterious to the recipient thereof.

Pharmaceutically useful excipients that may be used in the formulation of the diagnostic composition of the present invention may comprise, for example, carriers, vehicles, diluents, solvents such as monohydric alcohols such as ethanol, isopropanol and polyhydric alcohols such as glycols and edible oils such as soybean oil, coconut oil, olive oil, safflower oil cottonseed oil, oily esters such as ethyl oleate, isopropyl myristate, binders, adjuvants, solubilizers, thickening agents, stabilizers, disintegrants, glidants, lubricating agents, buffering agents, emulsifiers, wetting agents, suspending agents, sweetening agents, colorants, flavors, coating agents, preservatives, antioxidants, processing agents, drug delivery modifiers and enhancers such as calcium phosphate, magnesium stearate, talc, monosaccharides, disaccharides, starch, gelatin, cellulose, methylcellulose, sodium carboxymethyl cellulose, dextrose, hydroxypropyl-β-cyclodextrin, polyvinylpyrrolidone, low melting waxes, and ion exchange resins.

The routes for administration (delivery) of the compounds of formula (I) include, but are not limited to, one or more of: oral (e. g. as a tablet, capsule, or as an ingestible solution), topical, mucosal (e. g. as a nasal spray or aerosol for inhalation), nasal, parenteral (e. g. by an injectable form), gastrointestinal, intraspinal, intraperitoneal, intramuscular, intravenous, intrauterine, intraocular, intradermal, intracranial, intratracheal, intravaginal, intracerebroventricular, intracerebral, subcutaneous, ophthalmic (including intravitreal or intracameral), transdermal, rectal, buccal, epidural and sublingual.

For example, the compounds can be administered orally in the form of tablets, capsules, ovules, elixirs, solutions or suspensions, which may contain flavoring or coloring agents, for immediate-, delayed-, modified-, sustained-, pulsed- or controlled-release applications.

The tablets may contain excipients such as microcrystalline cellulose, lactose, sodium citrate, calcium carbonate, dibasic calcium phosphate and glycine, disintegrants such as starch (preferably corn, potato or tapioca starch), sodium starch glycolate, croscarmellose sodium and certain complex silicates, and granulation binders such as polyvinylpyrrolidone, hydroxypropylmethylcellulose (HPMC), hydroxypropylcellulose (HPC), sucrose, gelatin and acacia. Additionally, lubricating agents such as magnesium stearate, stearic acid, glyceryl behenate and talc may be included. Solid compositions of a similar type may also be employed as fillers in gelatin capsules. Preferred excipients in this regard include lactose, starch, a cellulose, milk sugar or high molecular weight polyethylene glycols. For aqueous suspensions and/or elixirs, the agent may be combined with various sweetening or flavoring agents, coloring matter or dyes, with emulsifying and/or suspending agents and with diluents such as water, ethanol, propylene glycol and glycerin, and combinations thereof.

Preferably, in diagnostic applications, the compounds of formula (I) are administered parenterally. If the compounds of formula (I) are administered parenterally, then examples of such administration include one or more of: intravenously (iv), intraarterially, intraperitoneally, intrathecally, intraventricularly, intraurethrally, intrasternally, intracranially, intramuscularly or subcutaneously administering the compounds; and/or by using infusion techniques. For parenteral administration, the compounds are best used in the form of a sterile aqueous solution which may contain other substances, for example, enough salts or glucose to make the solution isotonic with blood. The aqueous solutions should be suitably buffered (preferably to a pH of from 3 to 9), if necessary. The preparation of suitable parenteral formulations under sterile conditions is readily accomplished by standard pharmaceutical techniques well known to those skilled in the art.

As indicated, the compounds of formula (I) can be administered intranasally or by inhalation and are conveniently delivered in the form of a dry powder inhaler or an aerosol spray presentation from a pressurized container, pump, spray or nebulizer with the use of a suitable propellant, e. g. dichlorodifluoromethane, trichlorofluoromethane, dichlorotetrafluoroethane, a hydrofluoroalkane such as 1,1,1,2-tetrafluoroethane (HFA134AT) or 1,1,1,2,3,3,3-heptafluoropropane (HFA 227EA), carbon dioxide or other suitable gas. In the case of a pressurized aerosol, the dosage unit may be determined by providing a valve to deliver a metered amount. The pressurized container, pump, spray or nebulizer may contain a solution or suspension of the active compound, e. g. using a mixture of ethanol and the propellant as the solvent, which may additionally contain a lubricant, e. g. sorbitan trioleate. Capsules and cartridges (made, for example, from gelatin), for use with an inhaler or insufflator may be formulated to contain a powder mix of the compound and a suitable powder base such as lactose or starch.

Alternatively, the compounds of formula (I) can be administered in the form of a suppository or pessary, or it may be applied topically in the form of a gel, hydrogel, lotion, solution, cream, ointment or dusting powder. The compounds of formula (I) may also be dermally or transdermally administered, for example, by the use of a skin patch.

They may also be administered by the pulmonary or rectal routes. They may also be administered by the ocular route. For ophthalmic use, the compounds can be formulated as micronized suspensions in isotonic, pH was adjusted, sterile saline, or, preferably, as solutions in isotonic, pH was adjusted, sterile saline, optionally in combination with a preservative such as a benzylalkonium chloride. Alternatively, they may be formulated in an ointment such as petrolatum.

For application topically to the skin, the compounds of formula (I) can be formulated as a suitable ointment containing the active compound suspended or dissolved in, for example, a mixture with one or more of the following: mineral oil, liquid petrolatum, white petrolatum, propylene glycol, emulsifying wax and water. Alternatively, they can be formulated as a suitable lotion or cream, suspended or dissolved in, for example, a mixture of one or more of the following: mineral oil, sorbitan monostearate, a polyethylene glycol, liquid paraffin, polysorbate 60, cetyl esters wax, cetearyl alcohol, 2-octyldodecanol, benzyl alcohol and water.

Typically, a physician will determine the actual dosage which will be most suitable for an individual subject. The specific dose level and frequency of dosage for any particular individual may be varied and will depend upon a variety of factors including the activity of the specific compound employed, the metabolic stability and length of action of that compound, the age, body weight, general health, sex, diet, mode and time of administration, rate of excretion, drug combination, the severity of the particular condition, and the individual undergoing diagnosis.

Generally, the dose could preferably lie in the range 0.001 µg/kg to 10 µg/kg, preferably 0.01 µg/kg to 1.0 µg/kg. The dose will depend on the route of administration. It will be appreciated that it may be necessary to make routine variations to the dosage depending on the age and weight of the patient as well as the severity of the disorder or anormality. The precise dose and route of administration will ultimately be at the discretion of the attendant physician or veterinarian.

The diagnostic compositions of the invention can be produced in a manner known per se to the skilled person as described, for example, in Remington's Pharmaceutical Sciences, 15th Ed., Mack Publishing Co., New Jersey (1975).

For instance, the compounds of formula (I) can be employed in a liposomal composition as described in WO2016057812A1 which comprises a compound of formula (I) as a ligand for use in the selective detection of disorders and abnormalities associated with alpha-synuclein aggregates by nonradioactive magnetic resonance imaging (MRI).

The compounds of formula (I) are useful as an *in vitro* analytical reference or an *in vitro* screening tool. They are also useful in *in vivo* diagnostic methods.

The compounds of formula (I) can also be provided in the form of a mixture comprising a compound of formula (I) and at least one compound selected from an imaging agent different from the compound of formula (I), a pharmaceutically acceptable carrier, a diluent and an excipient. The imaging agent different from the compound of formula (I) is preferably present in a diagnostically effective amount. More preferably the imaging agent different from the compound of formula (I) is an abeta or Tau imaging agent.

Diagnosis of a disorder or abnormality associated with alpha-synuclein aggregates or of a preclinical state to a disorder or abnormality associated with alpha-synuclein aggregates in a patient may be achieved by detecting the specific binding of a compound of formula (I) to the alpha-synuclein aggregates in a sample or *in situ,* which includes:
(a) bringing the sample or a specific body part or body area suspected to contain the alpha-synuclein aggregates into contact with a compound of formula (I) which binds the alpha-synuclein aggregates,
(b) allowing the compound of formula (I) to bind to the alpha-synuclein aggregates to form a compound/(alpha-synuclein aggregate) complex (hereinafter "compound/(alpha-synuclein aggregate) complex" will be abbreviated as "compound/protein aggregate complex"),
(c) detecting the formation of the compound/protein aggregate complex,
(d) optionally correlating the presence or absence of the compound/protein aggregate complex with the presence or absence of alpha-synuclein aggregates in the sample, specific body part or area, and
(e) optionally comparing the amount of the compound/protein aggregate complex to a normal control value, wherein an increase in the amount of the compound/protein aggregate complex compared to a normal control value may indicate that the patient is suffering from or is at risk of developing a disorder or abnormality associated with alpha-synuclein aggregates.

The embodiment in which the compound is brought into contact with a sample is claimed. The embodiment in which the compound is brought into contact with a specific body part or body area is not claimed.

The compound of formula (I) can be brought into contact with the sample or the specific body part or body area suspected to contain the alpha-synuclein aggregates by a suitable method. In *in vitro* methods the compound of formula (I) and a liquid sample can be simply mixed. In *in vivo* tests the compound of formula (I) is typically administered to the patient by any suitable means. These routes of administration include, but are not limited to, one or more of: oral (e. g. as a tablet, capsule, or as an ingestible solution), topical, mucosal (e. g. as a nasal spray or aerosol for inhalation), nasal, parenteral (e. g. by an injectable form), gastrointestinal, intraspinal, intraperitoneal, intramuscular, intravenous, intrauterine, intraocular, intradermal, intracranial, intratracheal, intravaginal, intracerebroventricular, intracerebral, subcutaneous, ophthalmic (including intravitreal or intracameral), transdermal, rectal, buccal, epidural and sublingual. In some instances, parenteral administration can be preferred.

After the sample or a specific body part or body area has been brought into contact with the compound of formula (I), the compound is allowed to bind to the alpha-synuclein aggregates. The amount of time required for binding will depend on the type of test (e.g., *in vitro* or *in vivo*) and can be determined by a person skilled in the field by routine experiments.

The compound which is bound to the alpha-synuclein aggregates, can be subsequently detected by any appropriate method. The specific method chosen will depend on the detectable label which has been chosen. Examples of possible methods include, but are not limited to, a fluorescence imaging technique or a nuclear imaging technique such as positron emission tomography (PET), single photon emission computed tomography (SPECT), magnetic resonance imaging (MRI), and contrast-enhanced magnetic resonance imaging (MRI). These have been described and enable visualization of amyloid biomarkers. The fluorescence imaging technique and/or nuclear imaging technique can be employed for monitoring and/or visualizing the distribution of the detectably labeled compound within the sample or a specific body part or body area.

The presence or absence of the compound/protein aggregate complex is then optionally correlated with the presence or absence of alpha-synuclein aggregates in the sample or specific body part or area. Finally, the amount of the compound/protein aggregate complex can be compared to a normal control value which has been determined in a sample or a specific body part or body area of a healthy subject, wherein an increase in the amount of the compound/protein aggregate complex compared to a normal control value may indicate that the patient is suffering from or is at risk of developing a disorder or abnormality associated with alpha-synuclein aggregates.

The present invention also relates to a method of determining the amount of alpha-synuclein aggregates in a sample or a specific body part or body area. This method comprises the steps of:
(a) providing a sample or a specific body part or body area;
(b) testing the sample or a specific body part or body area for the presence of alpha-synuclein aggregates with a compound of the present invention;
(c) determining the amount of compound bound to the alpha-synuclein aggregates; and
(d) calculating the amount of alpha-synuclein aggregates in the sample or a specific body part or body area.
The embodiment in which the compound is brought into contact with a sample is claimed. The embodiment in which the compound is brought into contact with a specific body part or body area is not claimed. The sample or a specific body part or body area can be tested for the presence of alpha-synuclein aggregates with a compound of formula (I) by bringing the sample or a specific body part or body area into contact with a compound of formula (I), allowing the compound of formula (I) to bind to the alpha-synuclein aggregates to form a compound/protein aggregate complex and detecting the formation of the compound/protein aggregate complex as explained above.

Monitoring minimal residual disorder in a patient suffering from a disorder or abnormality associated with alpha-synuclein aggregates who has been treated with a medicament with a compound of formula (I) may be achieved by:
(a) bringing a sample or a specific body part or body area suspected to contain alpha-synuclein aggregates into contact with a compound of formula (I);
(b) allowing the compound to bind to the alpha-synuclein aggregates to form a compound/protein aggregate complex;
(c) detecting the formation of the compound/protein aggregate complex;
(d) optionally correlating the presence or absence of the compound/protein aggregate complex with the presence or absence of alpha-synuclein aggregates in the sample or specific body part or body area; and
(e) optionally comparing the amount of the compound/protein aggregate complex to a normal control value, wherein an increase in the amount of the aggregate compared to a normal control value may indicate that the patient may still suffer from a minimal residual disease.
The embodiment in which the compound is brought into contact with a sample is claimed. The embodiment in which the compound is brought into contact with a specific body part or body area is not claimed. How steps (a) to (e) can be conducted has already been explained above.

In the method for monitoring minimal residual disorder, the method can further comprise steps (i) to (vi) before step (a):
(i) bringing a sample or specific body part or body area suspected to contain alpha-synuclein aggregates into contact with a compound of formula (I), which compound specifically binds to the alpha-synuclein aggregates;
(ii) allowing the compound to bind to the alpha-synuclein aggregates to form a compound/(alpha-synuclein aggregate) complex;
(iii) detecting the formation of the compound/(alpha-synuclein aggregate) complex;
(iv) correlating the presence or absence of the compound/(alpha-synuclein aggregate) complex with the presence or absence of alpha-synuclein aggregates in the sample specific body part or body area;
(v) optionally comparing the amount of the compound/(alpha-synuclein aggregate) complex to a normal control value; and
(vi) treating the patient with the medicament.
The embodiment in which the compound is brought into contact with a sample is claimed. The embodiment in which the compound is brought into contact with a specific body part or body area is not claimed. Optionally the method can further comprise step (A) after step (d) or step (e):
(A) comparing the amount of the compound/(alpha-synuclein aggregate) complex determined in step (iv) to the amount of the compound/(alpha-synuclein aggregate) complex determined in step (d).

In order to monitor minimal residual disorder over time, steps (a) to (c) and optionally steps (d) and (e) of the method of monitoring minimal residual disorder can be repeated one or more times.

In the method for monitoring minimal residual disorder the amount of the compound/protein aggregate complex can be optionally compared at various points of time during the treatment, for instance, before and after onset of the treatment or at various points of time after the onset of the treatment. A change, especially a decrease, in the amount of the compound/protein aggregate complex may indicate that the residual disorder is decreasing.

Predicting responsiveness of a patient suffering from a disorder or abnormality associated with alpha-synuclein aggregates and being treated with a medicament can be achieved by
(a) bringing a sample or a specific body part or body area suspected to contain alpha-synuclein aggregates into contact with a compound of formula (I);
(b) allowing the compound to bind to the alpha-synuclein aggregates to form a compound/protein aggregate complex;
(c) detecting the formation of the compound/protein aggregate complex;
(d) optionally correlating the presence or absence of the compound/protein aggregate complex with the presence or absence of alpha-synuclein aggregates in the sample or specific body part or body area; and
(e) optionally comparing the amount of the compound/protein aggregate complex to a normal control value.
The embodiment in which the compound is brought into contact with a sample is claimed. The embodiment in which the compound is brought into contact with a specific body part or body area is not claimed. How steps (a) to (e) can be conducted has already been explained above.

In the method for predicting the responsiveness, the method can further comprise steps (i) to (vi) before step (a):
(i) bringing a sample or specific body part or body area suspected to contain alpha-synuclein aggregates into contact with the compound as a compound of formula (I), which compound specifically binds to the alpha-synuclein aggregates;
(ii) allowing the compound to bind to the alpha-synuclein aggregates to form a compound/(alpha-synuclein aggregate) complex;
(iii) detecting the formation of the compound/(alpha-synuclein aggregate) complex;
(iv) correlating the presence or absence of the compound/(alpha-synuclein aggregate) complex with the presence or absence of alpha-synuclein aggregates in the sample or specific body part or body area;
(v) optionally comparing the amount of the compound/(alpha-synuclein aggregate s) complex to a normal control value; and
(vi) treating the patient with the medicament.

The embodiment in which the compound is brought into contact with a sample is claimed. The embodiment in which the compound is brought into contact with a specific body part or body area is not claimed. In the present invention, the step of treating the patient is not covered by the claims.

Optionally the method can further comprise step (A) after step (d) or step (e):
(A) comparing the amount of the compound/(alpha-synuclein aggregate) complex determined in step (iv) to the amount of the compound/(alpha-synuclein aggregate) complex determined in step (d).

In order to determine the responsiveness over time, steps (a) to (c) and optionally steps (d) and (e) of the method of predicting responsiveness can be repeated one or more times.

In the method for predicting responsiveness the amount of the compound/protein aggregate complex can be optionally compared at various points of time during the treatment, for instance, before and after onset of the treatment or at various points of time after the onset of the treatment. A change, especially a decrease, in the amount of the compound/protein aggregate complex may indicate that the patient has a high potential of being responsive to the respective treatment.

Optionally, the diagnostic composition can be used before, during and after, surgical procedures (e.g. deep brain stimulation (DBS)) and non-invasive brain stimulation (such as repetitive transcranial magnetic stimulation (rTMS)), for visualizing alpha-synuclein aggregates before, during and after such procedures. Surgical techniques, including DBS, improve advanced symptoms of PD on top of the best currently used medical therapy. During the past 2 decades, rTMS has been closely examined as a possible treatment for PD (Ying-hui Chou et al. JAMA Neurol. 2015 April 1; 72(4): 432-440).

In a further embodiment of the invention, the diagnostic composition can be used in a method of collecting data for monitoring residual disorder in a patient suffering from a disorder or abnormality associated with alpha-synuclein aggregates who has been treated with a surgical procedure or non-invasive brain stimulation procedure, wherein the method comprises:
(a) bringing a sample or specific body part or body area suspected to contain alpha-synuclein aggregates into contact with a compound of formula (I), which compound specifically binds to the alpha-synuclein aggregates;
(b) allowing the compound to bind to the alpha-synuclein aggregates to form a compound/(alpha-synuclein aggregate) complex;
(c) detecting the formation of the compound/(alpha-synuclein aggregate) complex;
(d) optionally correlating the presence or absence of the compound/(alpha-synuclein aggregate) complex with the presence or absence of alpha-synuclein aggregates in the sample or specific body part or body area; and
(e) optionally comparing the amount of the compound/(alpha-synuclein aggregate) complex to a normal control value.
The embodiment in which the compound is brought into contact with a sample is claimed. The embodiment in which the compound is brought into contact with a specific body part or body area is not claimed. In the methods described above, "alpha-synuclein aggregates" include, but are not limited to, Lewy bodies and/or Lewy neurites.

In the methods described above, "a sample or specific body part or body area" is preferably a sample or a specific body part obtained from a patient, e.g., a tissue and/or a body fluid. It is understood that the sample or specific body part or body area should be representative of the sample or specific body part or body area under investigation.

A compound of formula (I) can also be incorporated into a test kit for detecting alpha-synuclein protein aggregates including, but not limited to, Lewy bodies and/or Lewy neurites. The test kit typically comprises a container holding one or more compounds of formula (I) and instructions for using the compound for the purpose of binding to alpha-synuclein aggregates including, but not limited to, Lewy bodies and/or Lewy neurites to form a compound/protein aggregate complex and detecting the formation of the compound/protein aggregate complex such that presence or absence of the compound/protein aggregate complex correlates with the presence or absence of the alpha-synuclein aggregates including, but not limited to, Lewy bodies and/or Lewy neurites.

The term "test kit" refers in general to any diagnostic kit known in the art. More specifically, the latter term refers to a diagnostic kit as described in Zrein et al., Clin. Diagn. Lab. Immunol., 1998, 5, 45-49.

### Radiopharmaceutical preparations

The compounds of formula (II) can also be employed in kits for the preparation of radiopharmaceutical preparations. Due to the radioactive decay, the radiopharmaceuticals are usually prepared immediately before use. The kit comprises a precursor of the compound of formula (I) and an agent which reacts with the precursor to introduce a radioactive label to the compound of formula (I). The precursor of the compound of formula (I) can, for example, be a compound having the formula (II). The agent can be an agent which introduces a radioactive label such as ¹⁸F.

Preferred compounds are illustrated in the examples.

The compounds of the present invention can be synthesized by one of the general methods shown in the following schemes. These methods are only given for illustrative purposes and should not to be construed as limiting.

### General synthetic schemes for the preparation of building blocks of this invention:

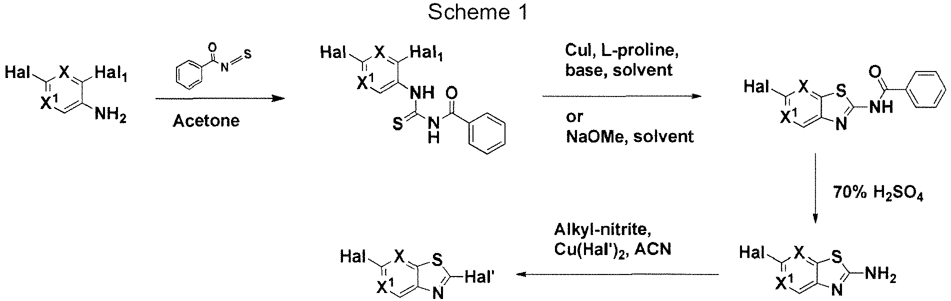

Commercially available halogenated aminopyridines are reacted with benzoyl isothiocyanate in a solvent such as acetone to afford the desired benzoyl thiourea pyridine derivatives after purification. Then, the thioureas were cyclized using a copper catalyst. Alternatively, the cyclization can processed using sodium methoxide in a suitable solvent. Deprotection of benzoyl groups was achieved using acidic conditions. Finally, the amino groups were transformed into halogen using standard conditions.

### General synthetic scheme for the preparation of compounds of this invention:

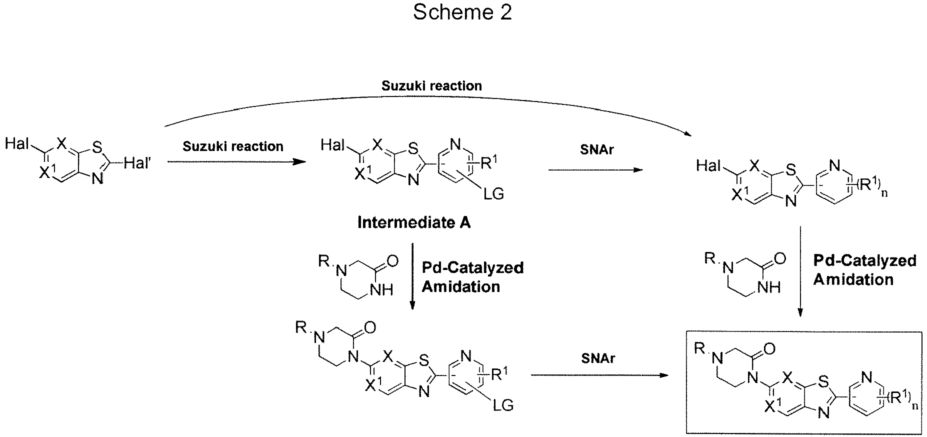

Bicyclic building blocks (Hal, Hal' = Br, Cl) were treated with pyridine boronic acids or esters in a solvent via palladium-catalyzed cross-coupling (Suzuki reaction) conditions to afford the desired derivative after purification. The intermediate A can be further functionalized using SNAr conditions followed by palladium catalyzed amidation or Ullmann reaction, for introducing the oxopiperazine derivative, to deliver desired compounds. Alteratively, the introduction of the oxopiperazine derivative can be performed before the SNAr rection.

### General synthesis of ¹⁸F-labeled compounds of the present invention

Compounds having the formula (I) which are labeled by ¹⁸F can be prepared by reacting a precursor compound, as described below, with an ¹⁸F-fluorinating agent, so that the LG comprised in the precursor compound is replaced by ¹⁸F.

Any suitable ¹⁸F-fluorinating agent can be employed. Typical examples include H¹⁸F, alkali or alkaline earth ¹⁸F-fluorides (e.g., K¹⁸F, Rb¹⁸F, Cs¹⁸F, and Na¹⁸F). Optionally, the ¹⁸F-fluorination agent can be used in combination with a chelating agent such as a cryptand (e.g.: 4,7,13,16,21,24-hexaoxa-1,10-diazabicyclo[8.8.8]-hexacosane - Kryptofix^{®}) or a crown ether (e.g.: 18-crown-6). Alternatively, the ¹⁸F-fluorinating agent can be a tetraalkylammonium salt of ¹⁸F or a tetraalkylphosphonium salt of ¹⁸F; e.g., tetra(C₁₋₆ alkyl)ammonium salt of ¹⁸F or a tetra(C₁₋₆ alkyl)phosphonium salt of ¹⁸F. Preferably, the ¹⁸F-fluorination agent is K¹⁸F, H¹⁸F, Cs¹⁸F, Na¹⁸F or tetrabutylammonium [¹⁸F]fluoride.

### General synthetic scheme for the preparation of precursor compounds

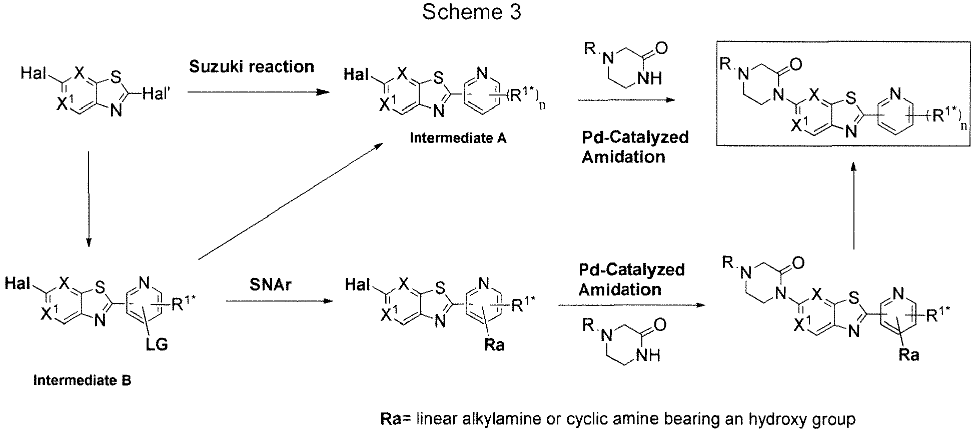

Bicyclic building blocks (Hal, Hal' = Br, Cl) were treated with pyridine boronic acids or esters in a solvent via palladium-catalyzed cross-coupling conditions (Suzuki reaction) to afford the desired intermediate A after purification. The intermediate A can be further functionalized using amidation reaction via palladium-catalyzed cross-coupling conditions or Ullmann conditions, for introducing the oxopiperazine derivative, to afford the desired precursor compounds containing a leaving group (LG) after purification. Alternative route can be used by reacting pyridine boronic acids or esters bearing a leaving group and bicyclic building blocks (Hal, Hal' = Br, Cl) to afford intermediate B. Intermediate B can be further functionalized to provide the intermediate A. A third route consists of introducing Ra substituent via SNAr reaction followed by amidation reaction using palladium-catalyzed cross-coupling conditions or Ullmann conditions, for introducing the oxopiperazine derivative. Finally, LG group is introduced on Ra group to afford the desired precursor compounds containing a leaving group (e.g. the hydroxyl group in the Ra substituent can be transformed into a sulfonate LG).

### General synthetic scheme for the preparation of ¹⁸F-labeled compounds

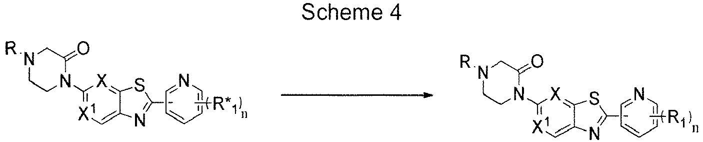

The reactions take place in the presence of a fluorinating agent and typically a solvent.

¹⁸F labeled compounds can be prepared by reacting the precursor compounds containing a LG with an ¹⁸F-fluorinating agent, so that the LG is replaced by ¹⁸F. The reagents, solvents and conditions which can be used for the ¹⁸F-fluorination are well-known to a skilled person in the field (L. Cai, S. Lu, V. Pike, Eur. J. Org. Chem 2008, 2853-2873; J. Fluorine Chem., 27 (1985):177-191; Coenen, Fluorine-18 Labeling Methods: Features and Possibilities of Basic Reactions, (2006), in: Schubiger P.A., Friebe M., Lehmann L., (eds), PET-Chemistry - The Driving Force in Molecular Imaging. Springer, Berlin Heidelberg, pp.15-50). Preferably, the solvents used in the ¹⁸F-fluorination are DMF, DMSO, acetonitrile, DMA, or mixtures thereof, preferably the solvent is acetonitrile, DMSO.

Although the reaction is shown above with respect to ¹⁸F as a radioactive label, other radioactive labels can be introduced following similar procedures.

The invention is illustrated by the following examples which, however, should not be construed as limiting.

### Examples

All reagents and solvents were obtained from commercial sources and used without further purification. Proton (¹H) spectra were recorded on a Bruker DRX-400 MHz NMR spectrometer or on a Bruker AV-400 MHz NMR spectrometer in deuterated solvents. Mass spectra (MS) were recorded on an Advion CMS mass spectrometer. Chromatography was performed using silica gel (Fluka: Silica gel 60, 0.063-0.2 mm) and suitable solvents as indicated in the specific examples. Flash purification was conducted with a Biotage Isolera One flash purification system using HP-Sil or KP-NH SNAP cartridges (Biotage) and the solvent gradient indicated in the specific examples. Thin layer chromatography (TLC) was carried out on silica gel plates with UV detection.

Although some of the present examples do not indicate that the respective compounds were detectably labeled, it is understood that corresponding detectably labeled compounds are intended and can be easily prepared, e.g., by using detectably labeled starting materials, such as starting materials containing C(³H)₃, (¹¹C)H₃ or ¹⁸F.

### Preparative Example 1

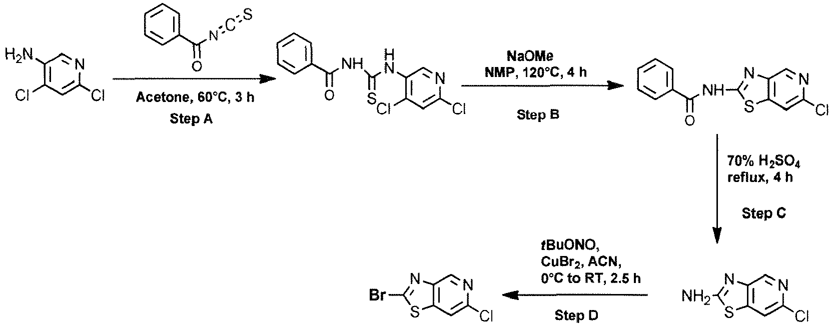

### Step A:

A solution of of 4,6-dichloropyridin-3-amine (5.0 g, 30.67 mmol) and benzoyl isothiocyanate (4.5 mL, 33.74 mmol, 1.1 eq) in acetone (75 mL, 15 vol) was stirred at 60°C for 3 hours, the reaction was monitored by TLC. The solvent was evaporated and the solid was filtered, washed with n-hexane (100 mL) and dried to give the desired product as 8.0 g of an off-white solid with 80 % yield.
¹H-NMR (400 MHz, DMSO-d₆) δ 12.38 (s, 1H), 12.00 (s, 1H), 8.74 (s, 1H), 8.01 (d, 1H), 7.97 (s, 1H), 7.68 (t, 1H), 7.55 (t, 2H).
MS (ESI); m/z = 326.2 [M+H]⁺

### Step B:

To a solution of *N*-((4,6-dichloropyridin-3-yl)carbamothioyl)benzamide (9.0 g, 27.69 mmol) in N-methyl-2-pyrrolidone (NMP) (45 mL, 5 vol) was added sodium methoxide (NaOMe) (2.99 g, 55.38 mmol, 2.0 eq), at 0°C. The mixture was then heated to 120°C and stirring was continued for 4 hours. The reaction was monitored by TLC. The reaction mixture was poured into 100 mL cold water and a white precipitate was obtained. The solid was filtered, and washed with water (200 mL) and n-hexane (100 mL). The compound was dried under vacuum for 6 h to give the desired product as 7.0 g of a white solid with 87% yield.
¹H-NMR (400 MHz, DMSO-d₆) δ 8.87 (s, 1H), 8.27 (s, 1H), 8.14 (d, 2H), 7.69 (t, 1H), 7.59 (t, 2H).
MS (ESI); m/z = 289.7 [M+H]⁺

### Step C:

A suspension of *N*-(6-chlorothiazolo[4,5-c]pyridin-2-yl)benzamide (5.0 g, 17.30 mmol) in 70% H₂SO₄ (15.0 mL, 3.0 vol) was heated at 110°C for 4 hours. The reaction mixture was cooled to room temperature and the reaction mixture was slowly poured into 200 mL of cold water (0°C). Then, the reaction mixture was adjusted to basic pH by addition of solid 50% aq. NaOH. Then, the compound was extracted with EtOAc (6 x 100 mL). The combined organic layers were dried over with Na₂SO₄ and filtered, then the solvent was removed to give the desired product as 1.8 g of a light yellow solid with 56% yield.
¹H-NMR (400 MHz, DMSO-d₆) δ 7.88 (s, 1H), 7.44 (s, 1H), 5.82 (br.s, 2H).
MS (ESI); m/z = 186.4 [M+H]⁺

### Step D:

To a suspension of 6-chlorothiazolo[4,5-c]pyridin-2-amine (2.5 g, 13.51 mmol) in acetonitrile (33.0 mL, 13.0 vol) at 0°C was added *tert*-butyl nitrite (2.4 mL, 20.27 mmol, 1.5 eq) over a period of 10 min with a syringe. Then, copper(II) bromide (4.5 g, 20.27 mmol, 1.5 eq) was added portionwise. After 30 minutes at 0°C, the reaction mixture was allowed to warm to room temperature for 2.5 hours. The progress of the reaction was monitored by TLC. After completion of the reaction, the solvent was evaporated and the reaction mixture was diluted with water (100 mL) and 5% methanol/dichloromethane (3 x 100 mL). The combined organics were washed with brine (50 mL), dried over Na₂SO₄, filtered and concentrated under reduced pressure. The crude compound was purified by silica gel (60-120) column chromatography, eluted with 1% methanol/dichloromethane to afford the title compound as 2.0 g of a white solid with 60 % yield.
¹H NMR (400 MHz, DMSO-d₆) δ 9.09 (s, 1H), 8.34 (s, 1H).
MS (ESI); m/z = 249.3 [M+H]⁺

### Preparative Example 2

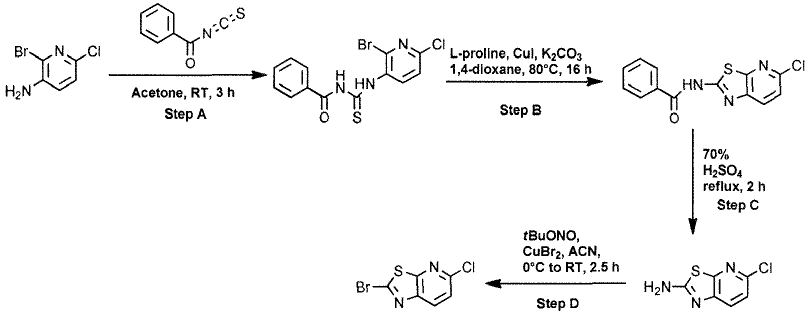

### Step A:

A solution of 2-bromo-6-chloropyridin-3-amine (30.0 g, 144.46 mmol) and benzoyl isothiocyanate (23.4 ml, 173.52 mmol, 1.2 eq) in acetone (600 mL, 20 vol) was stirred at room temperature for 3 hours, the reaction was monitored by the TLC. The solvent was evaporated and the solid was filtered and washed with n-hexane (500 mL) and dried to give the desired product as 37.0 g of an off-white solid with 69 % yield.
¹H-NMR (500 MHz, CDCl₃) δ 9.18 (brs, 1H), 8.84 (d, 1H), 7.93 (d, 2H), 7.69 (t, 1H), 7.59-7.56 (m, 2H), 7.37 (d, 1H).
MS (ESI); m/z = 369.8 [M-H]⁺

### Step B:

To a solution of *N*-((2-bromo-6-chloropyridin-3-yl)carbamothioyl)benzamide (36.0 g, 97.20 mmol) in 1,4-dioxane (540 mL, 15 vol) was added potassium carbonate (20.1 g, 145.81 mmol, 1.5 eq), L-proline (2.20 g, 19.45 mmol, 0.2 eq) and copper(I) iodide (3.70 g, 19.45 mmol, 0.2 eq). Then, the reaction mixture was stirred at 80°C for 16 hours, the reaction was monitored by TLC. The reaction mixture was poured into 1.0 L of water and 1.0 L of aqueous saturated solution of NH₄Cl. The suspension was stirred at room temperature for 1 hour. The solid was filtered, washed with aqueous saturated solution of NH₄Cl (2 x 500 mL), water (2 x 500mL) and dried to give the desired product as 25.2 g of an off-white solid with 89% yield.
¹H-NMR (500 MHz, CDCl₃) δ 10.15 (br.s, 1H), 7.99 (d, 2H), 7.74 (d, 1H), 7.66 (t,1H), 7.55 (t, 2H), 7.34 (d, 1H).

### Step C:

A suspension of *N*-(5-chlorothiazolo[5,4-b]pyridin-2-yl)benzamide (5.0 g, 17.30 mmol) in 70% H₂SO₄ (15.0 mL, 3.0 vol) was heated at 120°C for 2 hours. The reaction mixture was cooled to room temperature and the reaction mixture was slowly poured into 300 mL of cold water (0°C). Then, the reaction mixture was adjusted to basic pH by addition of solid 50% aq. NaOH. Then, the compound was extracted with EtOAc (6 x 200 mL). The combined organic layers were dried over Na₂SO₄ and filtered, then the solvent was removed to give the desired product as 1.5 g of a light yellow solid with 46% yield.
¹H-NMR (500 MHz, DMSO-d₆) δ 7.92 (br.s, 2H), 7.66 (d, 1H), 7.31 (d, 1H).

### Step D:

To a suspension of 5-chlorothiazolo[5,4-b]pyridin-2-amine (10.0 g, 54.05 mmol) in acetonitrile (125 mL, 12.5 vol) at 0°C was added tert-butyl nitrite (9.6 mL, 81.08 mmol, 1.5 eq) over a period of 10 min with a syringe. Then, copper(II) bromide (14.4 g, 64.81 mmol, 1.2 eq) was added portionwise. After 30 minutes at 0°C, the reaction mixture was allowed to warm to room temperature for 2.5 hours. The progress of the reaction was monitored by TLC. After completion of the reaction, the solvent was evaporated and the reaction mixture was diluted with water (300 mL) and 5% methanol/dichloromethane (3 x 300 mL). The combined organics were washed with brine (300 mL), dried over Na₂SO₄, filtered and concentrated under reduced pressure. The crude compound was purified by silica gel (60-120) column chromatography, eluted with 1% methanol/dichloromethane to afford the title compound as 10.0 g of an off-white solid with 74 %yield.
¹H NMR (500 MHz, DMSO-d6) δ 8.48 (d, 1H), 7.72 (d, 1H).
MS (ESI); m/z = 249.4 [M+H]⁺

### Preparative Example 3

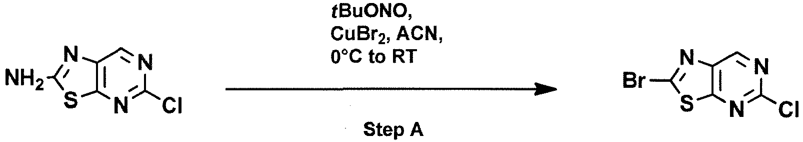

### Step A:

To a suspension of 5-chlorothiazolo[5,4-d]pyrimidin-2-amine prepared according to WO2010008847 (1g, 5.36 mmol) in acetonitrile (20 mL) at 0°C was added tert-butyl nitrite (0.829 g, 8.04 mmol) over 30 min. Then, copper(II) bromide (1.436 g, 6.43 mmol) was added portionwise. After 30 minutes at 0°C, the reaction mixture was allowed to warm to room temperature and stirred for 12 hours. Water (50 mL) and EtOAc (100 mL) were added and the phases were separated. The aqueous layer was extracted twice with EtOAc. The organics were combined, dried over Na₂SO₄ and the solvent was evaporated under reduced pressure. The residue was purified on HP-Sil SNAP cartridges using a Biotage Isolera One purification system employing EtOAc/n-heptane (20/80) to afford the title compound (660 mg, 40%)
¹H NMR (500 MHz, DMSO-d6) δ 9.41 (s, 1H).

### Preparative Example 3A

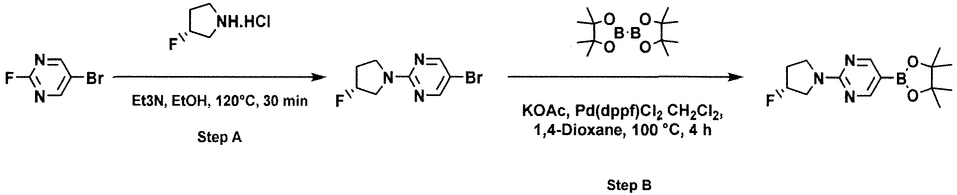

### Step A:

A solution of 2-fluoro-5-bromo-pyrimidine (0.500 g, 2.8248 mmol, 1.0 eq), (R)-3 fluoropyrrolidine hydrochloride (390 mg, 3.1073 mmol) and triethylamine (1.2 mL, 8.4745 mmol) in ethanol (5mL) was heated at 120°C for 30 min under microwave irradiation. Then, the reaction mixture was cooled to room temperature. The reaction mixture was filtered, washed with cold ethanol and pentane, dried under reduced pressure to give the desired product (0.370 g, 53 %).
¹H-NMR (500 MHz, DMSO-d6) δ 8.4 (s, 2H), 5.49-5.37 (m, 1H), 3.80-3.57 (m, 3H), 3.04 (q, 1H), 2.10-2.30 (m, 2H).
MS (ESI); m/z = 245.9 [M+H]⁺

### Step B:

A flask was charged with the bromo compound of step A (370 mg, 1.5040 mmol), KOAc (295 mg, 3.0081 mmol), and bis(pinacolato)diborane (764 mg, 3.0081 mmol) and 1,4-dioxane (18 mL) was degassed with nitrogen for 10 minutes. Then, Pd(dppf)Cl₂.DCM catalyst (123 mg, 0.1504 mmol) was added to the above reaction at room temperature. After being stirred at 100 °C for 4 hours, the reaction mass was cooled to room temperature. Water was added and the aqueous layer was extracted with DCM (3 X 50 mL). The combined organics were evaporated under reduced pressure and the obtained the solid was used to the next step without further purification (600 mg).
MS (ESI); m/z = 294.20 [M+H]⁺

### Preparative Example 4

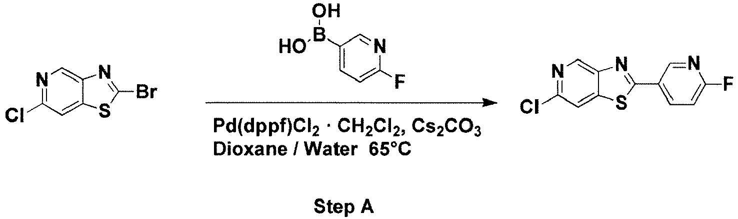

### Step A:

1,4-Dioxane (135 mL) and water (45 mL) were degassed for 10 min. Then, Preparative Example 1 (3.0 g, 0.01202 mol), 6-fluoro pyridine 3-boronic acid (1.86 g, 0.01320 mol), cesium carbonate (7.8 g, 0.02404 mol) and Pd(dppf)Cl₂•dichloromethane complex (980 mg, 0.001202 mol) were added and the reaction mixture was heated at 65°C for 12 h. The organic layer was separated and the aqueous layer was extracted two more times with 5% methanol in dichloromethane [2 x 200 mL]. The combined organic layers were concentrated under reduced pressure. The crude compound was purified by silica gel (100-200) column chromatography, eluted with 5% ethyl acetate in dichloromethane to give the desired product as an off-white solid (2.0 g, 62 %).
¹H-NMR (500 MHz, DMSO-d6) δ 9.0 (s, 1H), 8.70 (t, 1H), 8.56 (d, 1H), 7.75 (d, 1H), 7.47 (d, 1H), 7.68 (t, 1H), 7.55 (t, 2H).
MS (ESI); m/z = 266.02 [M+H]⁺

### Preparative Examples 5 to 7B

Following the Pd-coupling procedure as described in Preparative Example 4, using the dihalogenated starting material and the appropriate boronic acid or ester indicated in the table below, the following compounds were prepared.

**Table 1:**

| Halogenated Starting Material | Boronic acid/ester | Product **Example** | 1. Yield |
|---|---|---|---|
| | | | 2. ¹H-NMR |
| | | | 3. MH⁺(ESI) |
| 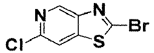 | 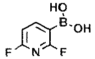 | 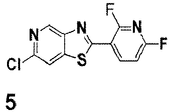 | 1.44 % |
| | | | 2. ¹H NMR (400 MHz, DMSO-d6) δ 9.23 (s, 1H), 9.06 - 8.97 (m, 1H), 8.46 (s, 1H), 7.48 (dd, 1H) |
| | | | 3. NA |
| 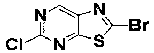 | 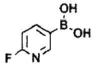 | 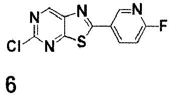 | 1. 11 % |
| | | | 2. ¹H NMR (400 MHz, DMSO-d₆) δ 9.51 (s, 1H), 9.05 (d, 1H), 8.74 (ddd, 1H), 7.50 (dd, 1H) |
| | | | 3. ESI-QTOF-MS: 266.9908 |
| 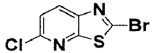 | 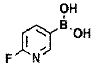 | 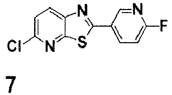 | 1. 49% |
| | | | 2. ¹H NMR (400 MHz, DMSO-d6) δ 9.00 (d, 1H), 8.71-8.68 (m, 1H), 8.56 (d, 1H), 7.75 (d, 1H), 7.46 (dd, 1H) |
| | | | 3. NA |
| 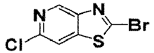 | 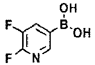 | 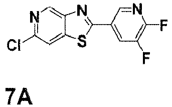 | 1.53 % |
| | | | 2. ¹H NMR (500 MHz, DMSO-d6) δ 9.35 (d, 1H), 8.91 - 8.87 (m, 1H), 8.83 - 8.77 (m, 1H), 8.25 (d, 1H). |
| | | | 3. 282.03 (MH⁻) |
| 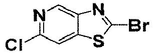 | 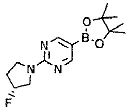 | 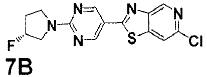 | 1. 50% |
| | | | 2. NA |
| | | | 3. 335.79 |

### Preparative Example 8

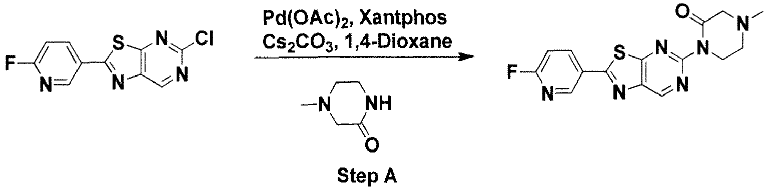

### Step A:

Palladium(II) acetate (0.020 g, 0.090 mmol), 4,5-bis(diphenylphosphino)-9,9-dimethylxanthene (0.078 g, 0.135 mmol), 4-methylpiperazin-2-one (0.061 g, 0.540 mmol), cesium carbonate (0.440 g, 1.350 mmol) and the title compound from Preparative Example 6 (0.120 g, 0.450 mmol) were added to a reaction vial followed by degassed 1,4-dioxane (5 ml). The vial was filled with argon gas and sealed and heated at 120 °C for 45 minutes. The reaction mixture was cooled to room temperature and the residue was taken up with dichloromethane (40 mL) and water (50 mL), the phases were separated and the aqueous phase was extracted again with dichloromethane (50 mL). The organics were combined, dried over Na₂SO₄ and the crude product was purified on a HP-Sil column (biotage) by employing a dichloromethane/methanol gradient (100/0 -> 90/10) to afford the title compound (83 mg, 54%).
MS (ESI); m/z = 345.12 [M+H]⁺
¹H-NMR (400 MHz, CDCl₃) δ 9.53 (s, 1H), 9.04 (d, 1H), 8.73 (ddd, 1H), 7.49 (dd, 1H), 3.98 - 3.83 (m, 2H), 3.22 (s, 2H), 2.87 - 2.66 (m, 2H), 2.33 (s, 3H).

### Preparative Examples 9 and 9A

Following the Pd-coupling procedure as described in Preparative Example 8, except using the dihalogenated starting material and the appropriate amide indicated in the table below, the following compounds were prepared.

**Table 2:**

| Halogenated Starting Material | Amide | Product **Example** | 1. Yield |
|---|---|---|---|
| | | | 2. ¹H-NMR |
| | | | 3. MH⁺(ESI) |
| 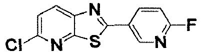 | 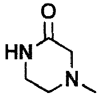 | 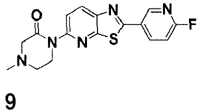 | 1. 76 % |
| | | | 2. NA |
| | | | 3. 344.47 |
| 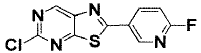 | 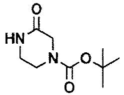 | 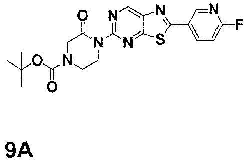 | 1. 66% |
| | | | 2. ¹H NMR (400 MHz, Chloroform-d) δ 9.32 (s, 1H), 8.98 - 8.87 (m, 1H), 8.51 (ddd, 1H), 7.14 (dd, 1H), 4.36 (s, 2H), 4.23 - 4.09 (m, 2H), 3.89 - 3.75 (m, 2H), 1.50 (s, 9H). |
| | | | 3. 431.10 |

### Preparative Example 10

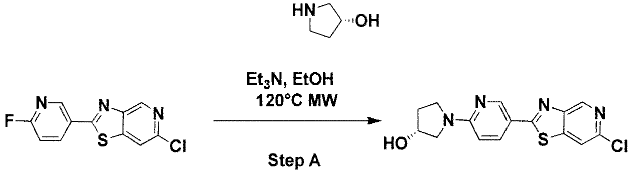

### Step A:

To a microwave tube were added the title compound from Preparative Example 4 (0.3 g, 1.129 mmol), (R)-pyrrolidin-3-ol (0.295 g, 3.39 mmol), ethanol (20 mL), followed by triethylamine (0.315 ml, 2.258 mmol). The tube was sealed and heated at 120 °C for 30 minutes using a Biotage Initiator microwave. The reaction mixture was poured into 1N aqueous NaOH and the suspension was extracted several times with dichloromethane/methanol. The combined organics were dried dried over Na₂SO₄ and concentrated under reduced pressure. The solid was then triturated in methanol and filtered to afford the title compound (0.250 g, 67 %).
¹H NMR (400 MHz, DMSO-d₆) δ 8.98 (s, 1H), 8.88 - 8.74 (m, 1H), 8.31 (s, 1H), 8.22 - 8.06 (m, 1H), 6.71 - 6.56 (m, 1H), 5.04 (s, 1H), 4.43 (s, 1H), 3.71 - 3.51 (m, 4H), 2.16 - 1.83 (m, 2H).
MS (ESI); m/z = 333.16 [M+H]⁺

### Preparative Examples 11 to 14E

Following the procedure described in Preparative Example 10, except using the fluoro derivatives and amines indicated in the table below, the following compounds were prepared.

**Table 3:**

| Fluoro Derivative | Amine | Product **Example** | 1. Yield |
|---|---|---|---|
| | | | 2. ¹H-NMR |
| | | | 3. MH⁺(ESI) |
| 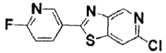 | 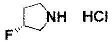 | 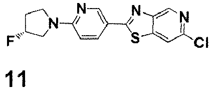 | 1. 62 % |
| | | | 2. NA |
| | | | 3. 335.11 |
| 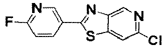 | 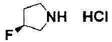 | 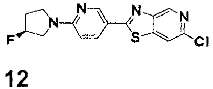 | 1. 73% |
| | | | 2. NA |
| | | | 3. 335.13 |
| 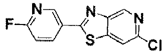 | 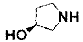 | 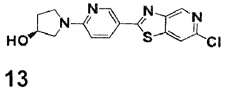 | 1.85% |
| | | | 2. ¹H NMR (400 MHz, DMSO-*d*₆) δ 8.99 (s, 1H), 8.80 (s, 1H), 8.31 (s, 1H), 8.14 (d, 1H), 6.63 (d, 1H), 5.05 (s, 1H), 4.44 (s, 1H), 3.69 - 3.39 (m, 4H), 2.16 - 1.86 (m, 2H). |
| | | | 3. 333.12 |
| 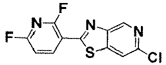 | 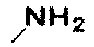 | 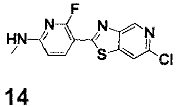 | 1.35% |
| | | | 2. ¹H NMR (400 MHz, DMSO-d₆) δ 9.02 (s, 1H), 8.48 - 8.21 (m, 2H), 7.98 (s, 1H), 6.58 (dd, 1H), 2.86 (d, 3H) |
| | | | 3. 295.41 |
| 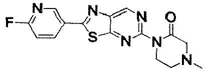 | 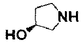 | 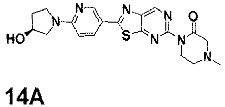 | 1.93 % |
| | | | 2. ¹H NMR (400 MHz, Chloroform-d) δ 9.15 (s, 1H), 8.80 (d, 1H), 8.15 - 8.04 (m, 1H), 6.54 - 6.42 (m, 1H), 4.68 (s, 1H), 4.13 - 4.00 (m, 2H), 3.80 - 3.54 (m, 4H), 3.38 (s, 2H), 2.86 (t, 2H), 2.43 (s, 3H), 2.30 - 2.06 (m, 2H) |
| | | | 3. 412.13 |
| 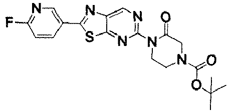 | 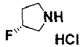 | 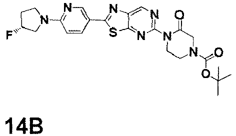 | 1.83% |
| | | | 2. ¹H NMR (400 MHz, Chloroform-d) δ 9.17 (s, 1H), 8.84 (d, J = 2.4 Hz, 1H), 8.14 (dd, J = 8.9, 2.5 Hz, 1H), 6.50 (d, J = 9.0 Hz, 1H), 5.42 (dt, J = 52.5, 3.6 Hz, 1H), 4.35 (s, 2H), 4.16 - 4.06 (m, 2H), 4.04 - 3.89 (m, 1H), 3.89 - 3.75 (m, 3H), 3.75 - 3.62 (m, 2H), 2.58 - 2.39 (m, 1H), 2.33 - 2.06 (m, 1H), 1.50 (s, 9H). |
| | | | 3. 500.23 |
| 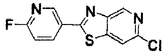 | 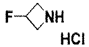 | 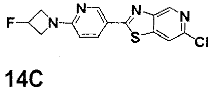 | 1. 66% |
| | | | 2. ¹H NMR (500 MHz, DMSO-d6) δ 9.03 (d, 1H), 8.83 (dd, 1H), 8.35 (d, 1H), 8.20 (dd, 1H), 6.61 (d, 1H), 5.56 (dtt, 1H), 4.43 (dddd, 2H), 4.17 (dddd, 2H). |
| | | | 3. 321.08 |
| 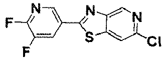 | 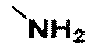 | 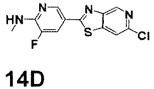 | 1. 65% |
| | | | 2. ¹H NMR (500 MHz, DMSO-d6) δ 9.14 (d, 1H), 8.68 (dd, 1H), 8.02 (d, 1H), 7.96 (dd, 1H), 7.74 - 7.66 (m, 1H), 2.95 (d, 3H). |
| | | | 3. 293.03 (MH⁻) |
| 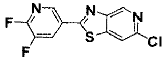 | 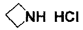 | 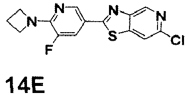 | 1. 49% |
| | | | 2. ¹H NMR (500 MHz, DMSO-d6) δ 8.99 (d, 1H), 8.60 (t, 1H), 8.33 (d, 1H), 8.02 - 7.95 (m, 1H), 4.30 - 4.17 (m, 4H), 2.37 (p, 2H). |
| | | | 3. 320.08 |

### Preparative Examples 15 to 17

Following the Pd-coupling procedure as described in Preparative Example 8, except using the dihalogenated starting material and the appropriate amide indicated in the table below, the following compounds were prepared.

**Table 4:**

| Halogenated Starting Material | Amide | Product **Example** | 1. Yield |
|---|---|---|---|
| | | | 2. ¹H-NMR |
| | | | 3. MH⁺(ESI) |
| 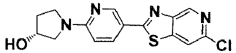 | 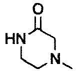 | 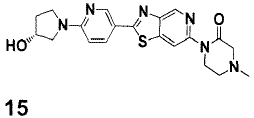 | 1. 41 % |
| | | | 2. ¹H NMR (400 MHz, DMSO-d₆) δ 9.04 (s, 1H), 8.82 - 8.75 (m, 1H), 8.48 (s, 1H), 8.12 (dd, 1H), 6.62 (d, 1H), 5.06 (s, 1H), 4.42 (s, 1H), 3.99 - 3.89 (m, 2H), 3.70 - 3.49 (m, 4H), 3.21 (s, 2H), 2.81 - 2.73 (m, 2H), 2.30 (s, 3H), 2.12 - 1.88 (m, 2H). |
| | | | 3. 411.17 |
| 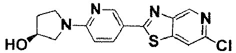 | 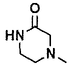 | 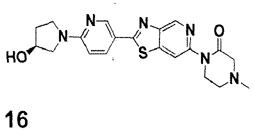 | 1. 68% |
| | | | 2. ¹H NMR (400 MHz, DMSO-d₆) δ 9.04 (s, 1H), 8.88 - 8.70 (m, 1H), 8.56-8.41 (m, 1H), 8.19-7.99 (m, 1H), 6.62 (d, 1H), 5.04 (s, 1H), 4.42 (s, 1H), 4.00 - 3.79 (m, 2H), 3.72 - 3.40 (m, 4H), 3.21 (s, 2H), 2.81 - 2.71 (m, 2H), 2.30 (s, 3H), 2.11 - 1.84 (m, 2H). |
| | | | 3. 411.19 |
| 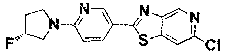 | 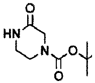 | 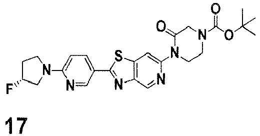 | 1. 42% |
| | | | 2. ¹H NMR (500 MHz, Chloroform-d) δ 9.03 (s, 1H), 8.82 (d, 1H), 8.49 (brs, 1H), 8.16 (dd, 1H), 6.49 (d, 1H), 5.48 - 5.36 (m, 1H), 4.32 (s, 1H), 4.22 - 4.20 (m, 2H), 4.02 - 3.90 (m, 1H), 3.80 - 3.67 (m, 5H), 2.50 - 2.42 (m, 1H), 2.25 - 2.15 (m, 1H), 1.51 (s, 9H). |
| | | | 3. 499.2 |

### Examples 1 to 3D

Following the Pd-coupling procedure as described in Preparative Example 8, using the halogenated starting material and the appropriate amide indicated in the table below, the following compounds were prepared.

**Table 5:**

| Halogenated Starting Material | Amide | Product **Example** | 1. Yield |
|---|---|---|---|
| | | | 2. ¹H-NMR |
| | | | 3. MH⁺(ESI) |
| 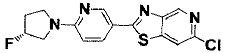 | 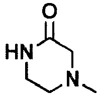 | 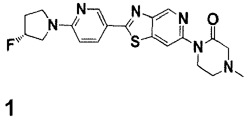 | 1.85% |
| | | | 2. ¹H NMR (400 MHz, DMSO-d₆) δ 9.06 (d, 1H), 8.82 (d, 1H), 8.54 - 8.45 (m, 1H), 8.17 (dd, 1H), 6.76 - 6.64 (m, 1H), 5.49 (d, 1H), 4.00 - 3.91 (m, 2H), 3.91 - 3.60 (m, 3H), 3.53 (td, 1H), 3.21 (s, 2H), 2.83 - 2.72 (m, 2H), 2.38 - 2.10 (m, 5H). |
| | | | 3. 413.09 |
| 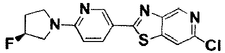 | 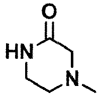 | 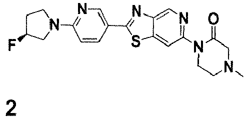 | 1.20% |
| | | | 2. ¹H NMR (400 MHz, Chloroform-*d*) δ 9.04 (d, *J* = 0.8 Hz, 1H), 8.82 (dd, *J* = 2.4, 0.7 Hz, 1H), 8.49 (d, *J* = 0.9 Hz, 1H), 8.16 (dd, *J* = 8.9, 2.5 Hz, 1H), 6.57 - 6.44 (m, 1H), 5.56 - 5.32 (m, 1H), 4.20 - 4.06 (m, 2H), 3.96 (dd, *J* = 25.0, 13.2 Hz, 1H), 3.85 - 3.60 (m, 3H), 3.35 (s, 2H), 2.93 - 2.71 (m, 2H), 2.42 (s, 4H), 2.33 - 2.01 (m, 1H). |
| | | | 3.413.22 |
| 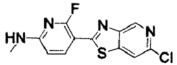 | 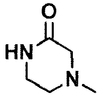 | 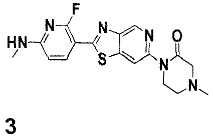 | 1. 15% |
| | | | 2. ¹H NMR (400 MHz, DMSO-d6) δ 9.07 (s, 1H), 8.48 (s, 1H), 8.36 (s, 1H), 7.93 (s, 1H), 6.67 - 6.49 (m, 1H), 4.02 - 3.87 (m, 2H), 3.21 (s, 2H), 2.85 (d, 3H), 2.80 - 2.73 (m, 2H), 2.30 (s, 3H) |
| | | | 3. 373.42 |
| 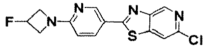 | 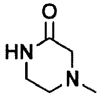 | 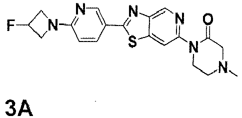 | 1.64% |
| | | | 2. ¹H NMR (500 MHz, Chloroform-d) δ 9.05 (s, 1H), 8.81 (d, 1H), 8.51 (s, 1H), 8.28 - 8.09 (m, 1H), 6.39 (d, 1H), 5.49 (dtt, 1H), 4.54 - 4.35 (m, 2H), 4.26 (ddd, 2H), 4.19 - 4.07 (m, 2H), 3.35 (s, 2H), 2.91 - 2.78 (m, 2H), 2.42 (s, 3H). |
| | | | 3. 399.10 |
| 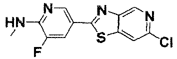 | 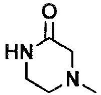 | 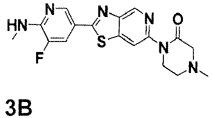 | 1.55% |
| | | | 2. ¹H NMR (500 MHz, DMSO-d6) δ 9.07 (d, 1H), 8.60 (dd, 1H), 8.51 (d, 1H), 7.96 (dd, 1H), 7.58 - 7.51 (m, 1H), 3.97 - 3.91 (m, 2H), 3.21 (s, 2H), 2.95 (d, 3H), 2.79 - 2.75 (m, 2H), 2.30 (s, 3H). |
| | | | 3. 373.0 |
| 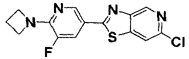 | 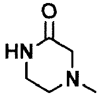 | 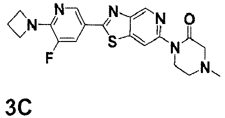 | 1. 48% |
| | | | 2. ¹H NMR (500 MHz, DMSO-d6) δ 9.09 (d, 1H), 8.64 - 8.60 (m, 1H), 8.53 (d, 1H), 8.01 (dd, 1H), 4.31 - 4.21 (m, 4H), 3.99 - 3.91 (m, 2H), 3.23 (s, 2H), 2.78 (t, 2H), 2.44 - 2.37 (m, 2H), 2.31 (s, 3H). |
| | | | 3. 399.10 |
| 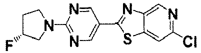 | 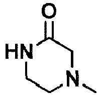 | 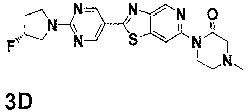 | 1.33% |
| | | | 2. ¹H NMR (500 MHz, DMSO-d6) δ 9.12 (d, 1H), 9.05 (d, 2H), 8.58 - 8.54 (m, 1H), 5.57 - 5.39 (m, 1H), 3.93 (dtd, 4H), 3.78 (ddd, 1H), 3.61 (td, 1H), 3.22 (s, 2H), 2.82 - 2.74 (m, 2H), 2.31 (s, 5H). |
| | | | 3. 414.22 |

### Examples 4 to 7C

Following the procedure described in Preparative Example 10, except using the fluoro derivatives and amines indicated in the table below, the following compounds were prepared.

**Table 6:**

| Fluoro Derivative | Amine | Product **Example** | 1. Yield |
|---|---|---|---|
| | | | 2. ¹H-NMR |
| | | | 3. MH⁺(ESI) |
| 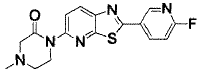 | 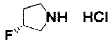 | 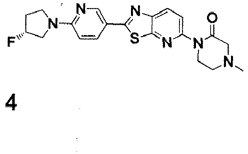 | 1.55% |
| | | | 2. ¹H NMR (400 MHz, DMSO-d6) δ 8.80 (s, 1H), 8.31 (d, 1H), 8.15 (d, 1H), 7.98 (d, 1H), 6.75 - 6.62 (m, 1H), 5.49 (d, 1H), 3.94 (t, 2H), 3.90 - 3.60 (m, 3H), 3.58 - 3.46 (m, 1H), 3.22 (s, 2H), 2.77 (t, 2H), 2.37 - 2.10 (m, 5H) |
| | | | 3. 413.51 |
| 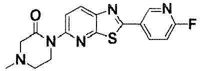 | 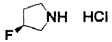 | 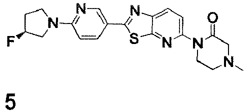 | 1.70% |
| | | | 2. ¹H NMR (400 MHz, DMSO-d6) δ 8.80 (d, 1H), 8.31 (d, 1H), 8.15 (dd, 1H), 7.98 (d, 1H), 6.68 (d, 1H), 5.49 (d, 1H), 3.94 (t, 2H), 3.91 - 3.61 (m, 3H), 3.52 (q, 1H), 3.22 (s, 2H), 2.77 (t, 2H), 2.41 - 2.11 (m, 5H) |
| | | | 3.413.53 |
| 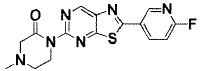 | 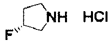 | 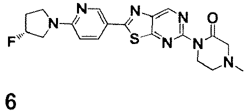 | 1.40% |
| | | | 2. ¹H NMR (400 MHz, Chloroform-*d*) δ 9.17 (s, 1H), 8.83 (d, 1H), 8.14 (dd, 1H), 6.49 (d, 1H), 5.42 (dt, 1H), 4.15 - 3.90 (m, 3H), 3.86 - 3.59 (m, 3H), 3.37 (s, 2H), 2.92 - 2.76 (m, 2H), 2.42 (s, 4H), 2.35 - 2.07 (m, 1H). |
| | | | 3.414.0 |
| 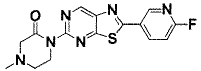 | 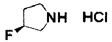 | 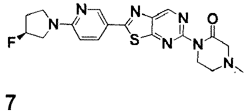 | 1.33% |
| | | | 2. ¹H NMR (400 MHz, Chloroform-*d*) δ 9.16 (s, 1H), 8.83 (d, 2H), 8.14 (dd, 1H), 6.49 (d, 1H), 5.57 - 5.32 (m, 1H), 4.15 - 3.87 (m, 3H), 3.87 - 3.59 (m, 3H), 3.36 (s, 2H), 2.84 (t, 2H), 2.41 (s, 4H), 2.38 - 2.01 (m, 1H). |
| | | | 3.414.0 |
| 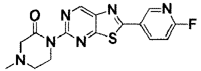 | 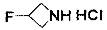 | 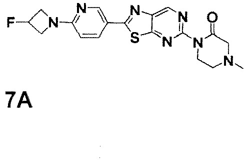 | 1.25% |
| | | | 2. ¹H NMR (400 MHz, Chloroform-d) δ 9.18 (s, 1H), 8.91 - 8.74 (m, 1H), 8.14 (dd, 1H), 6.39 (d, 1H), 5.49 (dtt, 1H), 4.45 (dq, 2H), 4.28 (dd, 2H), 4.19 - 4.07 (m, 2H), 3.47 (s, 2H), 2.96 (s, 2H), 2.50 (s, 3H). |
| | | | 3. 400.52 |
| 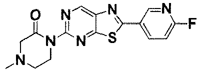 | 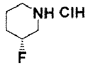 | 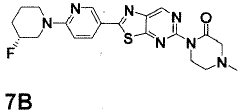 | 1.39% |
| | | | 2. ¹H NMR (500 MHz, Chloroform-d) δ 9.07 (s, 1H), 8.72 (d, 1H), 8.05 (dd, 1H), 6.75 - 6.68 (m, 1H), 4.90 - 4.57 (m, 1H), 4.07 - 3.95 (m, 3H), 3.81 (ddd, 1H), 3.72 (ddd, 1H), 3.32 (s, 2H), 3.29 (s, 1H), 2.88 - 2.76 (m, 2H), 2.36 (s, 3H), 2.02 - 1.79 (m, 3H), 1.65 - 1.52 (m, 1H). |
| | | | 3. 428.16 |
| 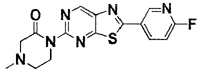 | 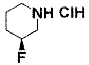 | 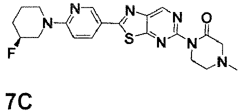 | 1.24% |
| | | | 2. ¹H NMR (500 MHz, DMSO-d6) δ 9.32 (s, 1H), 8.83 (d, 1H), 8.16 (dd, 1H), 7.06 (d, 1H), 4.98 - 4.68 (m, 1H), 4.19 (ddd, 1H), 4.08 - 3.97 (m, 1H), 3.93 - 3.85 (m, 2H), 3.73 (ddd, 1H), 3.44 (t, 1H), 3.19 (s, 2H), 2.82 - 2.73 (m, 2H), 2.31 (s, 3H), 2.01 - 1.84 (m, 2H), 1.83 - 1.69 (m, 1H), 1.65 - 1.51 (m, 1H). |
| | | | 3. 428.26 |

### Example 8

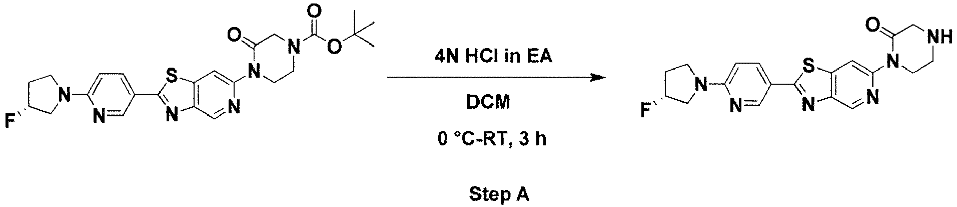

### Step A:

A solution of Preparative Example 17 (95 mg, 0.190 mmol) in dichloromethane (1.9 mL) was cooled to 0 °C and 4N HCl in EtOAc was added slowly (1 mL). The solution was stirred at room temperature for 2 h. The solvent was removed under reduced pressure and the crude product was dissolved in 10% methanol in dichloromethane, washed with saturated aqueous NaHCO₃, dried (Na₂SO₄), and concentrated. The resulting material was purified by solvent washings using pentane (10 mL) to provide the title product as a yellow solid (54 mg, 70%).
¹H NMR (500 MHz, Chloroform-d) δ 9.04 (d, 1H), 8.88 - 8.80 (m, 1H), 8.47 (s, 1H), 8.16 (dd, 1H), 6.49 (d, 1H), 5.51 - 5.33 (m, 1H), 4.14 - 4.05 (m, 2H), 4.04 - 3.88 (m, 1H), 3.85 - 3.61 (m, 5H), 3.33 - 3.22 (m, 2H), 2.51 - 2.39 (m, 1H), 2.31 - 2.09 (m, 1H).
MS (ESI); m/z = 399.0 [M+H]⁺

### Example 9

Following the procedure described in Example 8, the following compound was prepared.

**Table 7:**

| Boc Derivative | Product **Example** | 1. Yield |
|---|---|---|
| | | 2. ¹H-NMR |
| | | 3. MH⁺(ESI) |
| 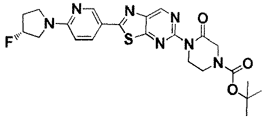 | 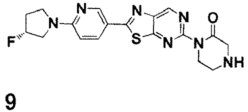 | 1.68 % |
| | | 2. ¹H NMR (400 MHz, Chloroform-*d*) δ 9.17 (s, 1H), 8.84 (d, J = 2.6 Hz, 1H), 8.15 (dd, J = 8.9, 2.5 Hz, 1H), 6.50 (d, J = 8.9 Hz, 1H), 5.42 (dt, J = 52.6, 3.8 Hz, 1H), 4.03 (t, J = 5.6 Hz, 2H), 4.01 - 3.88 (m, 1H), 3.88 - 3.79 (m, 1H), 3.77 (s, 2H), 3.74 - 3.62 (m, 2H), 3.28 (t, J = 5.6 Hz, 2H), 2.53 - 2.39 (m, 1H), 2.20 (m, 1H). |
| | | 3. 399.7 |

### Synthesis of ¹⁸F-labeled compounds

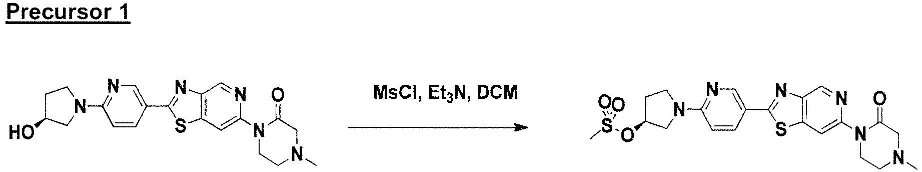

To a solution of Preparative Example 16 (60 mg, 0.146 mmol) and triethylamine (0.407 ml, 2.92 mmol) in dichloromethane (10 mL) was added drop by drop mesyl chloride (0.114 ml, 1.462 mmol). After 1h at room temperature, the crude product was poured into 1N NaOH and the aqueous phase was extracted several times with dichloromethane (4 x 20 mL). The combined organics were dried over Na₂SO₄, filtered and concentrated under reduced pressure. The residue was purified on HP-Sil SNAP cartridges using a Biotage Isolera One purification system employing a methanol/dichloromethane gradient (2/98 -> 10/90) to afford the title compound (36 mg, 50%).
¹H-NMR (400 MHz, DMSO-d6) δ 9.12 - 9.03 (m, 1H), 8.83 (d, 1H), 8.50 (s, 1H), 8.18 (dd, 1H), 6.71 (d, 1H), 5.46 (s, 1H), 4.00 - 3.92 (m, 2H), 3.92 - 3.67 (m, 3H), 3.55 (q, 1H), 3.28 (s, 3H), 3.21 (s, 2H), 2.82 - 2.73 (m, 2H), 2.41 - 2.27 (m, 5H).
MS (ESI); m/z = 488.95 [M+H]⁺

### Precursors 2 to 6

Following the procedure as described with respect to Precursor 1, except using the folowing hydroxyl starting material, the following compounds were prepared.

**Table 8:**

| Hydroxyl Starting Material | Product **Example** | 1. Yield |
|---|---|---|
| | | 2. ¹H-NMR |
| | | 3. MH⁺(ESI) |
| 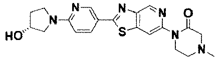 | 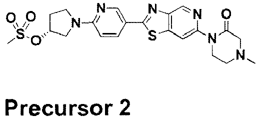 | 1.77% |
| | | 2. ¹H NMR (400 MHz, DMSO-d6) δ 9.06 (s, 1H), 8.88 - 8.78 (m, 1H), 8.50 (s, 1H), 8.18 (dd, 1H), 6.70 (d, 1H), 5.46 (s, 1H), 3.98 - 3.91 (m, 2H), 3.91 - 3.67 (m, 3H), 3.55 (q, 1H), 3.28 (s, 3H), 3.21 (s, 2H), 2.81 - 2.73 (m, 2H), 2.40 - 2.25 (m, 5H). |
| | | 3. 489.12 |
| 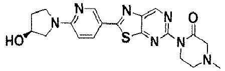 | 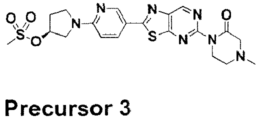 | 1.80% |
| | | 2. ¹H NMR (400 MHz, Chloroform-d) δ 9.17 (s, 1H), 8.83 (d, 1H), 8.15 (dd, 1H), 6.50 (d, 1H), 5.52 - 5.43 (m, 1H), 4.22 - 4.08 (m, 2H), 4.01 (d, 1H), 3.90 - 3.64 (m, 3H), 3.47 (s, 2H), 3.07 (s, 3H), 3.03 - 2.88 (m, 2H), 2.59 - 2.43 (m, 4H), 2.36 (dtd, 1H) |
| | | 3. 490.16 |
| 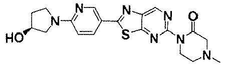 | 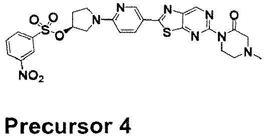 | 1.68% |
| | | 2. ¹H NMR (500 MHz, DMSO-d6) δ 9.32 (s, 1H), 8.80 (d, 1H), 8.63 - 8.57 (m, 2H), 8.42 (d, 1H), 8.17 (dd, 1H), 7.98 (t, 1H), 6.66 (d, 1H), 5.46 (s, 1H), 3.92 - 3.85 (m, 2H), 3.85 - 3.75 (m, 1H), 3.70 (dd, 2H), 3.53 (td, 1H), 3.19 (s, 2H), 2.81 - 2.73 (m, 2H), 2.35 - 2.19 (m, 5H). |
| | | 3. 597.4 |
| 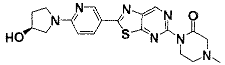 | 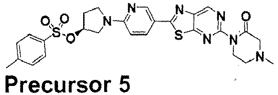 | 1.7% |
| | | 2. ¹H NMR (500 MHz, DMSO-d6) δ 9.32 (s, 1H), 8.82 (s, 1H), 8.21 - 8.14 (m, 1H), 7.84 (d, 2H), 7.49 (d, 2H), 6.66 (d, 1H), 5.25 (s, 1H), 3.91 - 3.86 (m, 2H), 3.69 (td, 3H), 3.50 (td, 1H), 3.18 (s, 2H), 2.80 - 2.74 (m, 2H), 2.42 (s, 3H), 2.30 (s, 4H), 2.19 - 2.09 (m, 1H). |
| | | 3. 566.0 |
| 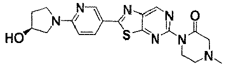 | 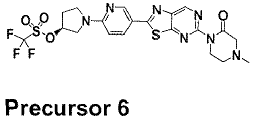 | 1. NA |
| | | 2. NA |
| | | 3. NA |

### Example ¹⁸F-1

[¹⁸F]Fluoride (964 mCi at start of synthesis), produced via the ¹⁸O(p,n)¹⁸F nuclear reaction by using a cyclotron equipped with a high-yield oxygen-18 water target, was purchased from PETNET. The [¹⁸F]fluoride in ~2 mL of [¹⁸O]H₂O was trapped on a Waters QMA cartridge pre-conditioned with HPLC-grade water (5 mL) to remove [¹⁸O]H₂O. [¹⁸F]Fluoride was eluted into the reaction vessel by passing K222/K₂CO₃ solution (7.5 mg/0.75 mg in 0.4 mL/0.4 mL of HPLC-grade acetonitrile/water) through the cartridge. The [¹⁸F]fluoride was then dried by heat (70°C) and a stream of nitrogen under full vacuum for 5 min, followed by only full vacuum at 100°C for 5 min. After drying, a solution of **Precursor 1** (2.0 mg) in anhydrous DMSO (1 mL) was added and the resulting solution was heated at 100°C with stirring for 10 min. The reaction mixture was then cooled to 50°C, followed by diluting with 4 mL of HPLC-grade water. The diluted reaction mixture was then transferred to the loop-loading vial. The contents of the loop-loading vial were transferred onto the semi-preparative HPLC for purification as described above. The product peak (Rt ~28 min) was collected into the HPLC dilution flask and diluted with HPLC-grade water (40 mL). The purified **¹⁸F-1** was then trapped on a C18-E cartridge (Phenomenex, C18-E-50 mg cartridge PN 8B-S001- DAK) pre-conditioned with ethanol (5 mL) and water (5 mL), followed by washing with water (5 mL). The trapped **¹⁸F-1** was eluted with ethanol (0.5 mL) into the formulation flask, followed by diluting with 4.5 mL of saline. The **¹⁸F-1** formulation was then transferred into a sterile empty vial and submitted for quality control testing. Chemical and radiochemical purities/identities were analyzed using an Agilent 1100 HPLC equipped with a radioactivity detector and an ultraviolet (UV) detector. Radiochemical purity for doses was >99%, and identity was confirmed by comparing the retention time of the radiolabeled product with that of the corresponding unlabeled reference standard **Example 1.**

### Example ¹⁸F-2

[¹⁸F]Fluoride (1148 mCi at start of synthesis), produced via the ¹⁸O(p,n)¹⁸F nuclear reaction by using a cyclotron equipped with a high-yield oxygen-18 water target, was purchased from PETNET. The [¹⁸F]fluoride in ~2 mL of [¹⁸O]H₂O was trapped on a Waters QMA cartridge pre-conditioned with HPLC-grade water (5 mL) to remove [¹⁸O]H₂O. [¹⁸F]Fluoride was eluted into the reaction vessel by passing K222/K₂CO₃ solution (7.5 mg/0.75 mg in 0.4 mL/0.4 mL of HPLC-grade acetonitrile/water) through the cartridge. The [¹⁸F]fluoride was then dried by heat (70°C) and a stream of nitrogen under full vacuum for 5 min, followed by only full vacuum at 100 °C for 5 min. After drying, a solution of **Precursor 2** (2.0 mg) in anhydrous DMSO (1 mL) was added and the resulting solution was heated at 100°C with stirring for 10 min. The reaction mixture was then cooled to 50°C, followed by diluting with 4 mL of HPLC-grade water. The diluted reaction mixture was then transferred to the loop-loading vial. The contents of the loop-loading vial were transferred onto the semi-preparative HPLC for purification. The product peak was collected into the HPLC dilution flask and diluted with HPLC-grade water (40 mL). The purified **¹⁸F-2** was then trapped on a C18-E cartridge (Phenomenex, C18-E-50 mg cartridge PN 8B-S001-DAK), pre-conditioned with ethanol (5 mL) and water (5 mL), followed by washing with water (5 mL). The trapped **¹⁸F-2** was eluted with ethanol (0.5 mL) into the formulation flask, followed by diluting with 4.5 mL of saline. The **¹⁸F-2** formulation was then transferred into a sterile empty vial and submitted for quality control testing. Chemical and radiochemical purities/identities were analyzed using an Agilent 1100 HPLC equipped with a radioactivity detector and an ultraviolet (UV) detector. Radiochemical purity for doses was >99%, and identity was confirmed by comparing the retention time of the radiolabeled product with that of the corresponding unlabeled reference standard **Example 2.**

### Example ¹⁸F-6

Following a similar **¹⁸F-2** radiolabelling procedure, **Precursor 3** was radiolabeled. Purification is performed by HPLC using a semi-preparative Phenomenex Gemini C18 column (5 µm, 250 × 10 mm) and eluted with a mixture of acetonitrile/phosphate buffer solution (25 mM) (25/75, v/v) at a flow rate of 4 mL/min. The product fraction is collected in a flask, containing 20 mL of sodium ascorbate in WFI. The diluted product mixture is passed through a C18 solid-phase extraction cartridge and the cartridge is rinsed with 10 mL of sodium ascorbate in WFI. The radiolabeled product **¹⁸F-6** is eluted from the SPE cartridge with 1 mL of 200-proof USP grade ethanol into the formulation flask, pre-loaded with 10 mL of formulation base. The cartridge is rinsed with 4 mL of formulation base and the rinse is mixed with the contents of the formulation flask. The resulting solution is passed through a sterilizing 0.2 µm membrane filter into a sterile, filter-vented vial (final product vial, FPV), pre-filled with 15 mL of normal saline. Chemical and radiochemical purities/identities were analyzed using an Agilent 1100 HPLC equipped with a radioactivity detector and an ultraviolet (UV) detector. Radiochemical purity for doses was >99%, and identity was confirmed by comparing the retention time of the radiolabeled product with that of the corresponding unlabeled reference standard **Example 6.**

### Radioligand synthesis

### Example ³H-1

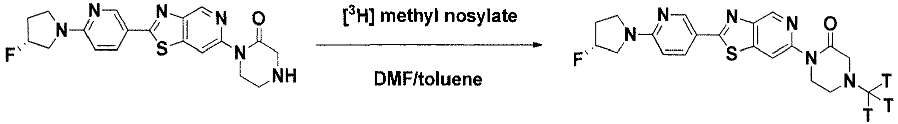

The Example 8 was dissolved in DMF/toluene (1/1, 0.3mL), potassium carbonate (1.0mg) was added and the mixture was added to dry methyl nosylate ([³H] 25 mCi) in a conical flask. The solution was stirred for overnight at room temperature. The reaction mixture was transferred to a larger flask and the reaction vessel was rinsed with 4 × 2 mL methanol. The combined methanol was removed under vacuum. The material was purified by HPLC. The mobile phase was removed. The entire process was repeated and the two final reaction products were combined. (3 mCi with radiochemical purity of >99% and a specific activity of 60 Ci/mmol).
MS (ESI): m/z = 419 (100%) [M+H]⁺

### Example ³H-6

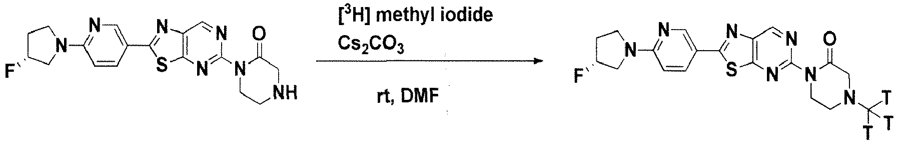

The Example 9 (1 mg) was dissolved in DMF (0.1mL), cesium carbonate (1.0mg) was added and the mixture was added to [³H] methyl iodide (75 mCi). The solution was stirred for overnight at room temperature. The reaction mixture was transferred to a larger flask and the reaction vessel was rinsed with 4 × 2 mL methanol. The combined methanol was removed under vacuum. The material was purified by HPLC. The mobile phase was removed. The entire process was repeated and the two final reaction products were combined. (10 mCi with radiochemical purity of >99% and a specific activity of 75 Ci/mmol).
MS (ESI): m/z = 442 (100%) [M+Na]⁺

### [³H] Example 47 of WO2017/153601

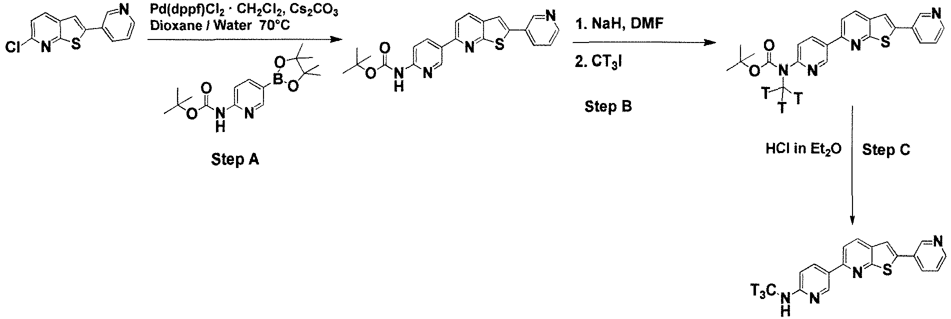

### Step A:

A mixture of 6-chloro-2-(pyridin-3-yl)thieno[2,3-b]pyridine prepared as described in WO 2017/153601 (58 mg, 0.203 mmol), tert-butyl (5-(4,4,5,5-tetramethyl-1,3,2-dioxaborolan-2-yl)pyridin-2-yl)carbamate (78 mg, 0.24 mmol), cesium carbonate (0.130 g, 0.406 mmol) and [1,1'-bis(diphenylphosphino)ferrocene]dichloropalladium(II) complex with dichloromethane (0.008 g, 0.010 mmol) were added into a dry pressure tube, followed by degassed dioxane (4 mL) and degassed water (1 mL). The reaction mixture was degassed with a stream of argon for 10 minutes and heated at 70 °C for 2 hours. The mixture was cooled to room temperature and the solvents were removed. The crude product was taken up in DCM (20 mL), washed with water (2 × 20 mL) and brine (10 mL), dried over Na₂SO₄, and concentrated under reduced pressure. The crude product was triturated with (10 mL) of isopropanol for 10 hours and the solid was filtered and dried under reduced pressure to afford the title compound (42 mg, 52%).
¹H NMR (400 MHz, Chloroform-d) δ 9.02 (dd, 2H), 8.63 (dd, 1H), 8.42 (dd, 1H), 8.11 (dd, 2H), 8.01 (dt, 1H), 7.78 - 7.67 (m, 2H), 7.56 (s, 1H), 7.41 (dd, 1H), 1.57 (d, 9H).
MS (ESI); m/z = 348.89 [M-Boc].

### Step B:

The title compound was prepared from the compound of Step A above using sodium hydride in DMF followed by addition of tritium labeled methyliodide (Moravek Biochemical and Radiochemicals (U.S.A.)).

### Step C:

The tritium labeled title compound from Step B above was deprotected using HCI in diethylether. After purification by HPLC (Phenomenex Prodigy ODS(2), 4.6 x 250 mm, 5 µm; solvents A: water with 0.1% TFA; B: acetonitrile; 0-20 minutes 0-100% B; hold to 30 minutes), [³H] Example 47 of WO 2017/153601 was obtained with a radiochemical purity of 99.3% and a specific activity of 70.0 Ci/mmol).
MS (ESI): m/z = 319 (9%) [M+H]+; 323 (54%) [M+H]+; 325 (100%) [M+H]⁺.

### [³H] Example 69 of WO2017/153601

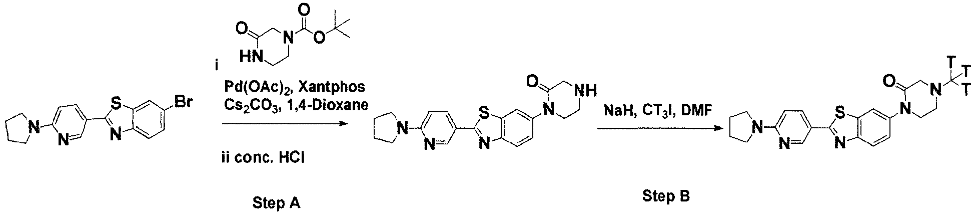

### Step A:

1,4-Dioxane (8 mL) was degassed 10 min. Diacetoxypalladium (93 mg, 0.416 mmol) and xanthphos (72.3 mg, 0.125 mmol) were added. The suspension was then heated at 110°C for 2 min. 6-Bromo-2-(6-(pyrrolidin-1-yl)pyridin-3-yl)benzo[d]thiazole prepared as described in WO 2017/153601 (150 mg, 0.416 mmol), tert-butyl 3-oxopiperazine-1-carboxylate (83 mg, 0.416 mmol) and cesium carbonate (136 mg, 0.416 mmol) were added and the stirring was continued at 110°C for 4h. The reaction mixture was cooled to 0°C and 10 mL of conc. HCl was added and stirred at room temperature for 4h. Water (20 mL) was added and extracted several times with DCM. The pH of the aqueous phase was then adjusted to pH 14 and extracted several times with DCM. The combined organics of the basic extractions were dried over Na₂SO₄, filtered and dried under reduced pressure. The residue was purified on HP-Sil SNAP cartridges using a Biotage Isolera One purification system employing a methanol/dichloromethane gradient (2/98 -> 20/80) to afford the title compound (70 mg, 44%).
¹H-NMR (400 MHz, DMSO-d6) (400 MHz, DMSO-d6) δ 8.76 (d, 1H), 8.10 (dd, 1H), 8.03 (d, 1H), 7.93 (d, 1H), 7.42 (dd, 1H), 6.60 (d, 1H), 3.66 (t, 2H), 3.54 - 3.44 (m, 4H), 3.42 (s, 2H), 3.04 (t, 2H), 2.80 (s, 1H), 2.05 - 1.87 (m, 4H).
MS (ESI); m/z = 380.14 [M+H]⁺

### Step B:

The title compound was prepared from the compound of Step A above using sodium hydride in DMF followed by addition of tritium labeled methyliodide (Moravek Biochemical and Radiochemicals (U.S.A.)). After purification by HPLC (Phenomenex Prodigy ODS(2), 4.6 x 250 mm, 5 µm; solvents A: water with 0.1% TFA; B: acetonitrile; 0-20 minutes 0-100% B; hold to 30 minutes), [³H] Example 69 of WO 2017/153601 was obtained with a radiochemical purity of 98.8% and a specific activity of 36.0 Ci/mmol) were obtained.
MS (ESI): m/z = 394 (100%) [M+H]+; 396 (39.1%) [M+H]+; 398 (58.3%) [M+H]+; 400 (51.1%) [M+H]+.

### BIOLOGICAL ASSAY DESCRIPTION

### Preparation of full-length aSyn Fibrils

Recombinant human full-length wild-type aSyn bacterially expressed was obtained from a commercial provider (rPeptide, US). Protein preparation was transferred into polycarbonate centrifuge tubes (8 x 51 mm; Beckman 355657) and centrifuged at 100,000 g (38,000 rounds per minute, RPM) in an ultracentrifuge (Beckman, XL100K) for 60 min at 4°C using the pre-cooled 50.4 Ti rotor (Beckman) to pellet any aggregated material. Supernatant was transferred into a sterile, low retention tube at a final concentration of 5 mg/mL and final volume of 0.5 mL in PBS. The protein was placed in a Thermomixer (Eppendorf) and incubated for 7 days at 37°C with orbital shaking at 1000 rpm to generate aSyn fibrils.

Material containing aSyn fibrils was transferred into polycarbonate centrifuge tubes (8 × 51 mm; Beckman 355657) and centrifuged at 100,000 g (38,000 RPM) in an ultracentrifuge (Beckman, XL100K) for 60 min at 4°C using the pre-cooled 50.4 Ti rotor. The supernatant containing still monomeric aSyn was transferred into new tubes and its concentration was determined. The pellet was resuspended with equal volume of PBS and subjected to further rounds of ultracentrifugation until the concentration obtained for the supernatant was below 0.1 mg/mL. The pellet obtained at the last round of ultracentrifugation containing aSyn fibrils was aliquoted and stored at -80°C until use. Aggregated aSyn was used at a concentration of 1 µM in radiobinding assays.

### Preparation of Insoluble fraction from human Alzheimer's disease brain

4.7 g of frontal lobe tissue from post-mortem brain of one Alzheimer's disease (AD) patient were purchased from an external provider (Tissue Solutions Ltd., UK). The patient was a Caucasian male and died at the age of 76 years. The brain sample was collected 7.7 hours post-mortem and characterized for Braak disease stage (Break, H.; Break, E. Acta Neuropathol., 1991, 82, 239-259) as stage VI. For the preparation of the insoluble fractions, around 3 g of the AD human brain was thawed on ice and homogenized with 8.9 mL of homogenization buffer (0.75 M NaCl, 5 mM EDTA, 50 mM Tris-HCI, pH 7.5) supplemented with protease inhibitors (Complete; Roche 11697498001) in a glass Dounce homogenizer. The homogenization was performed at 4°C. The homogenate was transferred into polycarbonate centrifuge tubes (16 × 76 mm; Beckman 355603) and centrifuged at 100,000 g (38,000 RPM) in an ultracentrifuge (Beckman, XL100K) for 60 min at 4°C using the pre-cooled 70.1 rotor (Beckman, 342184). Supernatants were discarded and pellets were re-suspended in 8.9 mL of homogenization buffer supplemented with 1% Triton X-100 (Sigma 93426) and processed with glass Dounce homogenizer at 4°C. The solution was then transferred into polycarbonate centrifuge bottles (16 × 76 mm; Beckman 355603) and centrifuged at 100,000 g (38,000 RPM) in an ultracentrifuge (Beckman, XL100K) for 60 min at 4°C using the 70.1 Ti rotor. The process was repeated one more time (supernatants were discarded and pellets were re-suspended in 8.9 mL of homogenization buffer supplemented with 1% Triton X-100 and 1M sucrose processed with glass Dounce homogenizer at 4°C. The solution was then centrifuged at 100,000 g (38,000 RPM) for 60 min at 4°C using the 70.1 Ti rotor). Supernatant was discarded and pellet was resuspended in 4 mL of PBS to obtain the insoluble fraction. The material was aliquoted and stored at -80°C until use.

### Determination of the binding affinity (Kᵢ) by radio-binding competition assay

Recombinant aSyn fibrils at concentration of 1 µM were incubated with [³H] Example 69 disclosed in WO2017/153601, used as aSyn reference binder, at concentrations between 25 nM and 35 nM. The example compounds of this invention were incubated at increasing concentrations in the range 0.4 nM to 2 µM for 120 minutes at 25°C. 90 µL of samples in duplicates were then filtered under vacuum on a GF/C filter plate (PerkinElmer 6005174) to trap the aSyn fibrils with the bound radio-ligand, and washed five times with Tris 50 mM buffer pH 7-5. The GF/C filters were then vacuum-dried, 50 µL scintillation liquid (Ultimate Gold MB, PerkinElmer) was added in each well, and the filters were analyzed on a Microbeta2 device. Non-specific signal was determined with an excess of un-labeled ligand and specific binding was calculated by subtracting the non-specific signal from the total signal. Competition was calculated as percent, where 0% was defined as the specific binding in the presence of vehicle (0.1 % DMSO in 50 mM Tris in 0.9% NaCl, 0.1% BSA) and 100% as the values obtained in the presence of excess cold Example 69 disclosed in WO2017/153601. All measurements were performed with at least two technical replicates. The Kᵢ values were calculated by nonlinear regression, one site specific binding using Prism V7 (GraphPad). Ki values to recombinant aSyn fibrils were determined in independent experiments and two different batches of aSyn fibrils, derived from the same source but different lots of monomeric aSyn. Results of radio-binding competition assay for the example compounds tested in parallel within the same experiment are shown in Table 9 as measurements A and B. Measurement A was performed on one batch of aSyn fibrils; the Kd for the radioligand [³H] Example 69 disclosed in WO2017/153601 for this batch of aSyn fibrils was 32 nM. Measurement B was performed on second batch of aSyn fibrils, the corresponding Kd for the radioligand [³H] Example 69 disclosed in WO2017/153601 for the second batch of aSyn fibrils was 60 nM.

As shown in Table 9 the compounds 1 to 7 of the present invention show superior binding to aSyn fibrils when compared to the corresponding compounds 70 and 71 disclosed in WO2017/153601. The results for compounds 1 and 2 were reproduced on two independent batches of aSyn fibrils. The Ki value on aSyn fibrils for compounds 1, 2, 4 and 5 of the present invention is improved by at least twofold when compared head-to-head within the same measurement to compounds 70 and 71 disclosed in WO2017/153601.

Compounds 3A to 3D and 7A to 7C display similar (7A and 3A) or improved (3B, 3C, 3D, 7B, 7C) binding to aSyn fibrils in comparison to Examples 70 and 71 of WO2017/153601. Compound 3C shows considerably greater affinity to aSyn fibrils when compared to Examples 70 and 71 of WO2017/153601, as indicated by the Ki value determined in the same measurement.

AD insoluble fractions diluted 1/100 were incubated in the presence of [³H] Example 47 disclosed in WO2017/153601 used as Aβ reference binder, at a concentration of 7 nM and the example compounds of this invention were prepared at increasing concentrations in the range 0.4 nM to 2 µM for 120 minutes at 25°C. 90 µL of samples in duplicates were then filtered under vacuum on a GF/C filter plate (PerkinElmer 6005174) to trap the Aβ aggregates within the AD insoluble fractions with the bound radio-ligand, and washed five times with Tris 50 mM buffer pH 7-5. The GF/C filters were then vacuum-dried, 50 µL scintillation liquid (Ultimate Gold MB, PerkinElmer) was added in each well, and the filters were analyzed on a Microbeta2 device. Non-specific signal was determined with an excess of un-labeled ligand and specific binding was calculated by subtracting the non-specific signal from the total signal. Competition was calculated as percent, where 0% was defined as the specific binding in the presence of vehicle (0.1 % DMSO in 50 mM Tris in 0.9% NaCl, 0.1% BSA) and 100% as the values obtained in the presence of excess cold Example 47 disclosed in WO2017/153601. The Kᵢ values were calculated by nonlinear regression, one site specific binding using Prism V7 (GraphPad). Ki values to AD insoluble fraction were determined in independent experiments. Results of radio-binding competition assay on AD insoluble fraction are shown in Table 9 as measurements C and D. The Kd for the radioligand [³H] Example 47 disclosed in WO2017/153601 on the AD insoluble fraction in measurements C and D was 8 nM.

Example compounds are ranked based on the measured Ki values to Aβ pathological aggregates present in the AD insoluble fractions. Example compounds showing affinity above 500 nM are assigned a score of +++, affinity below 50 nM are assigned a score of +, and finally example compounds with intermediate Ki values between 500 nM and 50 nM are assigned a score of ++.

Based on the radio-binding competition assay on these two targets all example compounds show good selectivity for aSyn over pathological Aβ aggregates present in the human AD insoluble fractions.

**Table 9**

| **Example** | **Ki on aSyn Fibrils (nM)^{a}** | | **Ki on human AD-Insoluble Fraction (nM)^{b}** | |
|---|---|---|---|---|
| | **A** | **B** | **C** | **D** |
| 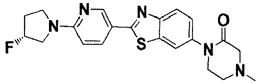 Example 70 of WO2017/153601 | 39.0 | 97.7 | +++ | ND |
| 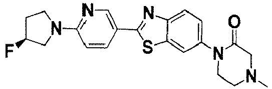 Example 71 of WO2017/153601 | 46.9 | 73.7 | +++ | ND |
| 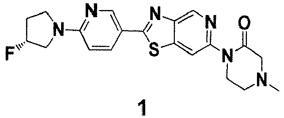 | 14.7 | 31.9 | +++ | ND |
| 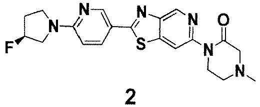 | 12.2 | 31.9 | +++ | +++ |
| 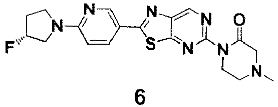 | ND | 28.3 | +++ | +++ |
| 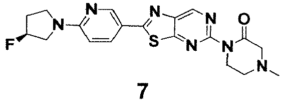 | ND | 52.8 | ND | +++ |
| 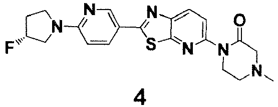 | ND | 41.7 | ND | +++ |
| 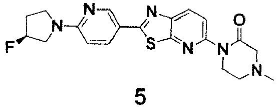 | ND | 32.9 | ND | +++ |
| 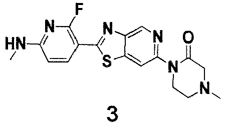 | ND | 66.9 | ND | ++ |
| 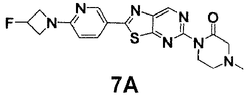 | 55.3 | ND | ND | +++ |
| 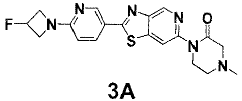 | ND | 96.8 | ND | +++ |
| 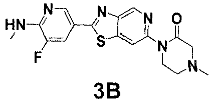 | 27.8 | ND | ++ | ++ |
| 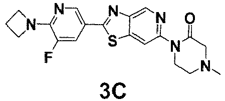 | 5.8 | ND | ND | +++ |
| 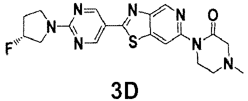 | 38.4 | ND | ND | +++ |
| 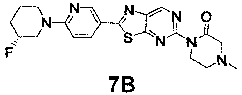 | 31.3 | ND | ND | +++ |
| 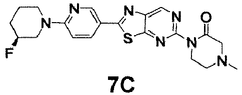 | 34.2 | ND | +++ | +++ |

| | | | | |
|---|---|---|---|---|
| Table Legend: ^{a}Ki on aSyn Fibrils shown for measurement A and measurement B on two different batches of aSyn fibrils. The Kd of the radioligand [³H] Example 69 of WO2017/153601 on the two batches of aSyn fibrils was 32 nM (on measurement A) and 60 nM (on measurement B). ^{b}Ki on human AD-Insoluble Fraction shown for measurement C and measurement D: +++ >500nM, ++ 500>x>50 nM, + < 50 nM; ND not determined The Kd of the radioligand [3H] Example 47 of WO2017/153601 on the AD-Insoluble Fraction in measurement C and measurement D was 8 nM. | | | | |

### PK studies in healthy monkey

NHP was injected intravenously (iv) with the 18F-labeled compound **¹⁸F-Example 70** of WO2017/153601 (3.49 mCi) using normal saline (0.9% NaCl) formulated with the intent to contain approximately 3.3% (v/v) ethanol (EtOH) and sodium ascorbate (4.67 mg/mL). PET scans were performed using a Siemens Focus 220. PET acquisition started immediately before the radioactive dose was injected. Images were generated as dynamic scans for 180 minutes.

NHP was injected intravenously (iv) with the ¹⁸F-labeled compound **¹⁸F-6** (4.92 mCi) using normal saline (0.9% NaCl) formulated with the intent to contain approximately 3.3% (v/v) ethanol (EtOH) and sodium ascorbate (4.67 mg/mL). PET scans were performed using a Siemens Focus 220. PET acquisition started immediately before the radioactive dose was injected. Images were generated as dynamic scans for 120 minutes.

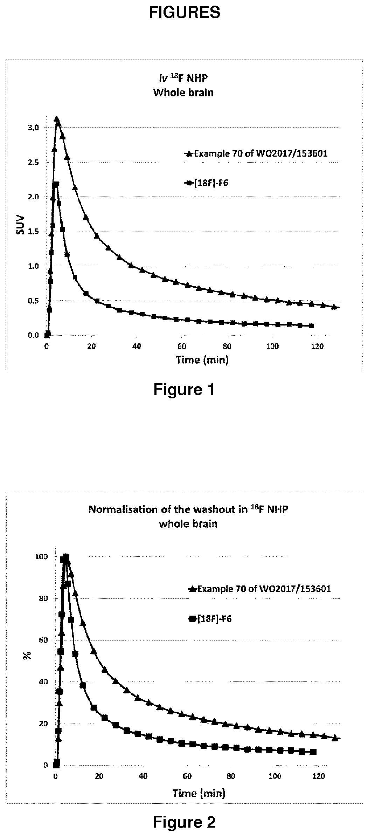

Compound **¹⁸F-Example 70** of WO2017/153601 has a peak uptake of SUVmax 3.1, a ratio of SUVmax/SUV30 min of 2.8 and ratio of SUVmax /SUV120 min of 7.8 (Figure 1 and 2). Compound **¹⁸F-6** peak uptake of SUVmax 2.2 but displays an improved washout with a ratio of SUVmax/SUV30 min of 7.3 and ratio of SUVmax /SUV120 min of 15.7 (Figure 1 and 2). The improved washout is a consequence of the decreased unspecific retention of compound **¹⁸F-6** and results in a significantly lower background and consequently in an enhanced signal to noise ratio.

### Assessment of specific binding of compound ³H-6 in brain sections from PD, PDD and non-demented control (NDC) by autoradiography

Frozen human sections from three Parkinson's disease (PD) cases labeled as PD1, PD2, and PD3, one Parkinson's disease with dementia (PDD) case and three non-demented control (NDC) cases (NDC1, NDC2, NDC3) were first briefly fixed for 15 minutes at 4°C with 4% paraformaldehyde and washed three times for five minutes with PBS (Dulbecco's phosphate buffered saline, Sigma) at room temperature (RT). All slides were then equilibrated for at least one hour in 50mM Tris-HCI pH 7.4 buffer prior to use in the experiment. Each brain section was incubated with 20nM of compound **³H-6** in Tris-HCI buffer overnight at 4°C in a humidity chamber. To determine non-specific binding (NSB), compound **³H-6** was mixed with 5µM of unlabeled compound **6** (self-block, '+').

The following day, slides were washed sequentially with ice-cold PBS for one minute; twice with ice-cold 50mM Tris-HCI pH 7.4 buffer for one minute; and PBS for one minute. Slides were allowed to air-dry before being placed under Phosphor imaging screens (GE healthcare, BAS-IP TR 2025) in imaging cassettes for 10 days. Imaging screens were scanned using a phosphor imaging system (Typhoon, FLA 7000) and resulting images were analyzed using Imaged. Specific binding was determined by subtracting the non-specific signal from the total signal.

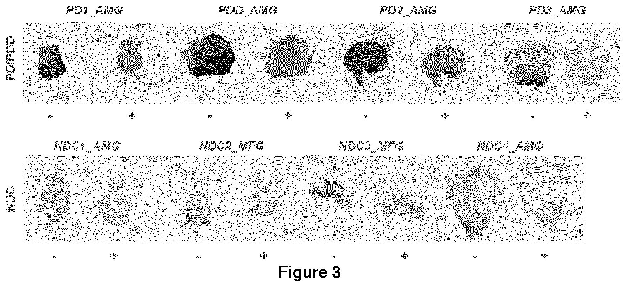

As shown in Figure 3, compound **³H-6** shows displaceable signal in different PD cases and weak signal in multiple non-demented control cases, indicating specific binding of the compound to Lewy bodies and neurites in PD brain tissue.

## Claims

1. A compound of formula (I): 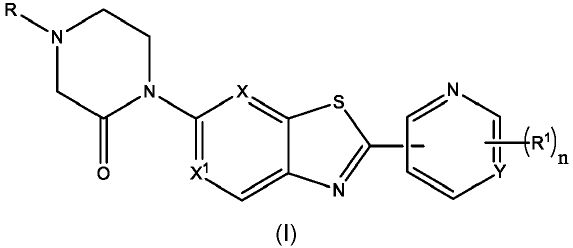
or stereoisomers, racemic mixtures, pharmaceutically acceptable salts, hydrates, solvates, and polymorphs thereof;
wherein
R is selected from the group consisting of hydrogen and alkyl;
**R¹** is independently selected from the group consisting of fluorine, 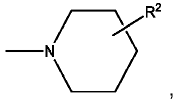 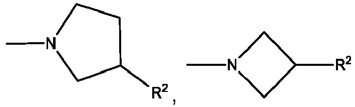 and -NR³R⁴;
**R²** is selected from the group consisting of fluorine and hydrogen;
**R³** and **R⁴** are independently selected from the group consisting of alkyl, fluoro-alkyl, alkyl-O-alkyl and hydrogen;
**X** and **X¹** are independently selected from the group consisting of N and CH, provided that at least one of X and X¹ is N;
**Y** is independently selected from the group consisting of N and CH or Y is C if Y is attached to R¹;
**n** is 1 or 2;
wherein the compound can optionally contain a detectable label selected from radionuclides, positron emitters, gamma emitters, fluorescent labels, luminescent labels and chromogenic labels.

2. The compound according to claim 1, which is a compound of the formula (la), (lb) or (Ic): 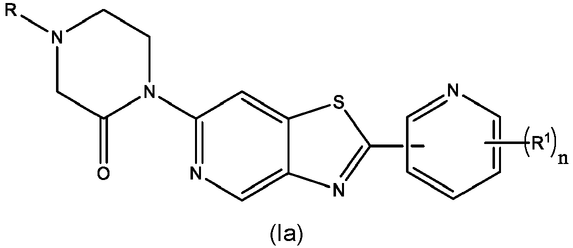 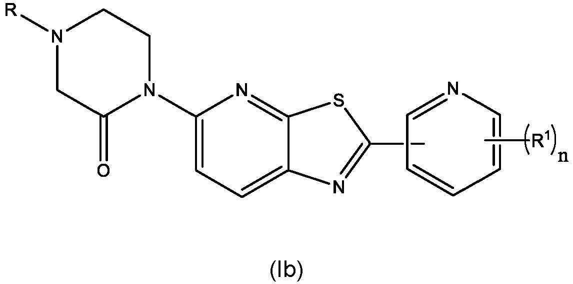 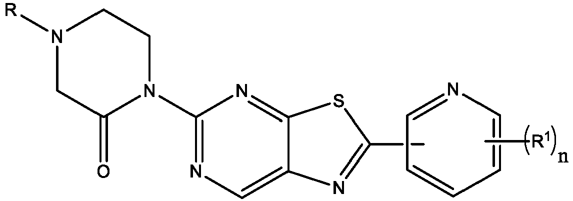 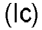 wherein **R, R¹** and n are as defined in claim 1.

3. The compound according to any one of claims 1 and 2, wherein **R¹** is

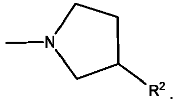

4. The compound according to any one of claims 1 to 3, wherein at least one of **R¹**, **R²**, **R³** and **R⁴** contains fluorine.

5. The compound according to any one of claims 1 to 4, wherein the compound is detectably labeled, preferably with ²H, ³H, ¹⁸F or ¹³N, more preferably with ¹⁸F.

6. A diagnostic composition comprising a compound according to any one of claims 1 to 5 and optionally a pharmaceutically acceptable carrier, diluent, adjuvant and/or excipient.

7. The compound according to any one of claims 1 to 5 for use in diagnostics.

8. The compound according to any one of claims 1 to 5 for use in the imaging of alpha-synuclein aggregates including, but not limited to, Lewy bodies and/or Lewy neuritespreferably wherein the compound is for use in the positron emission tomography imaging of alpha-synuclein aggregates including, but not limited to, Lewy bodies and/or Lewy neurites.

9. The compound according to any one of claims 1 to 5 for use in the diagnostics of a disorder or abnormality associated with alpha-synuclein aggregates including, but not limited to, Lewy bodies and/or Lewy neurites or a preclinical state thereof.

10. The compound for use according to claim 9, wherein the disorder is selected from Parkinson's disease (including sporadic, familial with alpha-synuclein mutations, familial with mutations other than alpha-synuclein, pure autonomic failure or Lewy body dysphagia), dementia with Lewy bodies (including "pure" Lewy body dementia), sporadic Alzheimer's disease, familial Alzheimer's disease with APP mutations, familial Alzheimer's disease with PS-1, PS-2 or other mutations, familial British dementia, Lewy body variant of Alzheimer's disease, Down syndrome, multiple system atrophy (including Shy-Drager syndrome, striatonigral degeneration or olivopontocerebellar atrophy), traumatic brain injury, chronic traumatic encephalopathy, motor neuron disease, neuroaxonal dystrophy, neurodegeneration with brain iron accumulation type 1 (including Hallervorden-Spatz syndrome), prion diseases, ataxia telangiectatica, Meige's syndrome, subacute sclerosing panencephalitis, Gaucher disease, lysosomal storage disorders (including Kufor-Rakeb syndrome and Sanfilippo syndrome) and rapid eye movement (REM) sleep behavior disorder.

11. The compound for use according to claim 9, wherein the disorder is Parkinson's disease, dementia with Lewy bodies or multiple system atrophy.

12. A method of collecting data for the diagnosis of a disorder or abnormality associated with alpha-synuclein aggregates in a patient comprising:
(a) bringing a sample suspected to contain alpha-synuclein aggregates into contact with a compound as defined in any one of claims 1 to 5;
(b) allowing the compound to bind to the alpha-synuclein aggregates;
(c) detecting the compound bound to the alpha-synuclein aggregates; and
(d) optionally correlating the presence or absence of compound binding with the alpha-synuclein aggregates with the presence or absence of alpha-synuclein aggregates in the sample.

13. A method of collecting data for diagnosis of the preclinical state of a disorder or abnormality associated with alpha-synuclein aggregates in a patient comprising detecting the specific binding of a compound as defined in any one of claims 1 to 5 to alpha-synuclein aggregates in a sample which comprises the steps of:
(a) bringing the sample suspected to contain the alpha-synuclein aggregates into contact with the compound as defined in any one of claims 1 to 5, which compound specifically binds to the alpha-synuclein aggregates;
(b) allowing the compound to bind to the alpha-synuclein aggregates to form a compound/(alpha-synuclein aggregate) complex;
(c) detecting the formation of the compound/(alpha-synuclein aggregate) complex;
(d) optionally correlating the presence or absence of the compound/(alpha-synuclein aggregate) complex with the presence or absence of alpha-synuclein aggregates in the sample; and
(e) optionally comparing the amount of the compound/(alpha-synuclein aggregate) complex to a normal control value.

14. A method of collecting data for monitoring residual disorder in a patient suffering from a disorder or abnormality associated with alpha-synuclein aggregates who has been treated with a medicament, wherein the method comprises:
(a) bringing a sample suspected to contain alpha-synuclein aggregates into contact with a compound as defined in any one of claims 1 to 5, which compound specifically binds to the alpha-synuclein aggregates;
(b) allowing the compound to bind to the alpha-synuclein aggregates to form a compound/(alpha-synuclein aggregate) complex;
(c) detecting the formation of the compound/(alpha-synuclein aggregate) complex;
(d) optionally correlating the presence or absence of the compound/(alpha-synuclein aggregate) complex with the presence or absence of alpha-synuclein aggregate in the sample; and
(e) optionally comparing the amount of the compound/(alpha-synuclein aggregate) complex to a normal control value.

15. A method of collecting data for predicting responsiveness of a patient suffering from a disorder or abnormality associated with alpha-synuclein aggregates and being treated with a medicament comprising:
(a) bringing a sample suspected to contain alpha-synuclein aggregates into contact with a compound as defined in any one of claims 1 to 5, which compound specifically binds to the alpha-synuclein aggregates;
(b) allowing the compound to bind to the alpha-synuclein aggregates to form a compound/(alpha-synuclein aggregate) complex;
(c) detecting the formation of the compound/(alpha-synuclein aggregate) complex;
(d) optionally correlating the presence or absence of the compound/(alpha-synuclein aggregate) complex with the presence or absence of alpha-synuclein aggregates in the sample; and
(e) optionally comparing the amount of the compound/(alpha-synuclein aggregate) complex to a normal control value.

16. A method of monitoring residual disorder in a patient suffering from a disorder or abnormality associated with alpha-synuclein aggregates who has been treated with a medicament, wherein the method comprises:
(a) bringing a sample suspected to contain alpha-synuclein aggregates into contact with a compound as defined in any one of claims 1 to 5, which compound specifically binds to the alpha-synuclein aggregates;
(b) allowing the compound to bind to the alpha-synuclein aggregates to form a compound/(alpha-synuclein aggregate) complex;
(c) detecting the formation of the compound/(alpha-synuclein aggregate) complex;
(d) optionally correlating the presence or absence of the compound/(alpha-synuclein aggregate) complex with the presence or absence of alpha-synuclein aggregates in the sample; and
(e) optionally comparing the amount of the compound/(alpha-synuclein aggregate) complex to a normal control value.

17. A method of predicting responsiveness of a patient suffering from a disorder or abnormality associated with alpha-synuclein aggregates and being treated with a medicament comprising:
(a) bringing a sample suspected to contain alpha-synuclein aggregates into contact with a compound as defined in any one of claims 1 to 5, which compound specifically binds to the alpha-synuclein aggregates;
(b) allowing the compound to bind to the alpha-synuclein aggregates to form a compound/(alpha-synuclein aggregate) complex;
(c) detecting the formation of the compound/(alpha-synuclein aggregate) complex;
(d) optionally correlating the presence or absence of the compound/(alpha-synuclein aggregate) complex with the presence or absence of alpha-synuclein aggregates in the sample; and
(e) optionally comparing the amount of the compound/(alpha-synuclein aggregate) complex to a normal control value.

18. The method according to any one of claims 12 to 17, wherein the disorder is selected from Parkinson's disease (including sporadic, familial with alpha-synuclein mutations, familial with mutations other than alpha-synuclein, pure autonomic failure or Lewy body dysphagia), dementia with Lewy bodies (including "pure" Lewy body dementia), sporadic Alzheimer's disease, familial Alzheimer's disease with APP mutations, familial Alzheimer's disease with PS-1, PS-2 or other mutations, familial British dementia, Lewy body variant of Alzheimer's disease, Down syndrome, multiple system atrophy (including Shy-Drager syndrome, striatonigral degeneration or olivopontocerebellar atrophy), traumatic brain injury, chronic traumatic encephalopathy, motor neuron disease, neuroaxonal dystrophy, neurodegeneration with brain iron accumulation type 1 (including Hallervorden-Spatz syndrome), prion diseases, ataxia telangiectatica, Meige's syndrome, subacute sclerosing panencephalitis, Gaucher disease, lysosomal storage disorders (including Kufor-Rakeb syndrome and Sanfilippo syndrome) and rapid eye movement (REM) sleep behavior disorder.

19. The method according to any one of claims 12 to 17, wherein the disorder is Parkinson's disease, dementia with Lewy bodies or multiple system atrophy.

20. A method of determining the amount of alpha-synuclein aggregates in a sample comprising:
(a) providing the sample;
(b) testing the sample for the presence of alpha-synuclein aggregates with a compound as defined in any one of claims 1 to 5;
(c) determining the amount of compound bound to the alpha-synuclein aggregates; and
(d) calculating the amount of alpha-synuclein aggregates in the sample.

21. The method according to any one of claims 12 to 20, wherein alpha-synuclein aggregates include Lewy bodies and/or Lewy neurites.

22. A mixture comprising a compound as defined in any one of claims 1 to 5 and at least one compound selected from an imaging agent different from the compound as defined in any one of claims 1 to 5, preferably an abeta or Tau imaging agent, a pharmaceutically acceptable carrier, a diluent and an excipient.

23. A compound of formula (II) 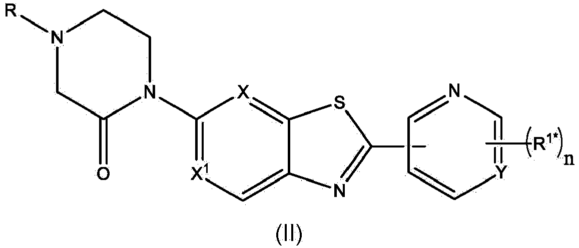
wherein **R, X, X¹**, **Y** and **n** are as defined in claim 1;
**R^{1*}** is independently selected from the group consisting of **LG,** 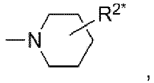 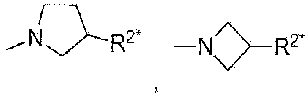 and -NR^{3*}R^{4*};
wherein
**R^{2*}** is selected from the group consisting of **LG** and hydrogen;
**R^{3*}** and **R^{4*}** are independently selected from the group consisting of alkyl, **LG**-alkyl, alkyl-O-alkyl, **PG** and hydrogen;
**LG** is a leaving group selected from halogen, trimethylammonium, C₁₋₄ alkyl sulfonate and C₆₋₁₀ aryl sulfonate; and
**PG** is an amino protecting group selected from carbobenzyloxy, p-methoxybenzyl carbonyl, tert-butyloxycarbonyl, 9-fluorenylmethyloxycarbonyl, benzyl, p-methoxybenzyl, 3,4-dimethoxybenzyl, p-methoxyphenyl, triphenylmethyl, methoxyphenyl diphenylmethyl, and dimethoxytrityl,
wherein at least one of **R^{1*}**, **R^{2*}**, **R^{3*}** and **R^{4*}** contains **LG.**

24. The compound according to claim 23, wherein **LG** is selected from mesylate, triflate, tosylate and nosylate.

25. A method for preparing the compound according to claim 5, wherein the compound is labelled by ¹⁸F, comprising reacting the compound according to claim 10 with a ¹⁸F-fluorinating agent, so that **LG** is replaced by ¹⁸F, preferably wherein the ¹⁸F-fluorinating agent is selected from K¹⁸F, H¹⁸F, Cs¹⁸F, Na¹⁸F and tetrabutylammonium [¹⁸F]fluoride.

26. Use of the compound according to any one of claims 1 to 5 as an *in vitro* analytical reference or an *in vitro* screening tool.

27. A test kit for use in the detection and/or diagnosis of a disorder or abnormality associated with alpha-synuclein aggregates including, but not limited to, Lewy bodies and/or Lewy neurites, wherein the test kit comprises at least one compound as defined in any one of claims 1 to 5.

28. A kit for preparing a radiopharmaceutical preparation, wherein the kit comprises a sealed vial containing at least one compound as defined in claim 23.

## Patentansprüche

1. Eine Verbindung der Formel (I): 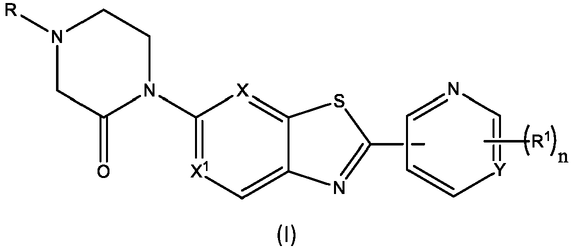
oder Stereoisomere, racemische Gemische, pharmazeutisch verträgliche Salze, Hydrate, Solvate und Polymorphe davon;
wobei
**R** ausgewählt ist aus der Gruppe bestehend aus Wasserstoff und Alkyl;
**R¹** unabhängig ausgewählt ist aus der Gruppe bestehend aus Fluor, 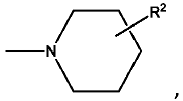 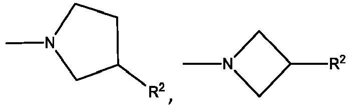 und -NR³R⁴;
**R²** ausgewählt ist aus der Gruppe bestehend aus Fluor und Wasserstoff;
**R³** und **R⁴** unabhängig ausgewählt sind aus der Gruppe bestehend aus Alkyl, Fluor-Alkyl, Alkyl-O-Alkyl und Wasserstoff;
**X** und **X¹** unabhängig ausgewählt sind aus der Gruppe bestehend aus N und CH, mit der Maßgabe, dass mindestens eines von X und X¹ für N steht;
**Y** unabhängig ausgewählt ist aus der Gruppe bestehend aus N und CH oder **Y** für C steht, wenn **Y** an **R¹** gebunden ist;
n gleich 1 oder 2 ist;
wobei die Verbindung gegebenenfalls eine detektierbare Markierung, ausgewählt aus Radionukliden, Positronenemittern, Gammaemittern, fluoreszierenden Markierungen, lumineszierenden Markierungen und chromogenen Markierungen enthalten kann.

2. Die Verbindung gemäß Anspruch 1, welche eine Verbindung der Formel (Ia), (Ib) oder (Ic) ist: 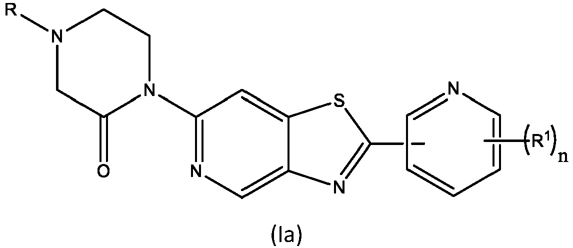 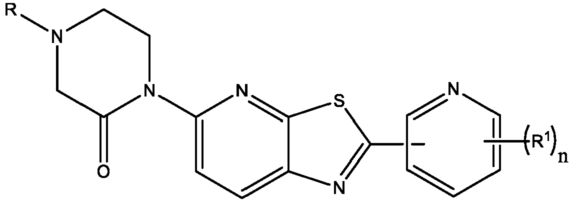 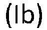 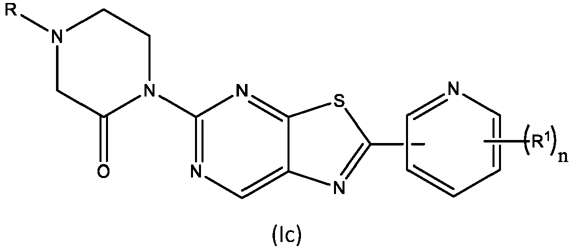 wobei **R, R¹** und **n** wie in Anspruch 1 definiert sind.

3. Die Verbindung gemäß einem der Ansprüche 1 und 2, wobei **R¹** für 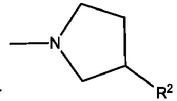 steht.

4. Die Verbindung gemäß einem der Ansprüche 1 bis 3, wobei mindestens eines von **R¹**, **R²**, **R³** und **R⁴** Fluor enthält.

5. Die Verbindung gemäß einem der Ansprüche 1 bis 4, wobei die Verbindung detektierbar markiert ist, bevorzugt mit ²H, ³H, ¹⁸F oder ¹³N, stärker bevorzugt mit ¹⁸F.

6. Eine diagnostische Zusammensetzung, umfassend eine Verbindung gemäß einem der Ansprüche 1 bis 5 und gegebenenfalls einen pharmazeutisch verträglichen Träger, ein Verdünnungsmittel, ein Hilfsmittel und/oder einen Exzipienten.

7. Die Verbindung gemäß einem der Ansprüche 1 bis 5 zur Verwendung bei der Diagnose.

8. Die Verbindung gemäß einem der Ansprüche 1 bis 5 zur Verwendung bei der Bildgebung von Alpha-Synuclein-Aggregaten einschließlich, aber nicht beschränkt auf Lewy-Körperchen und/oder Lewy-Neuriten, vorzugsweise wobei die Verbindung zur Verwendung bei der Positronenemissionstomographie-Bildgebung von Alpha-Synuclein-Aggregaten einschließlich, aber nicht beschränkt auf Lewy-Körperchen und/oder Lewy-Neuriten, ist.

9. Die Verbindung gemäß einem der Ansprüche 1 bis 5 zur Verwendung bei der Diagnose einer Störung oder Anomalie in Zusammenhang mit Alpha-Synuclein-Aggregaten einschließlich, aber nicht beschränkt auf Lewy-Körperchen und/oder Lewy-Neuriten, oder eines präklinischen Stadiums davon.

10. Die Verbindung zur Verwendung gemäß Anspruch 9, wobei die Störung ausgewählt ist aus Parkinson-Krankheit (einschließlich sporadischer, familiärer mit Alpha-Synuclein-Mutationen, familiärer mit anderen Mutationen außer Alpha-Synuclein, reinem autonomen Versagen oder Lewy-Körperchen-Dysphagie), Demenz mit Lewy-Körperchen (einschließlich "reiner" Lewy-Körperchen-Demenz), sporadischer Alzheimer-Krankheit, familiärer Alzheimer-Krankheit mit APP-Mutationen, familiärer Alzheimer-Krankheit mit PS-1, PS-2 oder anderen Mutationen, familiärer Britischer Demenz, Lewy-Körperchen-Variante der Alzheimer-Krankheit, Down-Syndrom, Multisystematrophie (einschließlich Shy-Drager-Syndrom, striatonigraler Degeneration oder olivopontozerebellärer Atrophie), traumatischer Hirnverletzung, chronisch traumatischer Enzephalopathie, Motoneuron-Erkrankung, neuroaxonaler Dystrophie, Neurodegeneration mit Eisenspeicherung im Gehirn vom Typ 1 (einschließlich Hallervorden-Spatz-Syndrom), Prionkrankheiten, Ataxie Telangiectatica, Meige-Syndrom, subakuter sklerosierender Panenzephalitis, Gaucher-Krankheit, lysosomalen Speicherkrankheiten (einschließlich Kufor-Rakeb-Syndrom und Sanfilippo-Syndrom) und Störung des REM-Schlafverhaltens.

11. Die Verbindung zur Verwendung gemäß Anspruch 9, wobei die Störung Parkinson-Krankheit, Demenz mit Lewy-Körperchen oder Multisystematrophie ist.

12. Ein Verfahren zum Sammeln von Daten für die Diagnose einer Störung oder Anomalie in Zusammenhang mit Alpha-Synuclein-Aggregaten bei einem Patienten, umfassend:
(a) Inkontaktbringen einer Probe, von der vermutet wird, dass sie Alpha-Synuclein-Aggregate enthält, mit einer Verbindung, wie in einem der Ansprüche 1 bis 5 definiert;
(b) Bindenlassen der Verbindung an die Alpha-Synuclein-Aggregate;
(c) Nachweisen der Verbindung, welche an die Alpha-Synuclein-Aggregate gebunden ist; und
(d) gegebenenfalls Korrelieren des Vorhandenseins oder der Abwesenheit der Verbindung, welche an die Alpha-Synuclein-Aggregate gebunden ist, mit dem Vorhandensein oder der Abwesenheit von Alpha-Synuclein-Aggregaten in der Probe.

13. Ein Verfahren zur zum Sammeln von Daten zur Diagnose des präklinischen Stadiums einer Störung oder Anomalie, die mit Alpha-Synuclein-Aggregaten assoziert ist, bei einem Patienten, umfassend das Nachweisen der spezifischen Bindung einer Verbindung, wie in einem der Ansprüche 1 bis 5 definiert, an Alpha-Synuclein-Aggregate in einer Probe, welches die Schritte umfasst:
(a) Inkontaktbringen der Probe, von der vermutet wird, dass sie Alpha-Synuclein-Aggregate enthält, mit der Verbindung wie in einem der Ansprüche 1 bis 5 definiert, wobei die Verbindung spezifischen an die Alpha-Synuclein-Aggregate bindet;
(b) Bindenlassen der Verbindung an die Alpha-Synuclein-Aggregate, um einen Verbindungs-/(Alpha-Synuclein-Aggregat)-Komplex zu bilden;
(c) Nachweisen der Bildung des Verbindungs-/(Alpha-Synuclein-Aggregat)-Komplexes;
(d) gegebenenfalls Korrelieren des Vorhandenseins oder der Abwesenheit des Verbindungs-/(Alpha-Synuclein-Aggregat)-Komplexes mit dem Vorhandensein oder der Abwesenheit von Alpha-Synuclein-Aggregaten in der Probe; und
(e) gegebenenfalls Vergleichen der Menge des Verbindungs-/(Alpha-Synuclein-Aggregat)-Komplexes mit einem normalen Kontrollwert.

14. Ein Verfahren zur zum Sammeln von Daten zur Überwachung einer Reststörung bei einem Patienten, der an einer Störung oder Anomalie, die mit Alpha-Synuclein-Aggregaten assoziert ist, leidet, der mit einem Medikament behandelt wurde, wobei das Verfahren umfasst:
(a) Inkontaktbringen einer Probe, von der vermutet wird, dass sie Alpha-Synuclein-Aggregate enthält, mit einer Verbindung wie in einem der Ansprüche 1 bis 5 definiert, wobei die Verbindung spezifisch an die Alpha-Synuclein-Aggregate bindet;
(b) Bindenlassen der Verbindung an die Alpha-Synuclein-Aggregate, um einen Verbindungs-/(Alpha-Synuclein-Aggregat)-Komplex zu bilden;
(c) Nachweisen der Bildung des Verbindungs-/(Alpha-Synuclein-Aggregat)-Komplexes;
(d) gegebenenfalls Korrelieren des Vorhandenseins oder der Abwesenheit des Verbindungs-/(Alpha-Synuclein-Aggregat)-Komplexes mit dem Vorhandensein oder der Abwesenheit von Alpha-Synuclein-Aggregaten in der Probe; und
(e) gegebenenfalls Vergleichen der Menge des Verbindungs-/(Alpha-Synuclein-Aggregat)-Komplexes mit einem normalen Kontrollwert.

15. Ein Verfahren zur zum Sammeln von Daten zur Vorhersage, ob ein Patient, welcher an einer Störung oder Anomalie, die mit Alpha-Synuclein-Aggregaten assoziert ist, leidet und mit einem Medikament behandelt wird, darauf anspricht, umfassend:
(a) Inkontaktbringen einer Probe, von der vermutet wird, dass sie Alpha-Synuclein-Aggregate enthält, mit einer Verbindung wie in einem der Ansprüche 1 bis 5 definiert, wobei die Verbindung spezifisch an die Alpha-Synuclein-Aggregate bindet;
(b) Bindenlassen der Verbindung an die Alpha-Synuclein-Aggregate, um einen Verbindungs-/(Alpha-Synuclein-Aggregat)-Komplex zu bilden;
(c) Nachweisen der Bildung des Verbindungs-/(Alpha-Synuclein-Aggregat)-Komplexes;
(d) gegebenenfalls Korrelieren des Vorhandenseins oder der Abwesenheit des Verbindungs-/(Alpha-Synuclein-Aggregat)-Komplexes mit dem Vorhandensein oder der Abwesenheit von Alpha-Synuclein-Aggregaten in der Probe; und
(e) gegebenenfalls Vergleichen der Menge des Verbindungs-/(Alpha-Synuclein-Aggregat)-Komplexes mit einem normalen Kontrollwert.

16. Ein Verfahren zur Überwachung einer Reststörung bei einem Patienten, der an einer Störung oder Anomalie, die mit Alpha-Synuclein-Aggregaten assoziert ist, leidet, der mit einem Medikament behandelt wurde, wobei das Verfahren umfasst:
(a) Inkontaktbringen einer Probe, von der vermutet wird, dass sie Alpha-Synuclein-Aggregate enthält, mit einer Verbindung wie in einem der Ansprüche 1 bis 5 definiert, wobei die Verbindung spezifisch an die Alpha-Synuclein-Aggregate bindet;
(b) Bindenlassen der Verbindung an die Alpha-Synuclein-Aggregate, um einen Verbindungs-/(Alpha-Synuclein-Aggregat)-Komplex zu bilden;
(c) Nachweisen der Bildung des Verbindungs-/(Alpha-Synuclein-Aggregat)-Komplexes;
(d) gegebenenfalls Korrelieren des Vorhandenseins oder der Abwesenheit des Verbindungs-/(Alpha-Synuclein-Aggregat)-Komplexes mit dem Vorhandensein oder der Abwesenheit von Alpha-Synuclein-Aggregaten in der Probe; und
(e) gegebenenfalls Vergleichen der Menge des Verbindungs-/(Alpha-Synuclein-Aggregat)-Komplexes mit einem normalen Kontrollwert.

17. Ein Verfahren zur Vorhersage, ob ein Patienten, welcher an einer Störung oder Anomalie, die mit Alpha-Synuclein-Aggregaten assoziert ist, leidet und mit einem Medikament behandelt wird, darauf anspricht, umfassend:
(a) Inkontaktbringen einer Probe, von der vermutet wird, dass sie Alpha-Synuclein-Aggregate enthält, mit einer Verbindung wie in einem der Ansprüche 1 bis 5 definiert, wobei die Verbindung spezifisch an die Alpha-Synuclein-Aggregate bindet;
(b) Bindenlassen der Verbindung an die Alpha-Synuclein-Aggregate, um einen Verbindungs-/(Alpha-Synuclein-Aggregat)-Komplex zu bilden;
(c) Nachweisen der Bildung des Verbindungs-/(Alpha-Synuclein-Aggregat)-Komplexes;
(d) gegebenenfalls Korrelieren des Vorhandenseins oder der Abwesenheit des Verbindungs-/(Alpha-Synuclein-Aggregat)-Komplexes mit dem Vorhandensein oder der Abwesenheit von Alpha-Synuclein-Aggregaten in der Probe; und
(e) gegebenenfalls Vergleichen der Menge des Verbindungs-/(Alpha-Synuclein-Aggregat)-Komplexes mit einem normalen Kontrollwert.

18. Das Verfahren gemäß einem der Ansprüche 12 bis 17, wobei die Störung ausgewählt ist aus Parkinson-Krankheit (einschließlich sporadischer, familiärer mit Alpha-Synuclein-Mutationen, familiärer mit anderen Mutationen außer Alpha-Synuclein, reinem autonomen Versagen oder Lewy-Körperchen-Dysphagie), Demenz mit Lewy-Körperchen (einschließlich "reiner" Lewy-Körperchen-Demenz), sporadischer Alzheimer-Krankheit, familiärer Alzheimer-Krankheit mit APP-Mutationen, familiärer Alzheimer-Krankheit mit PS-1, PS-2 oder anderen Mutationen, familiärer Britischer Demenz, Lewy-Körperchen-Variante der Alzheimer-Krankheit, Down-Syndrom, Multisystematrophie (einschließlich Shy-Drager-Syndrom, striatonigraler Degeneration oder olivopontozerebellärer Atrophie), traumatischer Hirnverletzung, chronisch traumatischer Enzephalopathie, Motoneuron-Erkrankung, neuroaxonaler Dystrophie, Neurodegeneration mit Eisenspeicherung im Gehirn vom Typ 1 (einschließlich Hallervorden-Spatz-Syndrom), Prionkrankheiten, Ataxie Telangiectatica, Meige-Syndrom, subakuter sklerosierender Panenzephalitis, Gaucher-Krankheit, lysosomalen Speicherkrankheiten (einschließlich Kufor-Rakeb-Syndrom und Sanfilippo-Syndrom) und Störung des REM-Schlafverhaltens.

19. Das Verfahren gemäß einem der Ansprüche 12 bis 17, wobei die Störung Parkinson-Krankheit, Demenz mit Lewy-Körperchen oder Multisystematrophie ist.

20. Ein Verfahren zur Bestimmung der Menge an Alpha-Synuclein-Aggregaten in einer Probe, umfassend:
(a) Bereitstellen der Probe;
(b) Testen der Probe auf das Vorhandensein von Alpha-Synuclein-Aggregaten mit einer Verbindung wie in einem der Ansprüche 1 bis 5 definiert;
(c) Bestimmen der Menge der Verbindung, welche an die Alpha-Synuclein-Aggregate gebunden ist; und
(d) Berechnen der Menge an Alpha-Synuclein-Aggregaten in der Probe.

21. Das Verfahren gemäß einem der Ansprüche 12 bis 20, wobei die Alpha-Synuclein-Aggregate Lewy-Körperchen und/oder Lewy-Neuriten beinhalten.

22. Ein Gemisch, umfassend eine Verbindung, wie in einem der Ansprüche 1 bis 5 definiert, und mindestens eine Verbindung ausgewählt aus einem Mittel zur Bildgebung, das sich von der Verbindung, wie in einem der Ansprüche 1 bis 5 definiert, unterscheidet, bevorzugt ein Mittel zur Abeta- oder Tau-Bildgebungs, einen pharmazeutisch verträglichen Träger, ein Verdünnungsmittel und einen Exzipienten.

23. Eine Verbindung der Formel (II) 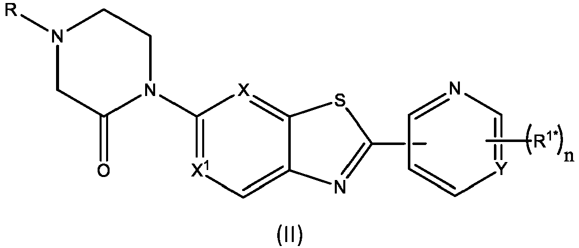
wobei **R, X, X¹**, **Y** und **n** wie in Anspruch 1 definiert sind;
**R^{1*}** unabhängig ausgewählt ist aus der Gruppe bestehend aus **LG,** 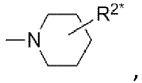 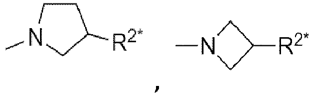 und -NR^{3*}R^{4*};
wobei
**R^{2*}** ausgewählt ist aus der Gruppe bestehend aus **LG** und Wasserstoff;
**R^{3*}** und **R^{4*}** unabhängig ausgewählt sind aus der Gruppe bestehend aus Alkyl, **LG**-Alkyl, Alkyl-O-Alkyl, **PG** und Wasserstoff;
**LG** eine Abgangsgruppe ist, ausgewählt aus Halogen, Trimethylammonium, C₁₋₄-Alkylsulfonat und C₆₋₁₀-Arylsulfonate; und
PG eine Aminschutzgruppe ist, ausgewählt aus Carbobenzyloxy, p-Methoxybenzylcarbonyl, tert-Butyloxycarbonyl, 9-Fluorenylmethyloxycarbonyl, Benzyl, p-Methoxybenzyl, 3,4-Dimethoxybenzyl, p-Methoxyphenyl, Triphenylmethyl, Methoxyphenyldiphenylmethyl und Dimethoxytrityl,
wobei mindestens eines von **R^{1*}**, **R^{2*}**, **R^{3*}** und **R^{4*} LG** enthält.

24. Die Verbindung gemäß Anspruch 23, wobei **LG** ausgewählt ist aus Mesylat, Triflat, Tosylat und Nosylat.

25. Ein Verfahren zur Herstellung der Verbindung gemäß Anspruch 5, wobei die Verbindung mit ¹⁸F markiert ist, umfassend das Umsetzen der Verbindung gemäß Anspruch 10 mit einem ¹⁸F-Fluorierungsmittel, so dass **LG** durch ¹⁸F ersetzt wird, bevorzugt wobei das ¹⁸F-Fluorierungsmittel ausgewählt ist aus K¹⁸F, H¹⁸F, Cs¹⁸F, Na¹⁸F und Tetrabutylammonium-[¹⁸F]Fluorid.

26. Verwendung der Verbindung gemäß einem der Ansprüche 1 bis 5 als eine *in vitro* analytische Referenz oder ein *in vitro*-Screening-Tool.

27. Ein Testkit zur Verwendung bei dem Nachweis und/oder Diagnose einer Störung oder Anomalie, die mit Alpha-Synuclein-Aggregaten, einschließlich, aber nicht beschränkt auf Lewy-Körperchen und/oder Lewy-Neuriten, assoziert ist, wobei das Testkit mindestens eine Verbindung, wie in einem der Ansprüche 1 bis 5 definiert, umfasst.

28. Ein Kit zur Herstellung eines radioaktiven Arzneimittels, wobei das Kit eine verschlossene Ampulle, enthaltend mindestens eine Verbindung, wie in Anspruch 23 definiert, umfasst.

## Revendications

1. Composé de formule (I) : 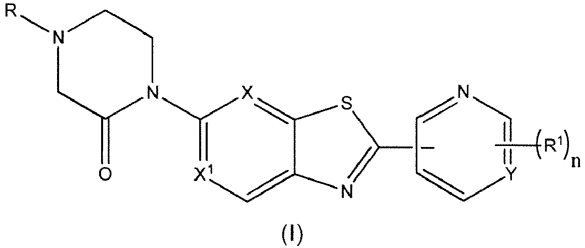
ou ses stéréoisomères, mélanges racémiques, sels pharmaceutiquement acceptables, hydrates, solvates, et polymorphes ;
dans lequel
R est choisi dans le groupe constitué par l'hydrogène et un alkyle ;
R¹ est indépendamment choisi dans le groupe constitué par le fluor, 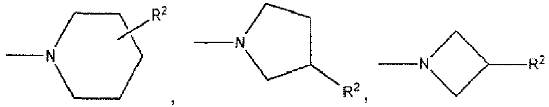 et -NR³R⁴ ;
R² est choisi dans le groupe constitué par le fluor et l'hydrogène ;
R³ et R⁴ sont indépendamment choisis dans le groupe constitué par un alkyle, un fluoroalkyle, un alkyl-O-alkyle et l'hydrogène ;
X et X¹ sont indépendamment choisis dans le groupe constitué par N et CH, sous réserve qu'au moins l'un de X et X¹ soit N ;
Y est indépendamment choisi dans le groupe constitué par N et CH ou Y est C si Y est rattaché à R¹ ;
n vaut 1 ou 2 ;
lequel composé peut éventuellement contenir un marqueur détectable choisi parmi les radionucléides, les émetteurs de positons, les émetteurs de rayons gamma, les marqueurs fluorescents, les marqueurs luminescents et les marqueurs chromogéniques.

2. Composé selon la revendication 1, qui est un composé de formule (la), (Ib) ou (Ic) : 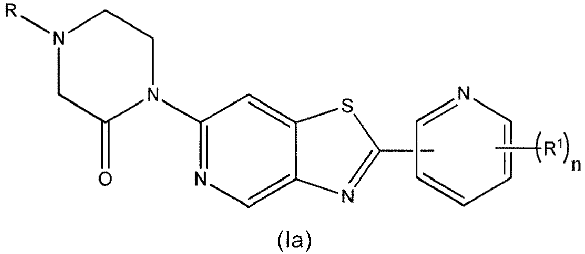 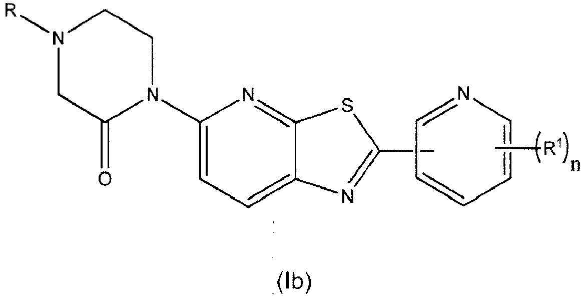 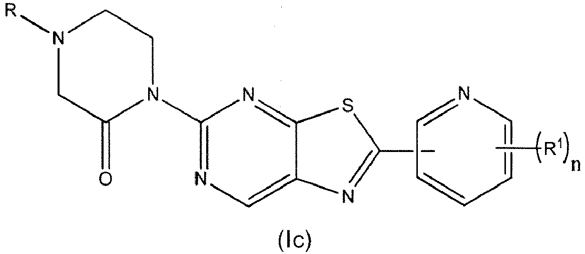 dans lequel R, R¹ et n sont tels que définis dans la revendication 1.

3. Composé selon l'une quelconque des revendications 1 et 2, dans lequel R¹ est

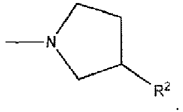

4. Composé selon l'une quelconque des revendications 1 à 3, dans lequel au moins l'un de R¹, R², R³ et R⁴ contient du fluor.

5. Composé selon l'une quelconque des revendications 1 à 4, lequel composé est marqué de manière détectable, de préférence avec ²H, ³H, ¹⁸F ou ¹³N, mieux encore avec ¹⁸F.

6. Composition diagnostique comprenant un composé selon l'une quelconque des revendications 1 à 5 et éventuellement un véhicule, diluant, adjuvant et/ou excipient pharmaceutiquement acceptable.

7. Composé selon l'une quelconque des revendications 1 à 5, pour une utilisation en diagnostic.

8. Composé selon l'une quelconque des revendications 1 à 5, pour une utilisation dans l'imagerie d'agrégats d'alpha-synucléine comprenant, mais sans s'y limiter, des corps de Lewy et/ou des neurites de Lewy, de préférence lequel composé est destiné à être utilisé en imagerie par tomographie par émission de positons d'agrégats d'alpha-synucléine comprenant, mais sans s'y limiter, des corps de Lewy et/ou des neurites de Lewy.

9. Composé selon l'une quelconque des revendications 1 à 5, pour une utilisation dans le diagnostic d'un trouble ou d'une anomalie associé à des agrégats d'alpha-synucléine comprenant, mais sans s'y limiter, des corps de Lewy et/ou des neurites de Lewy, ou d'un état préclinique de celui-ci.

10. Composé pour une utilisation selon la revendication 9, dans lequel le trouble est choisi parmi la maladie de Parkinson (y compris sporadique, familiale avec mutations d'alpha-synucléine, familiale avec mutations autres que l'alpha-synucléine, insuffisance autonomique pure ou dysphagie à corps de Lewy), une démence à corps de Lewy (y compris une démence à corps de Lewy "pure"), la maladie d'Alzheimer sporadique, la maladie d'Alzheimer familiale avec mutations d'APP, la maladie d'Alzheimer familiale avec mutations de PS-1, PS-2 ou autres, la démence britannique familiale, un variant à corps de Lewy de la maladie d'Alzheimer, le syndrome de Down, une atrophie multisystématisée (y compris le syndrome de Shy-Drager, une dégénérescence striatonigrique ou une atrophie olivo-ponto-cérébelleuse), un traumatisme crânien, une encéphalopathie traumatique chronique, une maladie des neurones moteurs, une dystrophie neuro-axonale, une neurodégénérescence avec accumulation de fer dans le cerveau de type 1 (y compris le syndrome de Hallervorden-Spatz), les maladies à prions, une ataxie télangiectasie, le syndrome de Meige, une panencéphalite sclérosante subaiguë, la maladie de Gaucher, les troubles du stockage des lysosomes (y compris le syndrome de Kufor-Rakeb et le syndrome de Sanfilippo) et un trouble du comportement du sommeil paradoxal (REM).

11. Composé pour une utilisation selon la revendication 9, dans lequel le trouble est la maladie de Parkinson, la démence à corps de Lewy, ou une atrophie multisystématisée.

12. Méthode de collecte de données pour le diagnostic d'un trouble ou d'une anomalie associé à des agrégats d'alpha-synucléine chez un patient, comprenant :
(a) la mise en contact d'un échantillon suspecté de contenir des agrégats d'alpha-synucléine avec un composé tel que défini dans l'une quelconque des revendications 1 à 5 ;
(b) le fait de laisser le composé se lier aux agrégats d'alpha-synucléine ;
(c) la détection du composé lié aux agrégats d'alpha-synucléine ; et
(d) éventuellement la corrélation de la présence ou de l'absence de liaison du composé avec les agrégats d'alpha-synucléine avec la présence ou l'absence d'agrégats d'alpha-synucléine dans l'échantillon.

13. Méthode de collecte de données pour le diagnostic de l'état préclinique d'un trouble ou d'une anomalie associé à des agrégats d'alpha-synucléine chez un patient, comprenant la détection de la liaison spécifique d'un composé tel que défini dans l'une quelconque des revendications 1 à 5 à des agrégats d'alpha-synucléine dans un échantillon, qui comprend les étapes suivantes :
(a) la mise en contact de l'échantillon suspecté de contenir les agrégats d'alpha-synucléine avec le composé tel que défini dans l'une quelconque des revendications 1 à 5, lequel composé se lie spécifiquement aux agrégats d'alpha-synucléine ;
(b) le fait de laisser le composé se lier aux agrégats d'alpha-synucléine pour former un complexe composé/(agrégats d'alpha-synucléine) ;
(c) la détection de la formation du complexe composé/(agrégats d'alpha-synucléine) ;
(d) éventuellement la corrélation de la présence ou de l'absence du complexe composé/(agrégats d'alpha-synucléine) avec la présence ou l'absence d'agrégats d'alpha-synucléine dans l'échantillon ; et
(e) éventuellement la comparaison de la quantité du complexe composé/(agrégats d'alpha-synucléine) avec une valeur témoin normale.

14. Méthode de collecte de données pour surveiller un trouble résiduel chez un patient souffrant d'un trouble ou d'une anomalie associé à des agrégats d'alpha-synucléine, qui a été traité avec un médicament, laquelle méthode comprend :
(a) la mise en contact d'un échantillon suspecté de contenir des agrégats d'alpha-synucléine avec un composé tel que défini dans l'une quelconque des revendications 1 à 5, lequel composé se lie spécifiquement aux agrégats d'alpha-synucléine ;
(b) le fait de laisser le composé se lier aux agrégats d'alpha-synucléine pour former un complexe composé/(agrégats d'alpha-synucléine) ;
(c) la détection de la formation du complexe composé/(agrégats d'alpha-synucléine) ;
(d) éventuellement la corrélation de la présence ou de l'absence du complexe composé/(agrégats d'alpha-synucléine) avec la présence ou l'absence d'agrégats d'alpha-synucléine dans l'échantillon ; et
(e) éventuellement la comparaison de la quantité du complexe composé/(agrégats d'alpha-synucléine) avec une valeur témoin normale.

15. Méthode de collecte de données pour prédire la réactivité d'un patient souffrant d'un trouble ou d'une anomalie associé à des agrégats d'alpha-synucléine, et qui est traité avec un médicament, comprenant :
(a) la mise en contact d'un échantillon suspecté de contenir des agrégats d'alpha-synucléine avec un composé tel que défini dans l'une quelconque des revendications 1 à 5, lequel composé se lie spécifiquement aux agrégats d'alpha-synucléine ;
(b) le fait de laisser le composé se lier aux agrégats d'alpha-synucléine pour former un complexe composé/(agrégats d'alpha-synucléine) ;
(c) la détection de la formation du complexe composé/(agrégats d'alpha-synucléine) ;
(d) éventuellement la corrélation de la présence ou de l'absence du complexe composé/(agrégats d'alpha-synucléine) avec la présence ou l'absence d'agrégats d'alpha-synucléine dans l'échantillon ; et
(e) éventuellement la comparaison de la quantité du complexe composé/(agrégats d'alpha-synucléine) avec une valeur témoin normale.

16. Méthode de surveillance d'un trouble résiduel chez un patient souffrant d'un trouble ou d'une anomalie associé à des agrégats d'alpha-synucléine, qui a été traité avec un médicament, laquelle méthode comprend :
(a) la mise en contact d'un échantillon suspecté de contenir des agrégats d'alpha-synucléine avec un composé tel que défini dans l'une quelconque des revendications 1 à 5, lequel composé se lie spécifiquement aux agrégats d'alpha-synucléine ;
(b) le fait de laisser le composé se lier aux agrégats d'alpha-synucléine pour former un complexe composé/(agrégats d'alpha-synucléine) ;
(c) la détection de la formation du complexe composé/(agrégats d'alpha-synucléine) ;
(d) éventuellement la corrélation de la présence ou de l'absence du complexe composé/(agrégats d'alpha-synucléine) avec la présence ou l'absence d'agrégats d'alpha-synucléine dans l'échantillon ; et
(e) éventuellement la comparaison de la quantité du complexe composé/(agrégats d'alpha-synucléine) avec une valeur témoin normale.

17. Méthode de prédiction de la réactivité d'un patient souffrant d'un trouble ou d'une anomalie associé à des agrégats d'alpha-synucléine, et qui est traité avec un médicament, comprenant :
(a) la mise en contact d'un échantillon suspecté de contenir des agrégats d'alpha-synucléine avec un composé tel que défini dans l'une quelconque des revendications 1 à 5, lequel composé se lie spécifiquement aux agrégats d'alpha-synucléine ;
(b) le fait de laisser le composé se lier aux agrégats d'alpha-synucléine pour former un complexe composé/(agrégats d'alpha-synucléine) ;
(c) la détection de la formation du complexe composé/(agrégats d'alpha-synucléine) ;
(d) éventuellement la corrélation de la présence ou de l'absence du complexe composé/(agrégats d'alpha-synucléine) avec la présence ou l'absence d'agrégats d'alpha-synucléine dans l'échantillon ; et
(e) éventuellement la comparaison de la quantité du complexe composé/(agrégats d'alpha-synucléine) avec une valeur témoin normale.

18. Méthode selon l'une quelconque des revendications 12 à 17, dans laquelle le trouble est choisi parmi la maladie de Parkinson (y compris sporadique, familiale avec mutations d'alpha-synucléine, familiale avec mutations autres que l'alpha-synucléine, insuffisance autonomique pure ou dysphagie à corps de Lewy), une démence à corps de Lewy (y compris une démence à corps de Lewy "pure"), la maladie d'Alzheimer sporadique, la maladie d'Alzheimer familiale avec mutations d'APP, la maladie d'Alzheimer familiale avec mutations de PS-1, PS-2 ou autres, la démence britannique familiale, un variant à corps de Lewy de la maladie d'Alzheimer, le syndrome de Down, une atrophie multisystématisée (y compris le syndrome de Shy-Drager, une dégénérescence striatonigrique ou une atrophie olivo-ponto-cérébelleuse), un traumatisme crânien, une encéphalopathie traumatique chronique, une maladie des neurones moteurs, une dystrophie neuro-axonale, une neurodégénérescence avec accumulation de fer dans le cerveau de type 1 (y compris le syndrome de Hallervorden-Spatz), les maladies à prions, une ataxie télangiectasie, le syndrome de Meige, une panencéphalite sclérosante subaiguë, la maladie de Gaucher, les troubles du stockage des lysosomes (y compris le syndrome de Kufor-Rakeb et le syndrome de Sanfilippo) et un trouble du comportement du sommeil paradoxal (REM).

19. Méthode selon l'une quelconque des revendications 12 à 17, dans laquelle le trouble est la maladie de Parkinson, la démence à corps de Lewy, ou une atrophie multisystématisée.

20. Méthode de détermination de la quantité d'agrégats d'alpha-synucléine dans un échantillon, comprenant :
(a) la fourniture de l'échantillon ;
(b) le test de l'échantillon pour la présence d'agrégats d'alpha-synucléine avec un composé tel que défini dans l'une quelconque des revendications 1 à 5 ;
(c) la détermination de la quantité de composé lié aux agrégats d'alpha-synucléine ; et
(d) le calcul de la quantité d'agrégats d'alpha-synucléine dans l'échantillon.

21. Méthode selon l'une quelconque des revendications 12 à 20, dans laquelle les agrégats d'alpha-synucléine comprennent des corps de Lewy et/ou des neurites de Lewy.

22. Mélange comprenant un composé tel que défini dans l'une quelconque des revendications 1 à 5 et au moins un composé choisi parmi un agent d'imagerie différent du composé tel que défini dans l'une quelconque des revendications 1 à 5, de préférence un agent d'imagerie abêta ou tau, un véhicule pharmaceutiquement acceptable, un diluant et un excipient.

23. Composé de formule (II) 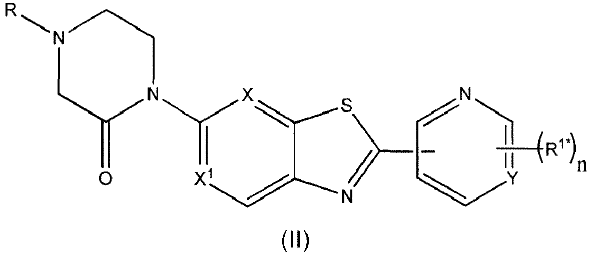
dans lequel R, X, X¹, Y et n sont tels que définis dans la revendication 1 ;
R^{1*} est indépendamment choisi dans le groupe constitué par LG, 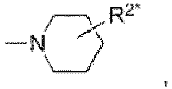 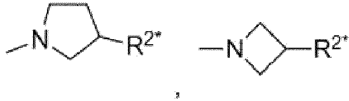 et -NR³R⁴ ;
dans lequel
**R^{2*}** est choisi dans le groupe constitué par LG et l'hydrogène ;
**R^{3*}** et **R^{4*}** sont indépendamment choisis dans le groupe constitué par un alkyle, un LG-alkyle, un alkyle-O-alkyle, PG et l'hydrogène ;
LG est un groupe partant choisi parmi un halogène, le triméthylammonium, un alkylsulfonate en C₁ à C₄ et un arylsulfonate en C₆ à C₁₀ ; et
PG est un groupe protecteur d'amino choisi parmi carbobenzyloxy, p-méthoxybenzylcarbonyle, tert-butyloxycarbonyle, 9-fluorénylméthyloxycarbonyle, benzyle, p-méthoxybenzyle, 3,4-diméthoxybenzyle, p-méthoxyphényle, triphénylméthyle, méthoxyphényl-diphénylméthyle, et diméthoxytrityle,
dans lequel au moins l'un de **R^{1*}**, **R^{2*}**, **R^{3*}** et **R^{4*}** contient LG.

24. Composé selon la revendication 23, dans lequel LG est choisi parmi le mésylate, le triflate, le tosylate et le nosylate.

25. Méthode pour préparer le composé selon la revendication 5, dans laquelle le composé est marqué au ¹⁸F, comprenant la réaction du composé selon la revendication 10 avec un agent de fluoration au ¹⁸F, de façon que LG soit remplacé par le ¹⁸F, de préférence dans laquelle l'agent de fluoration au ¹⁸F est choisi parmi K¹⁸F, H¹⁸F, Cs¹⁸F, Na¹⁸F et le [¹⁸F]fluorure de tétrabutylammonium.

26. Utilisation du composé selon l'une quelconque des revendications 1 à 5 en tant que référence analytique *in vitro* ou outil de dépistage *in vitro.*

27. Kit de test pour une utilisation dans la détection et/ou le diagnostic d'un trouble ou d'une anomalie associé à des agrégats d'alpha-synucléine comprenant, mais sans s'y limiter, des corps de Lewy et/ou des neurites de Lewy, lequel kit de test comprend au moins un composé tel que défini dans l'une quelconque des revendications 1 à 5.

28. Kit pour préparer une préparation radiopharmaceutique, lequel kit comprend un flacon scellé contenant au moins un composé tel que défini dans la revendication 23.
